# EUROPEAN PATENT APPLICATION

(11) **EP 3 998 081 A1**
(43) Date of publication of application: **18.05.2022**
(21) Application number: 20836994.2
(22) Date of filing: 03.07.2020
(51) Int. Cl.: A61K 39/395, A61K 45/00, A61P 35/00, A61P 35/02, C07K 16/28, C07K 16/30, C07K 16/46, C12N 15/13, C12N 15/62

(54) **TREATMENT OF HEMATOLOGIC CANCER WITH PD-1/CD3 DUAL SPECIFICITY PROTEIN**

(30) Priority: 05.07.2019 JP 2019126503; 30.07.2019 JP 2019139752
(71) Applicant: Ono Pharmaceutical Co., Ltd., Osaka-shi, Osaka 541-8526 (JP)
(72) Inventor: SHIBAYAMA, Shiro, Tsukuba-shi, Ibaraki 300-4247 (JP); SHIMBO, Takuya, Tsukuba-shi, Ibaraki 300-4247 (JP)
(74) Representative: Grünecker Patent- und Rechtsanwälte PartG mbB
(86) International application number: PCT/JP2020/026149
(87) International publication number: WO 2021/006199

(57) **Abstract**

The problem to be solved by the present invention is to provide a new agent for preventing, suppressing the progression of symptoms of or the recurrence of, and/or treating hematological cancer.

The inventors of the present invention have diligently studied, and focused on bispecific proteins having a first arm specifically binding to PD-1 and a second arm specifically binding to CD3 (preferably, PD-1/CD3 bispecific antibody or antibody fragment thereof) as a substance to dissolve such a problem, and found that they are useful for preventing, suppressing the progression of symptoms of or the recurrence of, and/or treating hematological cancer.

## Description

### [Technical Field]

The present invention relates to a bispecific protein capable of specifically binding to PD-1 and CD3, respectively (hereinafter, may be abbreviated as a "PD-1/CD3 bispecific protein"), and a pharmaceutical therapeutic use thereof.

### [Background Art]

PD-1 is an immunosuppressive receptor belonging to an immunoglobulin family, and a molecule having a function of suppressing the immune activation signals of T-cells activated by stimulation through an antigen receptor. From analysis of PD-1 knock-out mice or the like, it is known that PD-1 signals play important roles in suppression of autoimmune diseases such as autoimmune dilated cardiomyopathy, lupus-like syndrome, autoimmune encephalomyelitis, systemic lupus erythematosus, graft-versus-host disease, type I diabetes mellitus, rheumatoid arthritis and the like. Accordingly, it is pointed out that a substance enhancing the PD-1 signal could be a prophylactic or therapeutic agent for autoimmune diseases.

It is known so far that there is a bispecific antibody recognizing PD-1 as a substance enhancing the PD-1 signal (Patent Literatures 1 to 3). This bispecific antibody is in the form which an antigen-recognition site of an antibody recognizing CD3, which is a member of a T-cell receptor complex, and an antigen-recognition site of an antibody recognizing PD-1 are linked to each other in genetic engineering method, and has an activity to enhance the inhibitory signal of PD-1 against the T-cell receptor complex by increasing the frequency of bringing PD-1 to the vicinity of the T-cell receptor complex. Furthermore, the Patent Literatures also state that the PD-1 bispecific antibody can be used for preventing or treating autoimmune diseases.

However, there is no report that the PD-1/CD3 bispecific protein is useful for preventing or treating hematological cancer.

### [Citation List]

### [Patent Literature]

Patent Literature 1: International Publication No. WO2003/011911
Patent Literature 2: International Publication No. WO2004/072286
Patent Literature 3: International Publication No. WO2013/022091

### [Summary of Invention]

### [Technical Problem]

The object of the present invention is to provide a new pharmaceutical agent for preventing, suppressing the progression of symptoms of or the recurrence of or treating hematological cancer.

### [Solution to Problem]

The inventors of the present invention diligently studied and focused on the PD-1/CD3 bispecific antibody of the present invention as a substance capable of solving the above-mentioned problem, and confirmed that these antibodies are expected to have effects on preventing or treating hematological cancer, and then completed the present invention.

That is, the present invention relates to the followings.
[1] An agent for preventing, suppressing the progression of symptoms of or the recurrence of and/or treating hematological cancer, containing a bispecific protein having a first arm specifically binding to PD-1 and a second arm specifically binding to CD3 (in the present specification, it presents the same meaning as "a bispecific protein capable of specifically binding to PD-1 and CD3, respectively", and may also be abbreviated as "a PD-1/CD3 bispecific protein") as an active ingredient.
[2] The agent according to the preceding item [1], wherein the bispecific protein is a bispecific antibody having the first arm specifically binding to PD-1 and the second arm specifically binding to CD3 (hereinafter, may be abbreviated as "a PD-1/CD3 bispecific antibody") or an antibody fragment thereof (hereinafter, these may be collectively abbreviated as "a PD-1/CD3 bispecific antibody or the like").
[3] The agent according to the preceding item [2], wherein the first arm specifically binding to PD-1, of the bispecific antibody or the like, contains any one of the VHs selected from
   (A) a heavy chain variable region (hereinafter, the "heavy chain variable region" may be abbreviated as "VH") having
      (a) a complementary determining region 1 of the heavy chain variable region (hereinafter, the "complementary determining region 1 of the heavy chain variable region" may be abbreviated as "VH-CDR1") comprising the amino acid sequence set forth in SEQ ID No. 6,
      (b) a complementary determining region 2 of the heavy chain variable region (hereinafter, the "complementary determining region 2 of the heavy chain variable region" may be abbreviated as "VH-CDR2") comprising the amino acid sequence set forth in SEQ ID No. 7, and
      (c) a complementary determining region 3 of the heavy chain variable region (hereinafter, the "complementary determining region 3 of the heavy chain variable region" may be abbreviated as "VH-CDR3") comprising the amino acid sequence set forth in SEQ ID No. 8,
   (B) a VH having
      (a) a VH-CDR1 comprising the amino acid sequence set forth in SEQ ID No. 9,
      (b) a VH-CDR2 comprising the amino acid sequence set forth in SEQ ID No. 10, and
      (c) a VH-CDR3 comprising the amino acid sequence set forth in SEQ ID No. 11,
   (C) a VH having
      (a) a VH-CDR1 comprising the amino acid sequence set forth in SEQ ID No. 12,
      (b) a VH-CDR2 comprising the amino acid sequence set forth in SEQ ID No. 13, and
      (c) a VH-CDR3 comprising the amino acid sequence set forth in SEQ ID No. 14,
   (D) a VH having
      (a) a VH-CDR1 comprising the amino acid sequence set forth in SEQ ID No. 15,
      (b) a VH-CDR2 comprising the amino acid sequence set forth in SEQ ID No. 16, and
      (c) a VH-CDR3 comprising the amino acid sequence set forth in SEQ ID No. 17, and
   (E) a VH having
      (a) a VH-CDR1 comprising the amino acid sequence set forth in SEQ ID No. 18,
      (b) a VH-CDR2 comprising the amino acid sequence set forth in SEQ ID No. 19, and
      (c) a VH-CDR3 comprising the amino acid sequence set forth in SEQ ID No. 20, and
      wherein one to five arbitrary amino acid residues may be substituted with other amino acids (preferably, conservative amino acids thereof) in any one or more of VH-CDRs selected from the VH-CDR1, VH-CDR2 and VH-CDR3 in the first arm specifically binding to PD-1, respectively.
[4] The agent according to the preceding item [2] or [3], wherein the second arm specifically binding to CD3, of the bispecific antibody or the like, contains a VH having
   (a) a VH-CDR1 comprising the amino acid sequence set forth in SEQ ID No.37,
   (b) a VH-CDR2 comprising the amino acid sequence set forth in SEQ ID No.38, and
   (c) a VH-CDR3 comprising the amino acid sequence set forth in SEQ ID No.39, and
   wherein one to five arbitrary amino acid residues may be substituted with other amino acids (preferably, conservative amino acids thereof) in any one or more of VH-CDRs selected from the VH-CDR1, VH-CDR2 and VH-CDR3 in the second arm specifically binding to CD3, respectively.
[5] The agent according to any one of the preceding items [2] to [4], wherein the first arm specifically binding to PD-1, of the bispecific antibody or the like, contains any one of the VHs selected from
   (A) the VH having
      (a) the VH-CDR1 comprising the amino acid sequence set forth in SEQ ID No. 6,
      (b) the VH-CDR2 comprising the amino acid sequence set forth in SEQ ID No. 7, and
      (c) the VH-CDR3 comprising the amino acid sequence set forth in SEQ ID No. 8,
   (B) the VH having
      (a) the VH-CDR1 comprising the amino acid sequence set forth in SEQ ID No. 9,
      (b) the VH-CDR2 comprising the amino acid sequence set forth in SEQ ID No. 10, and
      (c) the VH-CDR3 comprising the amino acid sequence set forth in SEQ ID No. 11,
   (C) the VH having
      (a) the VH-CDR1 comprising the amino acid sequence set forth in SEQ ID No. 12,
      (b) the VH-CDR2 comprising the amino acid sequence set forth in SEQ ID No. 13, and
      (c) the VH-CDR3 comprising the amino acid sequence set forth in SEQ ID No. 14,
   (D) the VH having
      (a) the VH-CDR1 comprising the amino acid sequence set forth in SEQ ID No. 15,
      (b) the VH-CDR2 comprising the amino acid sequence set forth in SEQ ID No. 16, and
      (c) the VH-CDR3 comprising the amino acid sequence set forth in SEQ ID No. 17, and
   (E) the VH having
      (a) the VH-CDR1 comprising an amino acid sequence set forth in SEQ ID No. 18,
      (b) the VH-CDR2 comprising an amino acid sequence set forth in SEQ ID No. 19, and
      (c) the VH-CDR3 comprising an amino acid sequence set forth in SEQ ID No. 20.
[6] The agent according to any one of the preceding items [2] to [5], wherein the second arm specifically binding to CD3, of the bispecific antibody or the like, contains the VH having
   (a) the VH-CDR1 comprising the amino acid sequence set forth in SEQ ID No.37,
   (b) the VH-CDR2 comprising the amino acid sequence set forth in SEQ ID No.38, and
   (c) the VH-CDR3 comprising the amino acid sequence set forth in SEQ ID No.39.
[7] The agent according to the preceding item [2], wherein (i) the VH of the first arm specifically binding to PD-1, of the bispecific antibody or the like, has
   (a) the VH-CDR1 comprising the amino acid sequence set forth in SEQ ID No. 6,
   (b) the VH-CDR2 comprising the amino acid sequence set forth in SEQ ID No. 7, and
   (c) the VH-CDR3 comprising the amino acid sequence set forth in SEQ ID No. 8, and
   (ii) the VH of the second arm specifically binding to CD3, of the bispecific antibody or the like, has
   (a) the VH-CDR1 comprising the amino acid sequence set forth in SEQ ID No. 37,
   (b) the VH-CDR2 comprising the amino acid sequence set forth in SEQ ID No. 38, and
   (c) the VH-CDR3 comprising the amino acid sequence set forth in SEQ ID No. 39.
[8] The agent according to the preceding item [2], wherein (i) the VH of the first arm specifically binding to PD-1, of the bispecific antibody or the like, has
   (a) the VH-CDR1 comprising the amino acid sequence set forth in SEQ ID No. 9,
   (b) the VH-CDR2 comprising the amino acid sequence set forth in SEQ ID No. 10, and
   (c) the VH-CDR3 comprising the amino acid sequence set forth in SEQ ID No. 11, and
   (ii) the VH of the second arm specifically binding to CD3, of the bispecific antibody or the like, has
   (a) the VH-CDR1 comprising the amino acid sequence set forth in SEQ ID No. 37,
   (b) the VH-CDR2 comprising the amino acid sequence set forth in SEQ ID No. 38, and
   (c) the VH-CDR3 comprising the amino acid sequence set forth in SEQ ID No. 39.
[9] The agent according to the preceding item [2], wherein (i) the VH of the first arm specifically binding to PD-1, of the bispecific antibody or the like, has
   (a) the VH-CDR1 comprising the amino acid sequence set forth in SEQ ID No. 12,
   (b) the VH-CDR2 comprising the amino acid sequence set forth in SEQ ID No. 13, and
   (c) the VH-CDR3 comprising the amino acid sequence set forth in SEQ ID No. 14, and
   (ii) the VH of the second arm specifically binding to CD3, of the bispecific antibody or the like, has
   (a) the VH-CDR1 comprising the amino acid sequence set forth in SEQ ID No. 37,
   (b) the VH-CDR2 comprising the amino acid sequence set forth in SEQ ID No. 38, and
   (c) the VH-CDR3 comprising the amino acid sequence set forth in SEQ ID No. 39.
[10] The agent according to the preceding item [2], wherein (i) the VH of the first arm specifically binding to PD-1, of the bispecific antibody or the like, has
   (a) the VH-CDR1 comprising the amino acid sequence set forth in SEQ ID No. 15,
   (b) the VH-CDR2 comprising the amino acid sequence set forth in SEQ ID No. 16, and
   (c) the VH-CDR3 comprising the amino acid sequence set forth in SEQ ID No. 17, and
   (ii) the VH of the second arm specifically binding to CD3, of the bispecific antibody or the like, has
   (a) the VH-CDR1 comprising the amino acid sequence set forth in SEQ ID No. 37,
   (b) the VH-CDR2 comprising the amino acid sequence set forth in SEQ ID No. 38, and
   (c) the VH-CDR3 comprising the amino acid sequence set forth in SEQ ID No. 39.
[11] The agent according to the preceding item [2], wherein (i) the VH of the first arm specifically binding to PD-1, of the bispecific antibody or the like, has
   (a) the VH-CDR1 comprising the amino acid sequence set forth in SEQ ID No. 18,
   (b) the VH-CDR2 comprising the amino acid sequence set forth in SEQ ID No. 19, and
   (c) the VH-CDR3 comprising the amino acid sequence set forth in SEQ ID No. 20, and
   (ii) the VH of the second arm specifically binding to CD3, of the bispecific antibody or the like, has
   (a) the VH-CDR1 comprising the amino acid sequence set forth in SEQ ID No. 37,
   (b) the VH-CDR2 comprising the amino acid sequence set forth in SEQ ID No. 38, and
   (c) the VH-CDR3 comprising the amino acid sequence set forth in SEQ ID No. 39.
[12] An agent for preventing, suppressing the progression of symptoms of or the recurrence of and/or treating hematological cancer, containing a bispecific antibody or an antibody fragment thereof, having a first arm specifically binding to PD-1 and a second arm specifically binding to CD3 as an active ingredient, wherein the first arm specifically binding to PD-1 contains a VH having
   (a) a VH-CDR1 comprising the amino acid sequence represented by HYJ¹LH [wherein J¹ represents G (glycine) or A (alanine), and an alphabet represented by J¹ or other alphabets represent one-letter amino acid abbreviations, respectively],
   (b) a VH-CDR2 comprising the amino acid sequence represented by WJ²NTNTU²NPTX²AQGFTG [wherein J² represents L (leucine) or I (isoleucine), U² represents E (glutamic acid) or G (glycine), X² represents F (phenylalanine) or Y (tyrosine), and an alphabet represented by J², U² or X², or other alphabets represent the same as the above, respectively], and
   (c) a VH-CDR3 comprising the amino acid sequence represented by GDJ³VVPTTIWNYYU³X³MZ³V [wherein J³ represents M (methionine) or L (leucine), U³ represents H (histidine) or Y (tyrosine), X³ represents F (phenylalanine) or Y (tyrosine), Z³ represents D (aspartic acid) or E (glutamic acid), and an alphabet represented by J³, U³, X³ or Z³, or other alphabets represent the same as the above, respectively], and
   wherein the second arm specifically binding to CD3 contains the VH having
   (a) the VH-CDR1 comprising the amino acid sequence set forth in SEQ ID No. 37,
   (b) the VH-CDR2 comprising the amino acid sequence set forth in SEQ ID No. 38, and
   (c) the VH-CDR3 comprising the amino acid sequence set forth in SEQ ID No. 39.
[13] The agent according to the preceding item [12], wherein
   (a) J¹ represents G (glycine), J² represents L (leucine), U² represents E (glutamic acid), X² represents F (phenylalanine), J³ represents M (methionine), U³ represents H (histidine), X³ represents F (phenylalanine), and Z³ represents D (aspartic acid),
   (b) J¹ represents G (glycine), J² represents I (isoleucine), U² represents G (glycine), X² represents Y (tyrosine), J³ represents L (leucine), U³ represents H (histidine), X³ represents Y (tyrosine), and Z³ represents E (glutamic acid),
   (c) J¹ represents A (alanine), J² represents L (leucine), U² represents E (glutamic acid), X² represents Y (tyrosine), J³ represents M (methionine), U³ represents Y (tyrosine), X³ represents Y (tyrosine), and Z³ represents D (aspartic acid), or
   (d) J¹ represents A (alanine), J² represents L (leucine), U² represents E (glutamic acid), X² represents F (phenylalanine), J³ represents M (methionine), U³ represents H (histidine), X³ represents F (phenylalanine), and Z³ represents D (aspartic acid).
[14] The agent according to any one of the preceding items [2] to [13], wherein the framework region 1 (hereinafter, may be abbreviated as "FR1"), the framework region 2 (hereinafter, may be abbreviated as "FR2") and the framework region 3 (hereinafter, may be abbreviated as "FR3") in a framework region (hereinafter, "framework" may be abbreviated as "FR") within the VH of the first arm specifically binding to PD-1, of the bispecific antibody or the like, correspond to amino acid sequences encoded by the germ-line V gene IGHV7-4-1 or gene thereof with somatic mutation(s), respectively.
[15] The agent according to the preceding item [14], wherein the framework region 4 (hereinafter, "the framework 4" may be abbreviated as "FR4") in the VH of the first arm specifically binding to PD-1, of the bispecific antibody or the like, comprises an amino acid sequence (excluding an amino acid sequence included in the VH-CDR3 region) encoded by the germ-line J gene JH6c or gene thereof with somatic mutation(s).
[16] The agent according to the preceding item [14] or [15], wherein the FR region in the VH of the first arm specifically binding to PD-1 is encoded by the germ-line V gene IGHV7-4-1 which may have somatic mutation(s), and has the FR1 region in which in the amino acid sequence set forth in SEQ ID No. 21, by the somatic mutation(s), lysine at position 13 is or may be substituted with glutamine, alanine at position 16 is or may be substituted with valine, or lysine at position 19 is or may be substituted with methionine, respectively, or which is or may be carried out in an arbitrary combination of a plurality thereof.
[17] The agent according to any one of the preceding items [14] to [16], wherein the FR region in the VH of the first arm specifically binding to PD-1 is encoded by the germ-line V gene IGHV7-4-1 which may have somatic mutation(s), and has the FR2 region in which by the somatic mutation(s), valine at position 37 in the amino acid sequence set forth in SEQ ID No. 21 is or may be substituted with leucine.
[18] The agent according to any one of the preceding items [14] to [17], wherein the FR region in the VH of the first arm specifically binding to PD-1 is encoded by the germ-line V gene IGHV7-4-1 which may have somatic mutation(s), and has the FR3 region in which in the amino acid sequence set forth in SEQ ID No. 21, by the somatic mutation(s), serine at position 77 is or may be substituted with threonine or cysteine at position 84 is or may be substituted with serine or asparagine, respectively, or which is or may be carried out in an arbitrary combination of a plurality thereof.
[19] The agent according to any one of the preceding items [14] to [18], wherein the FR4 region in the VH of the first arm specifically binding to PD-1 is encoded by the germ-line J gene JH6c which may have somatic mutation(s) (excluding a gene region encoding VH-CDR3 region), and wherein in the amino acid sequence (Trp-Gly-Lys-Gly-Thr-Thr^{∗}-Val-Thr-Val-Ser-Ser)(SEQ ID No. 41) of the FR4 region, lysine (Lys) is or may be substituted with glutamine or asparagine and/or threonine (Thr) marked with an asterisk is or may be substituted with leucine.
[20] The agent according to any one of the preceding items [2] to [19], wherein the VH of the first arm specifically binding to PD-1 comprises the amino acid sequence set forth in any one selected from SEQ ID No. 1, SEQ ID No. 2, SEQ ID No. 3, SEQ ID No. 4 and SEQ ID No. 5, or an amino acid sequence having an identity of at least 80%, 90%, 95%, 98% or 99% to the amino acid sequence of the same VH
[21] The agent according to any one of the preceding items [2] to [11], wherein the VH of the first arm specifically binding to PD-1 comprises the amino acid sequence set forth in any one selected from SEQ ID No. 1, SEQ ID No. 2, SEQ ID No. 3, SEQ ID No. 4 and SEQ ID No. 5.
[22] The agent according to any one of the preceding items [2] to [21], wherein the VH of the second arm specifically binding to CD3 comprises the amino acid sequence set forth in SEQ ID No. 36 or an amino acid sequence having an identity of at least 80%, 90%, 95%, 98% or 99% to the amino acid sequence of the same VH
[23] The agent according to any one of the preceding items [2] to [21], wherein the VH of the second arm specifically binding to CD3 comprises the amino acid sequence set forth in SEQ ID No. 36.
[24] The agent according to the preceding items [2] to [6], wherein the VH of the first arm specifically binding to PD-1 comprises the amino acid sequence set forth in any one selected from SEQ ID No. 1, SEQ ID No. 2, SEQ ID No. 3, SEQ ID No. 4 and SEQ ID No. 5, and the VH of the second arm specifically binding to CD3 comprises the amino acid sequence set forth in SEQ ID No. 36.
[25] An agent for preventing, suppressing the progression of symptoms of or the recurrence of and/or treating hematological cancer, containing a bispecific antibody or an antibody fragment thereof, having a first arm specifically binding to PD-1 and a second arm specifically binding to CD3 as an active ingredient,
   wherein the VH of the first arm specifically binding to PD-1 comprises the amino acid sequence set forth in any one selected from SEQ ID No. 1, SEQ ID No. 2, SEQ ID No. 3, SEQ ID No. 4 and SEQ ID No. 5, or an amino acid sequence having an identity of at least 80%, 90%, 95%, 98% or 99% to the amino acid sequence of the same VH, and the VH of the second arm specifically binding to CD3 comprises the amino acid sequence set forth in SEQ ID No. 36 or an amino acid sequence having an identity of at least 80%, 90%, 95%, 98% or 99% to the amino acid sequence of the same VH
[26] The agent according to the preceding items [2] to [7], wherein the VH of the first arm specifically binding to PD-1 comprises the amino acid sequence set forth in SEQ ID No. 1, and the VH of the second arm specifically binding to CD3 comprises the amino acid sequence set forth in SEQ ID No. 36.
[27] The agent according to any one of the preceding items [2] to [6] and [8], wherein the VH of the first arm specifically binding to PD-1 comprises the amino acid sequence set forth in SEQ ID No. 2, and the VH of the second arm specifically binding to CD3 comprises the amino acid sequence set forth in SEQ ID No. 36.
[28] The agent according to any one of the preceding items [2] to [6] and [9], wherein the VH of the first arm specifically binding to PD-1 comprises the amino acid sequence set forth in SEQ ID No. 3, and the VH of the second arm specifically binding to CD3 comprises the amino acid sequence set forth in SEQ ID No. 36.
[29] The agent according to any one of the preceding items [2] to [6] and [10], wherein the VH of the first arm specifically binding to PD-1 comprises the amino acid sequence set forth in SEQ ID No. 4, and the VH of the second arm specifically binding to CD3 comprises the amino acid sequence set forth in SEQ ID No. 36.
[30] The agent according to any one of the preceding items [2] to [6] and [11], wherein the VH of the first arm specifically binding to PD-1 comprises the amino acid sequence set forth in SEQ ID No. 5, and the VH of the second arm specifically binding to CD3 comprises the amino acid sequence set forth in SEQ ID No. 36.
[31] The agent according to any one of the preceding items [2] to [30], wherein the first arm specifically binding to PD-1 and/or the second arm specifically binding to CD3, of the bispecific antibody or the like, have/has a light chain variable region (hereinafter, the "light chain variable region" may be abbreviated as "VL") having
   (a) a complementary determining region 1 of light chain variable region (hereinafter, the "complementary determining region 1 of light chain variable region" may be abbreviated as "VL-CDR1") comprising the amino acid sequence set forth in SEQ ID No. 26,
   (b) a complementary determining region 2 of light chain variable region (hereinafter, the "complementary determining region 2 of light chain variable region" may be abbreviated as "VL-CDR2") comprising the amino acid sequence set forth in SEQ ID No. 27, and (c) a complementary determining region 3 of light chain variable region (hereinafter, the "complementary determining region 3 of light chain variable region" may be abbreviated as "VL-CDR3") comprising the amino acid sequence set forth in SEQ ID No. 28, respectively.
[32] The agent according to any one of the preceding items [2] to [30], wherein the first arm specifically binding to PD-1 and/or the second arm specifically binding to CD3, of the bispecific antibody or the like, have/has the VL comprising the amino acid sequence set forth in SEQ ID No. 25, respectively.
[33] An agent for preventing, suppressing the progression of symptoms of or the recurrence of and/or treating hematological cancer, containing a bispecific antibody or an antibody fragment thereof, having a first arm specifically binding to PD-1 and a second arm specifically binding to CD3 as an active ingredient, wherein
   (A) the first arm specifically binding to PD-1 has a VH comprising the amino acid sequence set forth in any one selected from SEQ ID No. 1, SEQ ID No. 2, SEQ ID No. 3, SEQ ID No. 4 and SEQ ID No. 5, and a VL comprising the amino acid sequence set forth in SEQ ID No. 25, and
   (B) the second arm specifically binding to CD3 has a VH comprising the amino acid sequence set forth in SEQ ID No. 36, and a VL comprising the amino acid sequence set forth in SEQ ID No. 25.
[34] An agent for preventing, suppressing the progression of symptoms of or the recurrence of and/or treating hematological cancer, containing a bispecific antibody or an antibody fragment thereof, having a first arm specifically binding to PD-1 and a second arm specifically binding to CD3 as an active ingredient,
   wherein the first arm specifically binding to PD-1 cross-competes for (1) the binding to PD-1 with the first arm specifically binding to PD-1 having a VH comprising the amino acid sequence set forth in any one selected from SEQ ID No. 1, SEQ ID No. 2, SEQ ID No. 3, SEQ ID No. 4 and SEQ ID No. 5, and a VL comprising the amino acid sequence set forth in SEQ ID No. 25, or (2) the binding to PD-1 with a variable region of monoclonal antibody specifically binding to PD-1 having the same VH and VL.
[35] An agent for preventing, suppressing the progression of symptoms of or the recurrence of and/or treating hematological cancer, containing a bispecific antibody or an antibody fragment thereof, having a first arm specifically binding to PD-1 and a second arm specifically binding to CD3 as an active ingredient,
   wherein the binding to PD-1 with the first arm specifically binding to PD-1 is cross-competed by (1) the first arm specifically binding to PD-1 having a VH comprising the amino acid sequence set forth in any one selected from SEQ ID No. 1, SEQ ID No. 2, SEQ ID No. 3, SEQ ID No. 4 and SEQ ID No. 5, and a VL comprising the amino acid sequence set forth in SEQ ID No. 25, or (2) a variable region of monoclonal antibody specifically binding to PD-1 having the same VH and VL.
[36] The agent according to the preceding item [34] or [35], wherein the second arm specifically binding to CD3 further cross-competes for (1) the binding to CD3 with the second arm specifically binding to CD3 having the VH comprising the amino acid sequence set forth in SEQ ID No. 36, and the VL comprising the amino acid sequence set forth in SEQ ID No. 25, or (2) the binding to CD3 with a variable region of monoclonal antibody specifically binding to CD3 having the same VH and VL.
[37] The agent according to the preceding item [34] or [35], wherein the second arm specifically binding to CD3 has the VH having
   (a) the VH-CDR1 comprising the amino acid sequence set forth in SEQ ID No.37,
   (b) theVH-CDR2 comprising the amino acid sequence set forth in SEQ ID No.38, and (c) the VH-CDR3 comprising the amino acid sequence set forth in SEQ ID No.39.
[38] The agent according to the preceding item [34] or [35], wherein the VH of the second arm specifically binding to CD3 comprises the amino acid sequence set forth in SEQ ID No. 36, or an amino acid sequence having an identity of at least 80%, 90%, 95%, 98% or 99% to the amino acid sequence of the same VH
[39] The agent according to the preceding item [34] or [35], wherein the VH of the second arm specifically binding to CD3 comprises the amino acid sequence set forth in SEQ ID No. 36.
[40] The agent according to any one of the preceding items [34], [35], and [37] to [39], wherein the second arm specifically binding to CD3 has the VL having
   (a) the VL-CDR1 comprising the amino acid sequence set forth in SEQ ID No. 26,
   (b) the VL-CDR2 comprising the amino acid sequence set forth in SEQ ID No. 27, and
   (c) the VL-CDR3 comprising the amino acid sequence set forth in SEQ ID No. 28.
[41] The agent according to any one of the preceding items [34], [35], and [37] to [39], wherein the second arm specifically binding to CD3 has the VL comprising the amino acid sequence set forth in SEQ ID No. 25.
[42] The agent according to any one of the preceding items [2] to [41], wherein the bispecific antibody is an IgG antibody.
[43] The agent according to the preceding item [42], wherein the IgG antibody in the preceding item [42] is an IgG₁ or IgG₄ antibody.
[44] The agent according to the preceding item [42], wherein the IgG antibody in the preceding item [42] is an IgG₁ antibody.
[45] The agent according to the preceding item [42], wherein the IgG antibody in the preceding item [42] is an IgG₄ antibody.
[46] The agent according to the preceding item [44], wherein the binding of the IgG₁ antibody to the Fc receptor is eliminated or attenuated.
[47] The agent according to the preceding item [44], wherein in two heavy chain constant regions of the IgG₁ antibody in the preceding item [44], each leucine at position 235 according to the EU numbering system is substituted with glycine, and/or each glycine at position 236 is substituted with arginine.
[48] The agent according to the preceding item [44], [46] or [47], wherein in a constant region of heavy chain having the VH of the first arm specifically binding to PD-1, both of leucine at position 351 and threonine at position 366 according to the EU numbering system are substituted with lysine, and in a constant region of heavy chain having the VH of the second arm specifically binding to CD3, leucine at position 351 is substituted with aspartic acid and leucine at position 368 is substituted with glutamic acid.
[49] The agent according to the preceding item [44], [46] or [47], wherein in the constant region of heavy chain having the VH of the first arm specifically binding to PD-1, leucine at position 351 according to the EU numbering system is substituted with aspartic acid and leucine at position 368 is substituted with glutamic acid, and in the constant region of heavy chain having the VH of the second arm specifically binding to CD3, both of leucine at position 351 and threonine at position 366 are substituted with lysine.
[50] The agent according to any one of the preceding items [44] and [46] to [49], wherein in two heavy chain constant regions of the IgG₁ antibody in any one of the preceding items [44] and [46] to [49], each lysine at position 447 according to the EU numbering system is deleted.
[51] The agent according to any one of the preceding items [42] to [50], wherein the binding to neonatal Fc receptor (hereinafter, may be abbreviated as "FcRn".) is eliminated or attenuated.
[52] The agent according to any one of the preceding items [44] and [46] to [51], wherein in two heavy chain constant regions of the IgG₁ antibody, (1) each methionine at position 252 according to the EU numbering system is substituted with glutamic acid, proline, arginine or aspartic acid, (2) each asparagine at position 434 according to the EU numbering system is substituted with leucine and/or (3) each glutamine at position 438 according to the EU numbering system is substituted with glutamic acid.
[53] The agent according to any one of the preceding items [44] and [46] to [52], wherein each methionine at position 252 according to the EU numbering system in two heavy chain constant regions of the IgG₁ antibody is substituted with aspartic acid.
[54] The agent according to the preceding item [52] or [53], wherein the half-life in blood of the IgG₁ antibody of the preceding item [52] or [53] is shortened compared to the original antibody without the same amino acid substitutions.
[55] The agent according to the preceding item [52] or [53], wherein the half-life in blood of the IgG₁ antibody of the preceding item [52] or [53] is shortened at least 50%, at least 60%, at least 70%, at least 80% or at least 90%, compared to the original antibody without the same amino acid substitutions.
[56] The agent according to the preceding item [45], wherein in two heavy chain constant regions of the IgG₄ antibody in the preceding item [45], each serine at position 228 according to the EU numbering system is substituted with proline.
[57] The agent according to any one of the preceding items [2] to [42], [44], [46], [47] and [49] to [55], wherein the heavy chain having the VH of the first arm specifically binding to PD-1 has a heavy chain constant region comprising the amino acid sequence set forth in any one selected from SEQ ID No. 23, SEQ ID No. 42, SEQ ID No. 43, SEQ ID No. 44, SEQ ID No. 45, SEQ ID No. 46 and SEQ ID No. 47.
[58] The agent according to any one of the preceding items [2] to [42], [44], [46], [47], [49] to [55] and [57], wherein the heavy chain having the VH of the second arm specifically binding to CD3 has a heavy chain constant region comprising the amino acid sequence set forth in any one selected from SEQ ID No. 24, SEQ ID No. 48, SEQ ID No. 49, SEQ ID No. 50, SEQ ID No. 51, SEQ ID No. 52 and SEQ ID No. 53.
[59] The agent according to any one of the preceding items [2] to [58], wherein the light chain having the VL of the first arm specifically binding to PD-1 and/or the light chain having the VL of the second arm specifically binding to CD3 have/has a light chain constant region comprising the amino acid sequence set forth in SEQ ID No. 29.
[60] An agent for preventing, suppressing the progression of symptoms of or the recurrence of and/or treating hematological cancer, containing a bispecific antibody or an antibody fragment thereof, having a first arm specifically binding to PD-1 and a second arm specifically binding to CD3 as an active ingredient, wherein
   (A) a heavy chain having a VH of the first arm specifically binding to PD-1 has a VH comprising the amino acid sequence set forth in any one selected from SEQ ID No. 1, SEQ ID No. 2, SEQ ID No. 3, SEQ ID No. 4 and SEQ ID No. 5 and a heavy chain constant region comprising the amino acid sequence set forth in any one selected from SEQ ID No. 23, SEQ ID No. 42, SEQ ID No. 43, SEQ ID No. 44, SEQ ID No. 45, SEQ ID No. 46 and SEQ ID No. 47,
   (B) a light chain having a VL of the first arm specifically binding to PD-1 has a VL comprising the amino acid sequence set forth in SEQ ID No. 25 and a light chain constant region comprising the amino acid sequence set forth in SEQ ID No. 29,
   (C) a heavy chain having a VH of the second arm specifically binding to CD3 has a VH comprising the amino acid sequence set forth in SEQ ID No. 36 and a heavy chain constant region comprising the amino acid sequence set forth in any one selected from SEQ ID No. 24, SEQ ID No. 48, SEQ ID No. 49, SEQ ID No. 50, SEQ ID No. 51, SEQ ID No. 52 and SEQ ID No. 53, and
   (D) a light chain having a VL of the second arm specifically binding to CD3 has a VL comprising the amino acid sequence set forth in SEQ ID No. 25 and a light chain constant region comprising the amino acid sequence set forth in SEQ ID No. 29.
[61] The agent according to the preceding item [2], wherein the first arm specifically binding to PD-1, of the bispecific antibody or the like, has a VH and VL of an anti-PD-1 antibody and the second arm specifically binding to CD3, of the bispecific antibody or the like, has a VH and VL of an anti-CD3 antibody.
[62] The agent according to the preceding item [61], wherein the anti-PD-1 antibody is selected from any of Nivolumab, Cemiplimab, Pembrolizumab, Spartalizumab, Tislelizumab, Dostarlimab, Toripalimab, Camrelizumab, Genolimzumab, Sintilimab, Lodapolimab, Retifanlimab, Balstilimab, Serplulimab, Budigalimab, Prolgolimab, Sasanlimab, Cetrelimab, Zimberelimab, AMP-514, STI-A1110, ENUM 388D4, ENUM 244C8, GLS010, CS1003, BAT-1306, AK105, AK103, BI 754091, LZM009, CMAB819, Sym021, GB226, SSI-361, JY034, HX008 ISU106 and CX-188.
[63] The agent according to the preceding items [61] or [62], wherein the anti-CD3 antibody is selected from any of Foralumab, Muromonab-CD3, Otelixizumab, Teplizumab and Visilizumab.
[64] The agent according to any one of the preceding items [1] to [63], wherein PD-1 and CD3 are human PD-1 and human CD3, respectively.
[65] The agent according to any one of the preceding items [1] to [64], wherein CD3 is CD3ε.
[66] The agent according to any one of the preceding items [2] to [65], wherein the bispecific antibody is a monoclonal antibody.
[67] The agent according to any one of the preceding items [2] to [66], wherein the bispecific antibody is an isolated antibody.
[68] The agent according to the preceding item [1], wherein the bispecific protein is a Scaffold molecule or modified peptide.
[69] The agent according to the preceding item [68], wherein the Scaffold molecule is in the form of Adnectin, Affibody (registered trademark), Anticalin (registered trademark), Avimer, DARPin, LRRP, Affilin (registered trademark), Affitin or Fynomer, and the modified peptide is in the form of a special cyclic peptide, Addbody (registered trademark) or Mirabody (registered trademark).
[70] The agent according to any one of the preceding items [1] to [69], wherein the bispecific protein or the bispecific antibody or antibody fragment thereof specifically binds to PD-1 expressed on a hematological cancer cell as a target cell and CD3 expressed on a lymphocyte as an effector cell (e.g., T cell lymphocytes), respectively.
[71] The agent according to any one of the preceding items [1] to [69], wherein the bispecific protein or the bispecific antibody or antibody fragment thereof specifically binds to PD-1 and CD3 expressed on a T cell-derived cancer cell as a target cell, respectively.
[72] The agent according to any one of the preceding items [1] to [70], wherein the first arm specifically binding to PD-1 allows the interaction between PD-1 and PD-L1.
[73] The agent according to any one of the preceding items [1] to [72], wherein hematological cancer is/are one or more selected from multiple myeloma, malignant lymphoma, leukemia, primary central nervous system lymphoma and myeloproliferative syndrome.
[74] The agent according to the preceding item [73], wherein hematological cancer is malignant lymphoma, further which is Non-Hodgkin's lymphoma or Hodgkin's lymphoma.
[75] The agent according to the preceding item [74], wherein malignant lymphoma is Non-Hodgkin's lymphoma, further which is B-cell non-Hodgkin's lymphoma or T/NK-cell non-Hodgkin's lymphoma.
[76] The agent according to the preceding item [75], wherein Non-Hodgkin's lymphoma is B-cell non-Hodgkin's lymphoma, further which is precursor B-cell lymphoblastic lymphoma, precursor B-cell acute lymphoblastic leukemia, chronic B-lymphoid leukemia, B-cell prolymphocytic leukemia, lymphoplasmacytic lymphoma, nodal marginal zone B-cell lymphoma, extranodal marginal zone B-cell lymphoma, primary splenic marginal zone B-cell lymphoma, hairy cell leukemia, hairly cell leukemia-variant, follicular lymphoma, pediatric type follicular lymphoma, diffuse large B cell lymphoma, diffuse large B-cell lymphoma, not otherwise specified, splenic diffuse red pulp small B-cell lymphoma, lymphoplasmacytic lymphoma, primary mediastinal large B-cell lymphoma, primary effusion lymphoma, Burkitt's lymphoma, mantle cell lymphoma, monoclonal B-cell lymphocytosis, splenic B-cell lymphoma/leukemia, unclassifiable, IgM monoclonal gammopathy of undetermined significance, µ heavy chain disease, λ heavy chain disease, α heavy chain disease, plasma cell myeloma, solitary plasmacytoma of bone, extraosseous plasmacytoma, monoclonal immunoglobulin deposition disease, large B-cell lymphoma with IRF4 rearrangement, primary cutaneous follicle center lymphoma, T-cell/histiocyte-rich large B-cell lymphoma, primary diffuse large B-cell lymphoma of the central nervous system, primary cutaneous diffuse large B-cell lymphoma, leg type, EBV positive diffuse large B-cell lymphoma, not otherwise specified, EBV positive mucocutaneous ulcer, diffuse large B-cell lymphoma associated with chronic inflammation, lymphomatoid granulomatosis, intravascular large B-cell lymphoma, ALK positive large B-cell lymphoma, plasmablastic lymphoma, primary effusion lymphoma, HHV8 positive diffuse large B-cell lymphoma, not otherwise specified, Burkitt-like lymphoma with 11q aberration, high-grade B-cell lymphoma, with MYC and BCL2 and/or BCL6 rearrangement, high-grade B-cell lymphoma, not otherwise specified or B-cell lymphoma, unclassifiable, with intermediate features between diffuse large B-cell lymphoma and classical Hodgkin lymphoma.
[77] The agent according to the preceding item [75], wherein Non-Hodgkin's lymphoma is T/NK-cell non-Hodgkin's lymphoma, further which is precursor T-cell lymphoblastic lymphoma, chronic T-cell lymphocytic leukemia, T-cell acute lymphocytic leukemia (T-cell lymphoblastic leukemia), T-cell large granular lymphoblastic lymphoma, NK-cell large granular leukemia, aggressive NK-cell leukemia, peripheral T-cell lymphoma, peripheral T-cell lymphoma, not otherwise specified, unclassifiable peripheral T-cell lymphoma, angioimmunoblastic T-cell lymphoma, (CD30-positive) anaplastic large cell lymphoma, angiocentric lymphoma, intestinal T-cell lymphoma, enteropathy-type T-cell lymphoma, hepatosplenic gamma-delta T cell lymphoma, subcutaneous panniculitis-like T-cell lymphoma, mycosis fungoides, Sezary syndrome, Hodgkin-like/Hodgkin-related anaplastic large cell lymphoma, extranodal NK/T-cell lymphoma, adult T cell lymphoma, T-cell prolymphocytic leukemia, chronic lymphoproliferative disorder of NK-cells, systemic EBV positive T-cell lymphoma of childhood, hydroa vacciniforme-like lymphoproliferative disorder, extranodal NK/T-cell lymphoma, nasal type, enteropathy-associated T-cell lymphoma, monomorphic epitheliotropic intestinal T-cell lymphoma, indolent T-cell lymphoproliferative disorder of the GI tract, hepatosplenic T-cell lymphoma, primary cutaneous CD30 positive T-cell lymphoproliferative disorders, lymphomatoid papulosis, primary cutaneous anaplastic large cell lymphoma, primary cutaneous gamma-delta T-cell lymphoma, primary cutaneous CD8 positive aggressive epidermotropic cytotoxic T-cell lymphoma, primary cutaneous acral CD8 positive T-cell lymphoma, primary cutaneous CD4 positive small/medium T-cell lymphoproliferative disorder, follicular T-cell lymphoma, Nodal peripheral T-cell lymphoma with TFH phenotype, anaplastic large cell lymphoma, ALK positive, anaplastic large cell lymphoma, ALK negative or breast implant-associated anaplastic large-cell lymphoma.
[78] The agent according to the preceding item [74], wherein malignant lymphoma is Hodgkin's lymphoma, further which is classical Hodgkin's lymphoma (e.g., nodular sclerosis, mixed cellularity, lymphocyte-rich and lymphopenic) or nodular lymphocyte predominant Hodgkin's lymphoma.
[79] The agent according to the preceding item [73], wherein hematological cancer is leukemia, further which is acute myelogenous leukemia, acute promyelocytic leukemia, acute lymphoblastic leukemia, chronic lymphocytic leukemia, myelodysplastic syndrome or chronic myelogenous leukemia.
[80] The agent according to any one of the preceding items [1] to [79], wherein hematological cancer is pediatric hematological cancer.
[81] The agent according to the preceding item [75], wherein Non-Hodgkin's lymphoma is T/NK-cell non-Hodgkin's lymphoma, further which is peripheral T-cell lymphoma or adult T cell lymphoma.
[82] The agent according to any one of the preceding items [1] to [81], wherein hematological cancer is one on which therapeutic effect with other anti-cancer drugs is insufficient or is not sufficient.
[83] The agent according to any one of the preceding items [1] to [82], wherein hematological cancer was exacerbated after treatment with other anti-cancer drugs.
[84] The agent according to any one of the preceding items [1] to [81], wherein a patient having hematologic cancer has been untreated with any other anti-cancer drugs.
[85] The agent according to any one of the preceding items [1] to [84], wherein hematological cancer is relapsed and/or refractory.
[86] The agent according to any one of the preceding items [1] to [85], which further is used in combination with other anti-cancer drugs.
[87] The agent according to the preceding item [82], [83] or [86], wherein other anti-cancer drug(s) is/are one or more drugs selected from an alkylating agent, platinum agent, antimetabolite (e.g., antifolate, pyridine metabolism inhibitor and purine metabolism inhibitor), ribonucleotide reductase inhibitor, nucleotide analog, topoisomerase inhibitor, microtubule polymerization inhibitor, microtubule depolymerization inhibitor, antitumor antibiotic, cytokine preparation, molecular targeting drug and cancer immunotherapeutic drug.
[88] The agent according to the preceding item [86] or [87], which is administered to a patient to which any one or more of other anti-cancer drugs of the preceding item [87] are administered.
[89] The agent according to the preceding item [86] or [87], which is administered after administration of any one or more of other anti-cancer drugs of the preceding item [87].
[90] The agent according to the preceding item [86] or [87], which is administered prior to administration of any one or more of other anti-cancer drugs of the preceding item [87].
[91] The agent according to any one of the preceding items [1] to [85], which is administered along with a steroid drug.
[92] The agent according to any one of the preceding items [1] to [85], which is administered after administration of a steroid drug.
[93] The agent according to any one of the preceding items [1] to [85], which is for intravenous injection.
[94] A hematological cancer cell growth inhibitor containing the bispecific protein or the bispecific antibody or antibody fragment thereof according to any one of the preceding items [1] to [72] as an active ingredient.
   [1-1] A method for preventing, suppressing the progression of symptoms of or the recurrence of and/or treating hematological cancer, comprising administering an effective amount of the bispecific protein or the bispecific antibody or antibody fragment thereof of any one of the preceding items [1] to [72] to a patient.
   [2-1] A bispecific protein or a bispecific antibody or an antibody fragment thereof selected from the preceding items [1] to [72] in use for preventing, suppressing the progression of symptoms of or the recurrence of and/or treating hematological cancer.
   [3-1] Use of a bispecific protein or a bispecific antibody or an antibody fragment thereof selected from the preceding items [1] to [72] for manufacturing a drug for preventing, suppressing the progression of symptoms of or the recurrence of and/or treating hematological cancer.
   [4-1] A pharmaceutical composition containing a bispecific antibody or an antibody fragment thereof, having a first arm specifically binding to PD-1 and a second arm specifically binding to CD3 as an active ingredient,
      wherein the first arm specifically binding to PD-1 has any one of the VH selected from
      (A) a VH having
         (a) a VH-CDR1 comprising the amino acid sequence set forth in SEQ ID No. 6,
         (b) a VH-CDR2 comprising the amino acid sequence set forth in SEQ ID No. 7, and
         (c) a VH-CDR3 comprising the amino acid sequence set forth in SEQ ID No. 8,
      (B) a VH having
         (a) a VH-CDR1 comprising the amino acid sequence set forth in SEQ ID No. 9,
         (b) a VH-CDR2 comprising the amino acid sequence set forth in SEQ ID No. 10, and
         (c) aVH-CDR3 comprising the amino acid sequence set forth in SEQ ID No. 11,
      (C) a VH having
         (a) a VH-CDR1 comprising the amino acid sequence set forth in SEQ ID No. 12,
         (b) a VH-CDR2 comprising the amino acid sequence set forth in SEQ ID No. 13, and
         (c) a VH-CDR3 comprising the amino acid sequence set forth in SEQ ID No. 14,
      (D) a VH having
         (a) a VH-CDR1 comprising the amino acid sequence set forth in SEQ ID No. 15,
         (b) a VH-CDR2 comprising the amino acid sequence set forth in SEQ ID No. 16, and
         (c) a VH-CDR3 comprising the amino acid sequence set forth in SEQ ID No. 17, and
      (E) a VH having
         (a) a VH-CDR1 comprising the amino acid sequence set forth in SEQ ID No. 18,
         (b) a VH-CDR2 comprising the amino acid sequence set forth in SEQ ID No. 19, and
         (c) a VH-CDR3 comprising the amino acid sequence set forth in SEQ ID No. 20, and
      (F) a VL having
         (a) a VL-CDR1 comprising the amino acid sequence set forth in SEQ ID No. 26,
         (b) a VL-CDR2 comprising the amino acid sequence set forth in SEQ ID No. 27, and
         (c) a VL-CDR3 comprising the amino acid sequence set forth in SEQ ID No. 28, and the second arm specifically binding to CD3 has
            (A) a VH having
               (a) a VH-CDR1 comprising the amino acid sequence set forth in SEQ ID No.37,
               (b) a VH-CDR2 comprising the amino acid sequence set forth in SEQ ID No.38, and
               (c) a VH-CDR3 comprising the amino acid sequence set forth in SEQ ID No.39, and
            (B) a VL having
               (a) aVL-CDR1 comprising the amino acid sequence set forth in SEQ ID No. 26,
               (b) a VL-CDR2 comprising the amino acid sequence set forth in SEQ ID No. 27, and
               (c) a VL-CDR3 comprising the amino acid sequence set forth in SEQ ID No. 28.
   [4-2] The pharmaceutical composition according to the preceding item [4-1], wherein (i) the VH of the first arm specifically binding to PD-1 has
      (a) the VH-CDR1 comprising the amino acid sequence set forth in SEQ ID No. 6,
      (b) the VH-CDR2 comprising the amino acid sequence set forth in SEQ ID No. 7, and
      (c) the VH-CDR3 comprising the amino acid sequence set forth in SEQ ID No. 8, and
      (ii) the VH of the second arm specifically binding to CD3 has
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39.
   [4-3] The pharmaceutical composition according to the preceding item [4-1], wherein (i) the VH of the first arm specifically binding to PD-1 has
      (a) the VH-CDR1 comprising the amino acid sequence set forth in SEQ ID No. 9,
      (b) the VH-CDR2 comprising the amino acid sequence set forth in SEQ ID No. 10, and
      (c) the VH-CDR3 comprising the amino acid sequence set forth in SEQ ID No. 11, and
      (ii) the VH of the second arm specifically binding to CD3 has
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39.
   [4-4] The pharmaceutical composition according to the preceding item [4-1], wherein (i) the VH of the first arm specifically binding to PD-1 has
      (a) the VH-CDR1 comprising the amino acid sequence set forth in SEQ ID No. 12,
      (b) the VH-CDR2 comprising the amino acid sequence set forth in SEQ ID No. 13, and
      (c) the VH-CDR3 comprising the amino acid sequence set forth in SEQ ID No. 14, and
      (ii) the VH of the second arm specifically binding to CD3 has
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39.
   [4-5] The pharmaceutical composition according to the preceding item [4-1], wherein (i) the VH of the first arm specifically binding to PD-1 has
      (a) the VH-CDR1 comprising the amino acid sequence set forth in SEQ ID No. 15,
      (b) the VH-CDR2 comprising the amino acid sequence set forth in SEQ ID No. 16, and
      (c) the VH-CDR3 comprising the amino acid sequence set forth in SEQ ID No. 17, and
      (ii) the VH of the second arm specifically binding to CD3 has
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39.
   [4-6] The pharmaceutical composition according to the preceding item [4-1], wherein (i) the VH of the first arm specifically binding to PD-1 has
      (a) the VH-CDR1 comprising the amino acid sequence set forth in SEQ ID No. 18,
      (b) the VH-CDR2 comprising the amino acid sequence set forth in SEQ ID No. 19, and
      (c) the VH-CDR3 comprising the amino acid sequence set forth in SEQ ID No. 20, and
      (ii) the VH of the second arm specifically binding to CD3 has
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39.
   [4-7] The pharmaceutical composition according to the preceding item [4-1], wherein the VH of the first arm specifically binding to PD-1 comprises the amino acid sequence set forth in any one selected from SEQ ID No. 1, SEQ ID No. 2, SEQ ID No. 3, SEQ ID No. 4 and SEQ ID No. 5, and the VH of the second arm specifically binding to CD3 comprises the amino acid sequence set forth in SEQ ID No. 36.
   [4-8] The pharmaceutical composition according to the preceding item [4-1] or [4-2], wherein the VH of the first arm specifically binding to PD-1 comprises the amino acid sequence set forth in SEQ ID No. 1, and the VH of the second arm specifically binding to CD3 comprises the amino acid sequence set forth in SEQ ID No. 36.
   [4-9] The pharmaceutical composition according to the preceding item [4-1] or [4-3], wherein the VH of the first arm specifically binding to PD-1 comprises the amino acid sequence set forth in SEQ ID No. 2, and the VH of the second arm specifically binding to CD3 comprises the amino acid sequence set forth in SEQ ID No. 36.
   [4-10] The pharmaceutical composition according to the preceding item [4-1] or [4-4], wherein the VH of the first arm specifically binding to PD-1 comprises the amino acid sequence set forth in SEQ ID No. 3, and the VH of the second arm specifically binding to CD3 comprises the amino acid sequence set forth in SEQ ID No. 36.
   [4-11] The pharmaceutical composition according to the preceding item [4-1] or [4-5], wherein the VH of the first arm specifically binding to PD-1 comprises the amino acid sequence set forth in SEQ ID No. 4, and the VH of the second arm specifically binding to CD3 comprises the amino acid sequence set forth in SEQ ID No. 36.
   [4-12] The pharmaceutical composition according to the preceding item [4-1] tor [4-6], wherein the VH of the first arm specifically binding to PD-1 comprises the amino acid sequence set forth in SEQ ID No. 5, and the VH of the second arm specifically binding to CD3 comprises the amino acid sequence set forth in SEQ ID No. 36.
   [4-13] The pharmaceutical composition according to any one of the preceding items [4-1] to [4-12], wherein the first arm specifically binding to PD-1 and/or the second arm specifically binding to CD3 have/has the VL comprising an amino acid sequence set forth in SEQ ID No. 25, respectively.
   [4-14] A pharmaceutical composition containing a bispecific antibody or an antibody fragment thereof, having the first arm specifically binding to PD-1 and the second arm specifically binding to CD3 as an active ingredient, wherein
      (A) the first arm specifically binding to PD-1 has a VH comprising the amino acid sequence set forth in any one selected from SEQ ID No. 1, SEQ ID No. 2, SEQ ID No. 3, SEQ ID No. 4 and SEQ ID No. 5, and a VL comprising the amino acid sequence set forth in SEQ ID No. 25, and
      (B) the second arm specifically binding to CD3 has a VH comprising the amino acid sequence set forth in SEQ ID No. 36, and a VL comprising the amino acid sequence set forth in SEQ ID No. 25.
   [4-15] The pharmaceutical composition according to any one of the preceding items [4-1] to [4-14], wherein the bispecific antibody is an IgG antibody.
   [4-16] The pharmaceutical composition according to the preceding item [4-15], wherein the IgG antibody is an IgG₁ antibody.
   [4-17] The pharmaceutical composition according to the preceding item [4-16], wherein the binding of the IgG₁ antibody to Fc receptor is eliminated or attenuated.
   [4-18] The pharmaceutical composition according to the preceding item [4-16], wherein in two heavy chain constant regions of the IgG₁ antibody, each leucine at position 235 according to the EU numbering system is substituted with glycine, and/or each glycine at position 236 is substituted with arginine.
   [4-19] The pharmaceutical composition according to any one of the preceding items [4-16] to [4-18], wherein in a constant region of heavy chain having the VH of the first arm specifically binding to PD-1, leucine at position 351 according to the EU numbering system is substituted with lysine and threonine at position 366 is substituted with lysine, and in a constant region of heavy chain having the VH of the second arm specifically binding to CD3, leucine at position 351 is substituted with aspartic acid and leucine at position 368 is substituted with glutamic acid.
   [4-20] The pharmaceutical composition according to any one of the preceding items [4-16] to [4-18], wherein in a constant region of heavy chain having the VH of the first arm specifically binding to PD-1, leucine at position 351 according to the EU numbering system is substituted with aspartic acid and leucine at position 368 is substituted with glutamic acid, and in a constant region of heavy chain having the VH of the second arm specifically binding to CD3, leucine at position 351 is substituted with lysine and threonine at position 366 is substituted with lysine.
   [4-21] The pharmaceutical composition according to any one of the preceding items [4-16] to [4-20], wherein in two heavy chain constant regions of the IgG₁ antibody, each lysine at position 447 according to the EU numbering system is deleted.
   [4-22] The pharmaceutical composition according to any one of the preceding items [4-1] to [4-16], wherein the heavy chain having the VH of the first arm specifically binding to PD-1 has a heavy chain constant region comprising the amino acid sequence set forth in SEQ ID No. 23.
   [4-23] The pharmaceutical composition according to any one of the preceding items [4-1] to [4-16] and [4-22], wherein the heavy chain having the VH of the second arm specifically binding to CD3 has a heavy chain constant region comprising the amino acid sequence set forth in SEQ ID No. 24.
   [4-24] The pharmaceutical composition according to any one of the preceding items [4-1] to [4-23], wherein the light chain having the VL of the first arm specifically binding to PD-1 and/or the light chain having the VL of the second arm specifically binding to CD3 have/has a light chain constant region comprising the amino acid sequence set forth in SEQ ID No. 29.
   [4-25] A pharmaceutical composition containing a bispecific antibody or an antibody fragment thereof, having a first arm specifically binding to PD-1 and a second arm specifically binding to CD3 as an active ingredient, wherein
      (A) a heavy chain having the VH of the first arm specifically binding to PD-1 has a VH comprising the amino acid sequence set forth in any one selected from SEQ ID No. 1, SEQ ID No. 2, SEQ ID No. 3, SEQ ID No. 4 and SEQ ID No. 5 and a heavy chain constant region comprising the amino acid sequence set forth in SEQ ID No. 23,
      (B) a light chain having the VL of the first arm specifically binding to PD-1 has a VL comprising the amino acid sequence set forth in SEQ ID No. 25 and a light chain constant region comprising the amino acid sequence set forth in SEQ ID No. 29,
      (C) a heavy chain having the VH of the second arm specifically binding to CD3 has a VH comprising the amino acid sequence set forth in SEQ ID No. 36 and a heavy chain constant region comprising the amino acid sequence set forth in SEQ ID No. 24, and
      (D) a light chain having the VL of the second arm specifically binding to CD3 has a VL comprising the amino acid sequence set forth in SEQ ID No. 25 and a light chain constant region comprising the amino acid sequence set forth in SEQ ID No. 29.
   [4-26] The pharmaceutical composition according to the preceding items [4-1] to [4-25], further containing a pharmaceutically acceptable carrier.

### [Advantage Effects of Invention]

The PD-1/CD3 bispecific protein of the present invention (preferably, the PD-1/CD3 bispecific antibody or antibody fragment thereof) is expected to have effects on prevention, suppression of the progression of symptoms of or the recurrence of and/or treatment of hematological cancer.

### [Brief Description of the Drawings]

[Figure 1] It shows the respective amino acid sequences of VL and constant region of common light chain.
[Figure 2] It shows the respective CDR amino acid sequences in the VL of common light chain.
[Figure 3] It shows the amino acid sequences encoded by germ-line V genes, IGHV7-4-1 and IGHV3-33, respectively.
[Figure 4] It shows a sequence alignment among the VHs of the respective clones of antibodies specifically binding to PD-1 (hereinafter, may be abbreviated as an "anti-PD-1 antibody") and the germ-line genes IGHV7-4-1 and JH6c. In the amino acid sequences of the respective clones, "-" represents the same amino acid as that of the corresponding germ-line gene IGHV7-4-1 or JH6c, and abbreviations of amino acids represent amino acids different from that of the germ-line gene, respectively.
[Figure 5] It shows the VH amino acid sequences of the respective anti-PD-1 antibody clones.
[Figure 6] It shows the amino acid sequences of the respective CDRs in the VH of the respective anti-PD-1 antibody clones.
[Figure 7] It shows the VH amino acid sequence of the CD3-2 clone as an antibody specifically binding to CD3 (hereinafter, may be abbreviated as an "anti-CD3 antibody").
[Figure 8] It shows the respective amino acid sequences of the CDRs in the VH of the CD3-2 clone as an anti-CD3 antibody.
[Figure 9] It shows the amino acid sequences of the respective heavy chain constant regions of the PD-1/CD3 bispecific monoclonal antibody.
[Figure 10] It shows the VH amino acid sequence of the anti-CD3 antibody clone, 15C3 described in WO2005/118635. Note here that the underlined amino acid represents the 55th glycine which is converted into alanine to produce the CD3-1 clone.
[Figure 11] It shows the results of Biacore measurement verifying the binding activities to PD-1 and CD3 of the respective PD-1/CD3 bispecific monoclonal antibody clones, respectively.
[Figure 12] it shows flow cytometry verifying the simultaneous binding properties to PD-1 and CD3 of the respective PD-1/CD3 bispecific monoclonal antibody clones.
[Figure 13] It shows flow cytometry verifying an influence on the PD-1/PD-L1 interaction of the respective PD-1/CD3 bispecific monoclonal antibody clones.
[Figure 14] It shows the cross-competitive activity of PD1-5(Bi) against the bindings to PD-1 of the respective PD-1/CD3 bispecific monoclonal antibody clones.
[Figure 15] It shows the cell growth inhibitory effects on B lymphoma cell line EB-1 (A) and human PD-1-forced expressing SU-DHL-4 (B) of the PD-1/CD3 bispecific monoclonal antibody PD1-5(Bi) clone (0.01 to 1000 ng/mL).
[Figure 16] It shows the cell growth inhibitory effect on multiple myeloma cell line RPMI8226 of the PD-1/CD3 bispecific monoclonal antibody PD1-5(Bi) clone (0.01 to 1000 ng/mL).
[Figure 17] It shows the cell growth inhibitory effect on adult T-cell lymphoma cell line ILT-Mat of the PD-1/CD3 bispecific monoclonal antibody PD1-5(Bi) clone (0.1 to 1000 ng/mL).
[Figure 18] It shows the cell growth inhibitory effect on human PD-1-forced expressing Jurkat (acute T-cell leukemia cell line) of PD-1/CD3 bispecific monoclonal antibody PD1-5(Bi) clone (0.1 to 1000 ng/mL).
[Figure 19] It shows the respective results of the human FcRn binding properties of the PD-1/CD3 bispecific monoclonal antibodies, PD1-5 (Bi) and Mut1 to Mut3 clones, measured by Biacore (registered trademark).
[Figure 20] It shows the respective results of the human FcRn binding properties of the PD-1/CD3 bispecific monoclonal antibodies, Mut4 to Mut6 clones, measured by Biacore (registered trademark).
[Figure 21] It shows the respective results of mouse FcRn binding properties of the PD-1/CD3 bispecific monoclonal antibodies, PD1-5(Bi), Mut1 and Mut4 clones, measured by Biacore (registered trademark).
[Figure 22] It shows the cell growth inhibitory effect on T cell acute lymphocytic leukemia cell line HPB-ALL of the PD-1/CD3 bispecific monoclonal antibody PD1-5(Bi) clone (0.1 to 1000 ng/mL).
[Figure 23] It shows the relationship between the number of PD-1 molecules on a target cell and the cytotoxic activity (EC50 (ng/mL)) of human peripheral blood T cells to the target cell (human PD-1-expressing SU-DHL-4, EB-1 and RPMI8226) under the presence of the PD-1/CD3 bispecific monoclonal antibody PD1-5(Bi) clone.

### [Description of Embodiments]

PD-1 (Programmed Cell Death-1) in human is a membrane-type protein composed of the amino acid sequence represented by GenBank accession number NP_005009. In the present specification, the term "PD-1", unless specifically defined otherwise, may be used as a meaning including all of isoforms thereof and further variants thereof in which an epitope of the "first arm specifically binding to PD-1" of the present invention has been conserved. In the present invention, PD-1 is preferably human PD-1.

CD3 is a membrane-type protein forming T-cell receptor complex by association with T-cell receptor. In the present specification, the term "CD3", unless specifically defined otherwise, may be used as a meaning including subtypes thereof (ε, δ, γ and ζ subtype) and further variants thereof in which an epitope of the "second arm specifically binding to CD3" of the present invention has been conserved. In the present invention, CD3 is preferably CD3ε or human CD3, and more preferably human CD3ε.

In the present specification, the term "isolate" means becoming a single substantially pure component by being identified, separated and/or purified from impurities containing a plurality of or myriad number of components extracted from host cells.

In the present specification, the term "bispecific protein" means a protein having the binding specificity to two different antigen molecules or epitopes in one molecule, and as long as it has the binding specificity, it may be in any form, including, for example, a bispecific antibody or an antibody fragment thereof, Scaffold molecule (see Current Opinion in Biotechnology (2006), Vol. 17, No. 6, p.653-658, Current Opinion in Biotechnology (2007), Vol. 18, p.1-10, Current Opinion in Structural Biology (1997), Vol. 7, p. 463-469 or Protein Science (2006), Vol. 15, p. 14-27) and modified peptides (e.g.,, special cyclic peptides (J. Synth. Org. Chem., Jpn. (2017), Vol.75, No.11, p.1171-1178), Addbody (registered trademark), Mirabody (registered trademark), etc.) and the like, but it is not limited to those. Further, examples of such Scaffold molecules include Adnectin (see WO2001/64942), Affibody (registered trademark) (see WO95/19374 and WO2000/63243), Anticalin (registered trademark) (see WO99/16873), Avimer (see Nature Biotechnology (2005), Vol. 23, p. 1556-1561), DARPin (see Nature Biotechnology (2004), Vol. 22, p.575-582), LRRP (see Nature (2004), Vol. 430, No. 6996, p.174-180), Affilin (Registered trademark) (see WO2001/04144 and WO2004/106368), Affitin (see Journal of molecular biology (2008), Vol. 383, No. 5, p.1058-1068), Fynomer (WO2011/023685) and the like, but it is not limited to those.

In the present specification, the term "monoclonal antibody" means an antibody obtained from a substantially homogeneous antibody group binding to the same specific antigen.

In this specification, the term "bispecific antibody" means an antibody having the binding specificity to two different antigen molecules or epitopes in one molecule. Further, the term "bispecific monoclonal antibody" means a bispecific antibody obtained from a substantially homogeneous antibody group.

The present invention relates to a bispecific protein capable of specifically binding to PD-1 and CD3, respectively (in the present specification, may be abbreviated as a "PD-1/CD3 bispecific protein"). In the present invention, the PD-1/CD3 bispecific protein is preferably a PD-1/CD3 bispecific antibody or an antibody fragment thereof, and further the PD-1/CD3 bispecific antibody is preferably a PD-1/CD3 bispecific monoclonal antibody, and more preferably an isolated PD-1/CD3 bispecific monoclonal antibody, and furthermore preferably an isolated human PD-1/human CD3 bispecific monoclonal antibody. Herein, the "isolated human PD-1/human CD3 bispecific monoclonal antibody" means an isolated bispecific monoclonal antibody for human PD-1 and human CD3.

Herein, examples of forms of the bispecific antibodies include a diabody, bispecific sc(Fv)₂, bispecific minibody, bispecific F(ab')₂, bispecific hybrid antibody, covalent diabody (bispecific DART), bispecific (FvCys)₂, bispecific F(ab'-zipper)₂, bispecific (Fv-zipper)₂, bispecific three-chain antibody and bispecific mAb² and the like.

The diabody is a dimer of single-chain peptides in which a VH and VL recognizing different antigens were linked to each other with a peptide linker (see Proc. Natl. Acad. Sci. USA, 1993, Vol. 90, No. 14, pp. 6444-6448).

The bispecific sc(Fv)₂ is a low-molecular antibody modified such that two pairs of VH/VL of two antibodies recognizing different antigens are linked with a peptide linker to form a continuous single chain (see J. Biological Chemistry, 1994, 269: pp. 199-206).

The bispecific F(ab')₂ is a low-molecular antibody in which Fab' fragments of antibodies recognizing two different antigens were covalently bound through a disulfide bond or the like.

The bispecific minibody is a low-molecular antibody in which the low-molecular antibody fragments modified in such a manner that the constant region CH3 domains of the antibodies are linked to scFv recognizing different antigens, respectively, was covalently bonded by disulfide bonds or the like on the CH3 domains (see Biochemistry, 1992, Vo. 31, No .6, pp. 1579-1584).

The bispecific hybrid antibody is an intact antibody in which the heavy chain/light chain complexes recognizing two different antigens were covalently bound each other by disulfide bonds or the like.

In the present invention, the form of the bispecific antibody is preferably a bispecific hybrid antibody.

The bispecific hybrid antibody can be produced from a hybridoma produced by, for example, hybrid hybridoma method (see US4474893). Alternatively, the bispecific hybrid antibody can be produced by having mammal animal cells co-express four kinds of cDNAs encoding a heavy chain and light chain of antibody recognizing different antigens, respectively, and secrete it.

The monoclonal antibodies used in the present invention can be produced by hybridoma method (see, e.g., Kohler and Milstein et al., Nature (1975), Vol. 256, p.495-97, Hongo et al., Hybridoma (1995), Vol. 14, No. 3, pp.253-260, Harlow et al., Antibodies: A Laboratory Manual, (Cold Spring Harbor Laboratory Press (1988), Vol. 2) and Hammerling et al., Monoclonal Antibodies and T-Cell Hybridomas, pp.563-681 (Elsevier, N.Y, 1981)), recombinant DNA method (see, e.g., US4816567), phage display method (see, e.g., Ladner et al., US5223409, US5403484 and US5571698, Dower et al., US5427908 and US5580717, McCafferty et al., US5969108 and US6172197, and Griffiths et al., US5885793, US6521404, US6544731, US6555313, US6582915 and US6593081).

An antibody or monoclonal antibody, when being administered to human, can be produced in a form of a chimeric antibody, humanized antibody or complete human antibody in order to reduce or eliminate its antigenicity.

The term "chimeric antibody" means an antibody of which the variable region sequence and constant region sequence are derived from different mammalian. Examples thereof include an antibody of which the variable region sequence is derived from a mouse antibody and the constant region sequence is derived from a human antibody. The chimeric antibody can be produced by linking a gene encoding an antibody variable region isolated from antibody-producing hybridomas isolated by the above-mentioned hybridoma method, recombinant DNA method or phage display method, by well-known techniques, to a gene encoding the constant region of human-derived antibody using well-known methods (see, e.g., Cabilly et al., US4816567).

The term "humanized antibody" means an antibody of which complementarity determining region (CDR) sequences derived from a germ line of other mammals such as mice were grafted into human framework sequences. The humanized antibody can also be produced by linking genes encoding the CDRs of antibody isolated from antibody-producing hybridomas isolated by the above-mentioned method, by well-known techniques, to a gene encoding a framework region of the human-derived antibody using well-known methods (see, e.g., Winter, US5225539 and US5530101; Queen et al., US5585089 and US6180370).

The term "human antibody" or "complete human antibody" means an antibody in which both of variable regions composed of framework regions and CDRs and constant regions are derived from human germline immunoglobulin sequences. The human antibody to be used in the present invention can be produced by a method using mice transformed to produce a human antibody, for example, Humab mice (see, e.g., Lonberg and Kay et al., US5545806, US5569825, US5625126, US5633425, US5789650, US5877397, US5661016, US5814318, US5874299 and US5770429), KM mice (see, e.g., Ishida et al., WO2002/43478), Xeno mice (see, e.g., US5939598, US6075181, US6114598, US6150584 and US6162963), or Tc mice (see, e.g., Tomizuka et al., Proc. Natl. Acad. Sci. USA (2000), pp.722-727). Alternatively, the human antibody can also be prepared using SCID mice in which human immune cells have been reconstructed such that the human antibody response is made upon immunization (see, e.g., Wilson et al., US5476996 and US5698767). Furthermore, the human antibody to be used in the present invention can also be produced according to the above-mentioned phage display method.

In the present specification, the term "antibody fragment" of the PD-1/CD3 bispecific antibody is a part of the full-length antibody, and is an antibody having an antigen binding part to PD-1 and an antigen binding part to CD3. Examples thereof include F(ab')₂, and the like. Herein, the antigen binding part means a minimum unit of an antibody which can bind to an antigen thereof, for example, it is composed of three CDRs in the respective VH and VL and framework regions to arrange CDRs such that the target antigen can be recognized by combination of those CDRs.

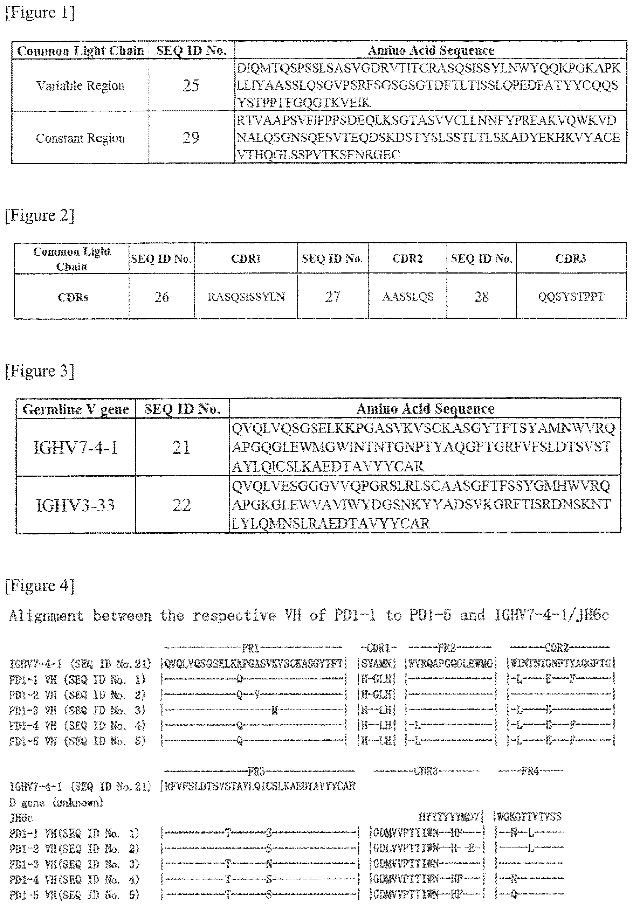

In the present specification, the term "common light chain" means a light chain which can associate with two or more different heavy chains and can exhibit the binding ability to each antigen (De Wildt RM et al., J. Mol. Biol. (1999), Vol. 285, pp.895-901, De Kruif et al., J. Mol. Biol. (2009), Vol. 387, pp.548-58, WO2004/009618, WO2009/157771 and WO2014/051433). Preferable examples of such common light chains include a light chain encoded by human κ light chain IgVκ1-39^{∗}01/IGJκ1^{∗}01 (nomenclatures of IMGT database) germ-line gene (hereinafter, may be abbreviated as "IGVK1-39/JK1 common light chain"). More preferable examples thereof include a light chain having a VL having the CDR1 comprising the amino acid sequence set forth in SEQ ID No. 26, the CDR2 comprising the amino acid sequence set forth in SEQ ID No. 27, and the CDR3 comprising the amino acid sequence set forth in SEQ ID No. 28, and further preferable examples thereof include a light chain having the VL comprising the amino acid sequence set forth in SEQ ID No. 25. Furthermore, preferable examples of the constant regions of common light chain include the light chain constant region comprising the amino acid sequence set forth in SEQ ID No. 29. The respective amino acid sequences of the VL and the constant region of common light chain used in the present invention are shown in Figure 1, and the respective amino acid sequences of CDRs of the variable region are shown in Figure 2.

In the present specification, the term "isotype" means the antibody class (e.g., IgM or IgG) which is encoded by heavy chain constant region genes. The isotype for the PD-1/CD3 bispecific antibody of the present invention is preferably IgG, and more preferably, IgG₁ or IgG₄. Herein, the IgG₁ is preferably of which the binding to Fc receptor (e.g., Fc-gamma receptor) was eliminated or decreased. Specifically, the IgG₁ antibody of which the binding to Fc receptor was eliminated or decreased can be obtained by substituting, deleting or inserting arbitrary amino acids of the heavy chain constant region thereof. Examples thereof include an antibody in which leucine at position 235 according to the EU numbering system was substituted with glycine and/or glycine at position 236 was substituted with arginine on two heavy chain constant regions or hinge regions thereof, respectively. Further, in order to reduce the heterogeneity of antibody, an antibody in which an amino acid at the C-terminus, for example, lysine at position 447 according to the EU numbering system has been deleted is preferable. Furthermore, in order to shorten the half-life in blood, in particular, those of which the binding to FcRn receptor was eliminated or attenuated are also preferable. Specifically, it can be abolished or attenuated by substitution or deletion of amino acids at the binding site to FcRn receptor of the heavy chain constant region, but in the case of IgG₁ antibody, examples of such antibodies include those of which (1) each methionine at position 252 according to the EU numbering system was substituted with glutamic acid, proline, arginine or aspartic acid, (2) each asparagine at position 434 according to the EU numbering system was substituted with leucine, and/or (3) each glutamine at position 438 according to the EU numbering system was substituted with glutamic acid. On the other hand, when the bispecific antibody is IgG₄, in order to suppress the swapping in an antibody molecule, a variant in which an arbitrary amino acid in a heavy chain constant region thereof was substituted, deleted or inserted is more preferable. For example, the antibody of which serine at position 228 according to the EU numbering system, located in the hinge region, was substituted with proline is preferable. Note herein that in the present specification, amino acid positions assigned to CDRs and framework in variable regions of antibody may be specified according to Kabat (see Sequences of Proteins of Immunological Interest (National Institute of Health, Bethesda, Md., 1987 and 1991)). Further, amino acids in the constant region are indicated according to the EU numbering system according to Kabat's amino acid positions (see Sequences of proteins of immunological interest, NIH Publication No. 91-3242).

In the Fc regions of the PD-1/CD3 bispecific antibody of the present invention, arbitrary amino acids therein may be substituted such that two different heavy chains are easily associated with each other. Examples of preferable embodiments thereof include a PD-1/CD3 bispecific antibody of which in the constant region of the heavy chain having the VH of the first arm specifically binding to PD-1, leucine at position 351 according to the EU numbering system was substituted with lysine, and threonine at position 366 was substituted with lysine, and of which in the constant region of the heavy chain having the VH of the second arm specifically binding to CD3, leucine at position 351 was substituted with aspartic acid, and leucine at position 368 was substituted with glutamic acid. Further, examples thereof also include a PD-1/CD3 bispecific antibody of which in the constant region in the heavy chain having the VH of the first arm specifically binding to PD-1, leucine at position 351 according to the EU numbering system was substituted with aspartic acid, and leucine at position 368 was substituted with glutamic acid, and of which in the constant region in the heavy chain having the VH of the second arm specifically binding to CD3, leucine at position 351 was substituted with lysine, and threonine at position 366 was substituted with lysine.

### The first arm specifically binding to PD-1

In the present specification, the "first arm specifically binding to PD-1" (hereinafter, may be abbreviated as the "first arm") means a protein specifically binding to PD-1, regardless of whether it is contained in a part of the bispecific protein of the present invention, or exists as a simple substance, and when the bispecific protein is a bispecific antibody, the first arm means a part of antibody containing at least a VH and VL of antibody specifically binding to PD-1 (hereinafter, may be abbreviated as an "anti-PD-1 antibody") and capable of specifically binding to PD-1, regardless of whether it is contained in a part of antibody or antibody fragment thereof, or exists not as a part but as a simple substance. For example, the first arm like this includes a Fv of the antibody comprising the VH and VL composing antigen binding parts of the anti-PD-1antibody, as well as a Fab' and Fab of the antibody containing the same VH and VL. Herein, the term "specifically binding to PD-1" is used as a feature of directly binding to PD-1 with higher binding affinity than at least 1 x 10⁻⁵ M, preferably 1 x 10⁻⁷ M, and more preferably 1 x 10⁻⁹ M (dissociation constant (Kd value)), and not substantially binding to any receptor members belonging to a so-called CD28 family receptor, such as at least, CD28, CTLA-4 and ICOS. Furthermore, the "antibody" in the "antibody specifically binding to PD-1" or the "anti-PD-1 antibody" means a full-length antibody, that is, a full-length antibody consisting of two heavy chains and two light chains linked with disulfide bonds, and preferably a monoclonal antibody thereof.

Herein, when the "first arm specifically binding to PD-1" consists of a part of antibody, preferable embodiments thereof include those having the VH having
(a) the VH-CDR1 comprising the amino acid sequence represented by HYJ¹LH [wherein J¹ represents G (glycine) or A (alanine), and an alphabet represented by J¹ and other alphabets represent one-letter amino acid abbreviations, respectively],
(b) the VH-CDR2 comprising the amino acid sequence represented by WJ²NTNTU²NPTX²AQGFTG [wherein J² represents L (leucine) or I (isoleucine), U² represents E (glutamic acid) or G (glycine), X² represents F (phenylalanine) or Y (tyrosine), and an alphabet represented by J², U² or X², and other alphabets represent the same as the above, respectively], and
(c) the VH-CDR3 comprising the amino acid sequence represented by GDJ³VVPTTIWNYYU³X³MZ³V [wherein J³ represents M (methionine) or L (leucine), U³ represents H (histidine) or Y (tyrosine), X³ represents F (phenylalanine) or Y (tyrosine), Z³ represents D (aspartic acid) or E (glutamic acid), and an alphabet represented by J³, U³, X³ or Z³, and other alphabets represent the same as the above, respectively]. Herein, examples of preferable embodiments of the "first arm specifically binding to PD-1" include those having
   (1a) a VH having the respective VH-CDRs in which J¹ in the HYJ¹LH sequence as VH-CDR1 represents G (glycine), in the WJ²NTNTU²NPTX²AQGFTG sequence as VH-CDR2, J² represents L (leucine), U² represents E (glutamic acid) and X² represents F (phenylalanine), respectively, in the GDJ³VVPTTIWNYYU³X³MZ³V sequence as VH-CDR3, J³ represents M (methionine), U³ represents H (histidine), X³ represents F (phenylalanine) and Z³ represents D (aspartic acid), respectively,
   (2a) a VH having the respective VH-CDRs in which J¹ in the HYJ¹LH sequence as VH-CDR1 represents G (glycine), in the WJ²NTNTU²NPTX²AQGFTG sequence as VH-CDR2, J² represents I (isoleucine), U² represents G (glycine) and X² represents Y (tyrosine), respectively, in the GDJ³VVPTTIWNYYU³X³MZ³V sequence as VH-CDR3, J³ represents L (leucine), U³ represents H (histidine), X³ represents Y (tyrosine) and Z³ represents E (glutamic acid), respectively,
   (3a) a VH having the respective VH-CDRs in which J¹ in the HYJ¹LH sequence as VH-CDR1 represents A (alanine), in the WJ²NTNTU²NPTX²AQGFTG sequence as VH-CDR2, J² represents L (leucine), U² represents E (glutamic acid) and X² represents Y (tyrosine), respectively, in the GDJ³VVPTTIWNYYU³X³MZ³V sequence as VH-CDR3, J³ represents M (methionine), U³ represents Y (tyrosine), X³ represents Y (tyrosine) and Z³ represents D (aspartic acid), respectively, or
   (4a) a VH having the respective VH-CDRs in which J¹ in the HYJ¹LH sequence as VH-CDR1 represents A (alanine), in the WJ²NTNTU²NPTX²AQGFTG sequence as VH-CDR2, J² represents L (leucine), U² represents E (glutamic acid) and X² represents F (phenylalanine), respectively, in the GDJ³VVPTTIWNYYU³X³MZ³V sequence as VH-CDR3, J³ represents M (methionine), U³ represents H (histidine), X³ represents F (phenylalanine) and Z³ represents D (aspartic acid), respectively.

Furthermore, when the "first arm specifically binding to PD-1" consists of a part of antibody, examples of other preferable embodiments thereof include those having any one of VH selected from
(1b) a VH having the VH-CDR1 comprising the amino acid sequence set forth in SEQ ID No. 6, the VH-CDR2 comprising the amino acid sequence set forth in SEQ ID No. 7, and the VH-CDR3 comprising the amino acid sequence set forth in SEQ ID No. 8,
(2b) a VH having the VH-CDR1 comprising the amino acid sequence set forth in SEQ ID No. 9, the VH-CDR2 comprising the amino acid sequence set forth in SEQ ID No. 10, and the VH-CDR3 comprising the amino acid sequence set forth in SEQ ID No. 11,
(3b) a VH having the VH-CDR1 comprising the amino acid sequence set forth in SEQ ID No. 12, the VH-CDR2 comprising the amino acid sequence set forth in SEQ ID No. 13, and the VH-CDR3 comprising the amino acid sequence set forth in SEQ ID No. 14,
(4b) a VH having the VH-CDR1 comprising the amino acid sequence set forth in SEQ ID No. 15, the VH-CDR2 comprising the amino acid sequence set forth in SEQ ID No. 16, and the VH-CDR3 comprising the amino acid sequence set forth in SEQ ID No. 17, and
(5b) a VH having the VH-CDR1 comprising the amino acid sequence set forth in SEQ ID No. 18, the VH-CDR2 comprising the amino acid sequence set forth in SEQ ID No. 19, and the VH-CDR3 comprising the amino acid sequence set forth in SEQ ID No. 20.

Furthermore, when the "first arm specifically binding to PD-1" of the present invention consists of a part of antibody, they also include those of which one to five arbitrary amino acid residues are substituted with other amino acids (preferably, conservative amino acids thereof) in the respective VH-CDRs of any one of VH selected from the above-mentioned (1a) to (4a) or (1b) to (5b), and which have substantially the same binding activity to PD-1 as that of the original first arm without any substitutions with the same amino acids. Examples thereof include those of which one amino acid residue in the VH-CDR1 is substituted with other amino acids (preferably, a conservative amino acid thereof), and one to five arbitrary amino acid residues in the VH-CDR2 or VH-CDR3 are substituted with other amino acids (preferably, conservative amino acids thereof), respectively. As shown in Figure 4, in the respective CDRs of the anti-PD-1 antibody clone corresponding to the first arm specifically binding to PD-1, respectively, amino acids different among the clones or any combination of a plurality thereof can be exchangeable among the clones. Herein, the substitution with a conservative amino acid means the exchangeability with a residue having a similar side-chain. For example, a group of amino acids having an aliphatic side-chain includes glycine, alanine, valine, leucine and isoleucine, a group of amino acids having an aliphatic hydroxyl side-chain includes serine and threonine, a group of amino acids having amide-containing side-chain includes asparagine and glutamine, a group of amino acids having an aromatic side-chain includes phenylalanine, tyrosine and tryptophane, a group of amino acids having a basic side-chain includes lysine, arginine and histidine, and a group of amino acids having a sulfur-containing side-chain includes cysteine and methionine. Examples of preferable substitutions with a conservative amino acid include that among valine, leucine and isoleucine, that between phenylalanine and tyrosine, that between lysine and arginine, that between alanine and valine, as well as that between asparagine and glutamine. Furthermore, herein, the sentence "which have substantially the same binding activity to PD-1 as that of the original first arm without any substitutions with the same amino acids" mentioned above means that the binding activity to PD-1 of the first arm substituted with the amino acids is 95% or more, preferably 98% or more and more preferably 99% or more to that of the original first arm without any substitutions with the same amino acids.

Furthermore, when the "first arm specifically binding to PD-1" of the present invention consists of a part of antibody, they include those having a VH which contains the respective VH-CDRs having the above-mentioned specific amino acid sequences therein, and of which the amino acid sequence of framework is encoded by a specific germ-line gene or a gene thereof with somatic mutation(s). For example, the VH represented by any one selected from the above-mentioned (1a) to (4a) or (1b) to (5b) can be encoded by the VDJ recombinant gene or gene thereof with somatic mutation(s) in which the germ-line V gene is IGHV7-4-1 and the germ-line J gene is JH6c. Herein, the amino acid sequence encoded by the germ-line V gene IGHV7-4-1 corresponds to that set forth in SEQ ID No. 21 (Figure 3).

When the first arm specifically binding to PD-1 of the present invention consists of a part of antibody, the framework in the VH may be encoded by the germ-line VDJ recombinant gene with somatic mutation(s). For example, since the FR1, FR2 and FR3 in the VH represented by any one selected from the above-mentioned (1a) to (4a) or (1b) to (5b) of which the germ-line V gene is IGHV7-4-1 are different from the amino acid sequence encoded by the IGHV7-4-1 gene at the positions of the amino acids shown in Figure 4, they have undergone somatic mutations at the respective same positions. For example, as to the FR1 region, in the amino acid sequence set forth in SEQ ID No. 21, lysine at position 13 may be substituted with glutamine, alanine at position 16 may be substituted with valine, or lysine at position 19 may be substituted with methionine, respectively, or which may be substituted in an arbitrary combination of a plurality thereof. As to the FR2 region, valine at position 37 in the amino acid sequence set forth in SEQ ID No. 21 may be substituted with leucine. As to the FR3 region, in the amino acid sequence set forth in SEQ ID No. 21, serine at position 77 may be substituted with threonine, or cysteine at position 84 may be substituted with serine or asparagine, respectively, or which may be substituted in an arbitrary combination of a plurality thereof. Furthermore, as to the FR4 region of the VH represented by any one selected from the above-mentioned (1a) to (4a) or (1b) to (5b), in the amino acid sequence (Trp-Gly-Lys-Gly-Thr-Thr^{∗}-Val-Thr-Val-Ser-Ser) (SEQ ID No. 41) of the FR4 region derived from the J gene JH6c, lysine (Lys) may be substituted with glutamine or asparagine and/or threonine (Thr) marked with an asterisk may be substituted with leucine. The respective FR1, FR2, FR3 and FR4 having combination of any of amino acid substitutions mentioned above have no substantial effect on the functions of the first arm specifically binding to PD-1, and can be used as framework.

Further, when the first arm specifically binding to PD-1 of the present invention consists of a part of antibody, they also include those which contains the respective CDRs having the amino acid sequence specified as the above, and of which the FR amino acid sequences in the VH are encoded by the specific germ-line genes or genes thereof with somatic mutation(s). Examples of such first arms include those having the VH comprising the amino acid sequence set forth in any one selected from SEQ ID Nos. 1 to 5.

Furthermore, examples of such "first arms specifically binding to PD-1" include also those which have a VH comprising an amino acid sequence having at least 80% identity, preferably at least 90% identity, more preferably at least 95% identity, furthermore preferably at least 98% identity, and further more preferably at least 99% identity to the amino acid sequence set forth in any one selected from SEQ ID Nos. 1 to 5, and in which the difference from the VH amino acid sequence of the original first arm has no substantial effect on the binding activity to PD-1 (hereinafter, may be abbreviated as a "homologous first arm"). Herein, the term "% identity" used in comparison of the identity of amino acid sequences is defined as the percentage of amino acid sequence identical to the reference amino acid sequence (herein, when being needed to maximize the sequence identity, the reference amino acid sequence in which the gap has been introduced) when two sequences are aligned. Further, herein, the sentence "the different from the VH amino acid sequence of the original first arm has no substantial effect on the binding activity to PD-1" means that the binding activity to PD-1 of the homologous first arm is 95% or more, preferably 98% or more, and more preferably 99% or more to that of the original first arm.

In a yet another embodiment, when the first arm specifically binding to PD-1 of the present invention consists of a part of antibody, they also include those having a variable region (herein, the variable region contains a VH and VL constituting it.) of the anti-PD-1 antibody cross-competing for (1) the binding to PD-1 with the first arm having the VH presented by any one selected from the above-mentioned (1a) to (4a) or (1b) to (5b) or the VH comprising the amino acid sequence set forth in any one selected from SEQ ID Nos. 1 to 5 and the VL of the common light chain, or (2) the binding to PD-1 with the variable region of the monoclonal antibody specifically binding to PD-1 having the same VH and VL, and further include those having the variable region of the anti-PD-1 antibody with which the binding to PD-1 is cross-competed by (3) the first arm having the VH presented by any one selected from the above-mentioned (1a) to (4a) or (1b) to (5b) or the VH comprising the amino acid sequence set forth in any one selected from SEQ ID Nos. 1 to 5 and the VL of common light chain or (4) the variable region of the monoclonal antibody specifically binding to PD-1 having the same VH and VL. Herein, the sentence "cross-competing for the binding to PD-1" means inhibiting the binding of the first arm to PD-1, regardless of the degree thereof, by binding to the epitope which is the same as or partially overlaps with that of the first arm exemplified in this specification, or that the binding to PD-1 of the antibody binding to the epitope which is the same as or partially overlaps with that of the exemplified first arm is inhibited by the exemplified first arm, regardless of the degree thereof. Whether it cross-competes or not can be evaluated by a competitive binding assay. For example, it can be determined using Biacore analysis, ELISA assay, flow cytometry, enzyme linked immunosorbent assay (ELISA), fluorescence energy transfer method (FRET) or fluorometric microvolume assay technology (FMAT (registered trademark)).

Examples of the first arm cross-competing for the binding to PD-1 by the first arm having the VH presented by the above-mentioned (5b) and the VL of common light chain include the first arm having the VH presented by any one selected from the above-mentioned (1b) to (4b) and the VL of common light chain (preferably, the VL having the VL-CDR1 comprising the amino acid sequence set forth in SEQ ID No. 26, the VL-CDR2 comprising the amino acid sequence set forth in SEQ ID No. 27 and the VL-CDR3 comprising the amino acid sequence set forth in SEQ ID No. 28), and further the first arm having the VH comprising the amino acid sequence set forth in any one selected from SEQ ID Nos. 1 to 4 and the VL of common light chain (preferably, the VL comprising the amino acid sequence set forth in SEQ ID No. 25).

Furthermore, examples of the first arm cross-competing for the binding to PD-1 with the first arm having the VH presented by any one selected from the above-mentioned (1b) to (4b) or the VH comprising the amino acid sequence set forth in any one selected from SEQ ID Nos. 1 to 4 and the VL of common light chain include the first arm having the VH presented by the above-mentioned (5b) and the VL of common light chain (preferably, the VL having the VL-CDR1 comprising the amino acid sequence set forth in SEQ ID No. 26, the VL-CDR2 comprising the amino acid sequence set forth in SEQ ID No. 27 and the VL-CDR3 comprising the amino acid sequence set forth in SEQ ID No. 28), and further the first arm having the VH comprising the amino acid sequence set forth in SEQ ID Nos. 5 and the VL of common light chain (preferably, the VL comprising the amino acid sequence set forth in SEQ ID No. 25).

Herein, when the first arm specifically binding to PD-1 of the present invention consists of a part of antibody, preferable examples thereof include the first arm having the VH represented by any one selected from the above-mentioned (1b) to (5b). Further, as mentioned above, preferable examples of the first arm also include those in which one to five arbitrary amino acid residues are substituted with other amino acids (preferably, conservative amino acids thereof) in the CDRs of the same VH and the same substitutions do not substantially affect the binding activity to PD-1. Furthermore, as mentioned above, they also include those having the VH in which the amino acid sequences of framework are encoded by the germ-line V gene IGHV7-4-1 or J gene JH6c or genes thereof with somatic mutation(s). Then, more preferable examples of the first arm include those having the VH comprising the amino acid sequence set forth in any one selected from SEQ ID Nos. 1 to 5.

Furthermore, when the first arm specifically binding to PD-1 of the present invention consists of a part of antibody, a VL thereof is preferably that of a common light chain, and such a common light chain is preferably the IGVK1-39/JK1 common light chain. A more preferable example thereof is a light chain having the VL having the CDR1 comprising the amino acid sequence set forth in SEQ ID No. 26, the CDR2 comprising the amino acid sequence set forth in SEQ ID No. 27 and the CDR3 comprising the amino acid sequence set forth in SEQ ID No. 28, and a further preferable example thereof is the light chain containing the VL comprising the amino acid sequence set forth in SEQ ID No. 25. Furthermore, preferable examples of the constant regions of common light chain include the light chain constant region comprising the amino acid sequence set forth in SEQ ID No. 29.

One of embodiments of the "first arm specifically binding to PD-1" allows the interaction between PD-1 and PD-L1, interaction between PD-1 and PD-L2 or both of interactions thereof. Herein, the sentence "allows the interaction between PD-1 and PD-L1, interaction between PD-1 and PD-L2, or both of interactions thereof' means that even when there is the PD-1/CD3 bispecific antibody of the present invention at 20-fold excess over the concentration of the soluble form of PD-L1 or PD-L2, the interaction between PD-L1 and PD-1, interaction between PD-L2 and PD-1, or both of interactions thereof are maintained 50% or more, preferably 70% or more, and more preferably 80% or more, compared with those when there is no PD-1/CD3 bispecific antibody of the present invention. Furthermore, the definition of "allowing the interaction between PD-1 and PD-L1, interaction between PD-1 and PD-L2, or both of interactions thereof' may have the same meaning as that of "which does not substantially inhibit the interaction between PD-1 and PD-L1, interaction between PD-1 and PD-L2 or both of interactions thereof."

As another embodiment, the "first arm specifically binding to PD-1" of the present invention may inhibit the interaction between PD-1 and PD-L1, interaction between PD-1 and PD-L2, or both of interactions thereof.

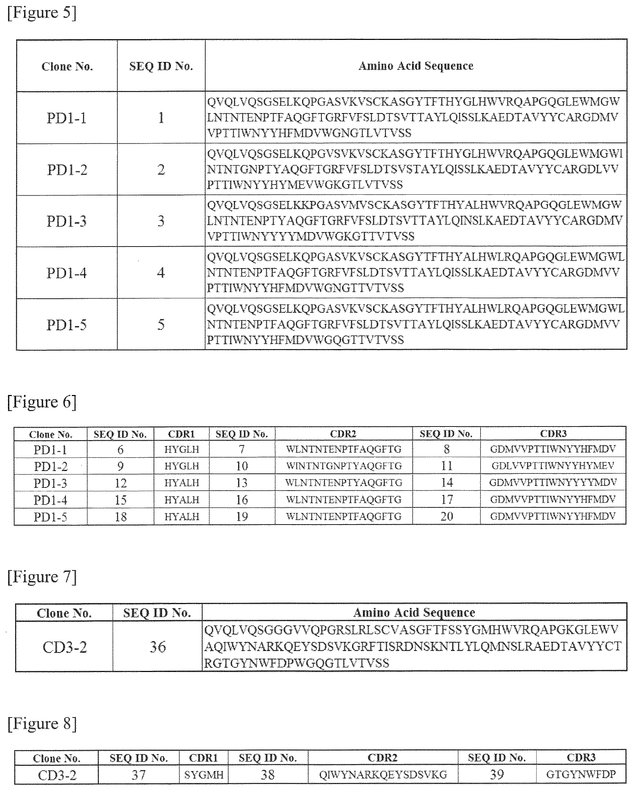

The correspondence relations between the respective anti-PD-1 monoclonal antibody clones obtained to construct the PD-1/CD3 bispecific antibody of the present invention and VH amino acid sequence thereof and SEQ ID numbers thereof are shown in Figure 5. The correspondence relations between the CDR amino acid sequences in the VH of the respective anti-PD-1 monoclonal antibody clones and SEQ ID number thereof are shown in Figure 6.

Further, examples of "the first arm specifically binding to PD-1 also include the combination of the VH and VL of already-known anti-PD-1 antibody, and examples of anti-PD-1 antibodies like those include Nivolumab, Cemiplimab, Pembrolizumab, Spartalizumab, Tislelizumab, Dostarlimab, Toripalimab, Camrelizumab, Genolimzumab, Sintilimab, Lodapolimab, Retifanlimab, Balstilimab, Serplulimab, Budigalimab, Prolgolimab, Sasanlimab, Cetrelimab, Zimberelimab, AMP-514, STI-A1110, ENUM 388D4, ENUM 244C8, GLS010, CS1003, BAT-1306, AK105, AK103, BI 754091, LZM009, CMAB819, Sym021, GB226, SSI-361, JY034, HX008, ISU106, CX-188 and the like. Examples of the first arm specifically binding to PD-1 also include the combination of the VH and VL of the anti-PD-1 antibody disclosed in the patent publication identified by US20180312564, WO2004056875, WO2006121168, WO2008156712, WO2010029434, WO2010029435, WO2010036959, WO2011110604, WO2011110621, WO2014179664, WO2014194302, WO2014206107, WO2015035606, WO2015036394, WO2015085847, WO2015112800, WO2016014688, WO2016015685, WO2016068801, WO2016077397, WO2016092419, WO2016106159, WO2016127179, WO2016197497, WO2016210129, WO2017011580, WO2017016497, WO2017019846, WO2017024465, WO2017024515, WO2017025016, WO2017025051, WO2017040790, WO2017055443, WO2017055547, WO2017058115, WO2017058859, WO2017079112, WO2017079116, WO2017087599, WO2017096026, WO2017106656, WO2017107885, WO2017124050, WO2017125815, WO2017132827, WO2017133540, WO2017166804, WO2017198741, WO2017201766, WO2017214182, WO2018020476, WO2018026248, WO2018034226, WO2018036472, WO2018052818, WO2018053106, WO2018068336, WO2018085358, WO2018085468, WO2018087143, WO2018091661, WO2018113258, WO2018119474, WO2018162944, WO2018192089, WO2018210230, WO2018217227, WO2018226580, WO2019005635 or WO2019051164, respectively. Further, examples of the first arm specifically binding to PD-1 also include the combination of the VH of the above-mentioned the anti-PD-1 antibody and the common light chain.

### The second arm specifically binding to CD3

In the present specification, the "second arm specifically binding to CD3" (hereinafter, may be abbreviated as the "second arm") means a protein specifically binding to CD3, regardless of whether it is contained in a part of the bispecific protein of the present invention, or exists as a simple substance, and when the bispecific protein is a bispecific antibody, the second arm means an antibody portion having at least a VH and VL of an antibody specifically binding to CD3 (hereinafter, may be abbreviated as an "anti-CD3 antibody") and capable of specifically binding to CD3, regardless of whether it is contained in a part of antibody or antibody fragment thereof, or exists not as a part but as a simple substance. For example, the second arm includes a Fv of the antibody comprising the VH and VL composing antigen binding parts of the anti-CD3 antibody, as well as a Fab' and Fab of the antibody containing the same VH and VL. Herein, the sentence "specifically binding to CD3" is used as a feature of directly binding to CD3 with higher binding affinity than at least 1 x 10⁻⁵ M, preferably 1 x 10⁻⁷ M, and more preferably 1 x 10⁻⁹ M (dissociation constant (Kd value)) and not substantially binding to any other proteins. Furthermore, the "antibody" in the "antibody specifically binding to CD3" or "anti-CD3 antibody" means a full-length antibody, that is, a full-length antibody consisting of two heavy chains and two light chains linked with disulfide bonds, and preferably a monoclonal antibody thereof.

Herein, when the second arm specifically binding to CD3 of the present invention consists of a part of antibody, preferable examples thereof include those containing the VH having (1c) the VH-CDR1 comprising the amino acid sequence set forth in SEQ ID No. 37, the VH-CDR2 comprising the amino acid sequence set forth in SEQ ID No. 38, and the VH-CDR3 comprising the amino acid sequence set forth in SEQ ID No. 39.

Further, when the second arm specifically binding to CD3 of the present invention consists of a part of antibody, they also include those in which one to five arbitrary amino acid residues are substituted with other amino acids (preferably, conservative amino acids thereof) in the respective CDRs of the VH of the above-mentioned (1c), and which have substantially the same binding activity to CD3 as that of the original second arm without any substitutions with the same amino acids. Examples thereof include those of which one amino acid residue in CDR1 is substituted with other amino acids (preferably, a conservative amino acid thereof), and one to five amino acid residues in CDR2 or CDR3 are substituted with other amino acids (preferably, conservative amino acids thereof), respectively. Herein, the sentence "which have substantially the same binding activity to CD3 as that of the original second arm without any substitutions with the same amino acid(s)" mentioned above means that the binding activity to CD3 of the second arm substituted with the amino acid is 95% or more, preferably 98% or more and more preferably 99% or more to that of the original second arm without any substitutions with the same amino acids. Note here that examples of the "substitution with conservative amino acids" in the respective VH-CDRs of the second arm include those of the amino acid substitutions in the first arm mentioned above.

Furthermore, when the second arm specifically binding to CD3 of the present invention consists of a part of antibody, they also include those having a VH which contains the respective CDRs comprising the above-mentioned specific amino acid sequence, and of which the FR amino acid sequences are encoded by a specific germ-line gene or gene thereof with somatic mutation(s). For example, the VH of the above-mentioned (1c) is encoded by a VDJ recombinant gene or gene thereof with somatic mutation(s) in which the germ-line V gene is IGHV3-33. Herein, the amino acid sequence encoded by the germ line V gene IGHV3-33 (SEQ ID No. 22) is shown in Figure 3. Further, examples of such second arms include those having the VH comprising the amino acid sequence set forth in SEQ ID No. 36. Furthermore, examples of such second arms also include a VH which comprises an amino acid sequence having at least 80%, preferably at least 90%, more preferably at least 95%, further more preferably at least 98%, and still further preferably at least 99% identity to the amino acid sequence set forth in SEQ ID No. 36, and in which the difference from the VH amino acid sequence of the original second arm has no substantial effect on the binding activity to CD3 (hereinafter, may be abbreviated as a "homologous second arm"). Herein, the sentence "the difference from the VH amino acid sequence of the original second arm has no substantial effect on the binding activity to CD3" means that the binding activity of the homologous second arm to CD3 is 95% or more, preferably 98% or more and more preferably 99% or more to that of the original second arm.

In a yet another embodiment, when the second arm specifically binding to CD3 of the present invention consists of a part of antibody, they also include those having a variable region of the anti-CD3 antibody (herein, the variable region includes the VH and VL constituting it) cross-competing for (1) the binding to CD3 with a second arm having the VH presented by the above-mentioned (1c) or the VH comprising the amino acid sequence set forth in SEQ ID No. 36 and the VL of common light chain, or (2) the binding to CD3 with the variable region of the monoclonal antibody specifically binding to CD3 having the same VH and VL. Herein, the "cross-competing for the binding to CD3" means inhibiting the binding of the second arm to CD3, regardless of the degree thereof, by binding to an epitope which is the same as or partially overlaps with the second arm exemplified in the present specification. Herein, whether it cross-competes or not can be similarly evaluated according to the same method as described in descriptions regarding the "first arm specifically binding to PD-1".

Herein, when the second arm specifically binding to CD3 of the present invention consists of a part of antibody, preferable examples thereof include the second arm having the VH presented by the above-mentioned (1c). Further, as mentioned above, preferable examples of the second arm also include those in which one to five arbitrary amino acid residues are substituted with other amino acids (preferably, conservative amino acids thereof) in the respective CDRs of the VH and the same substitutions with the amino acid do not substantially affect the binding activity to CD3. Furthermore, as mentioned above, they also include those having the VH in which the amino acid sequences of framework are encoded by the germ-line gene IGHV3-33 or gene thereof with somatic mutation(s). Then, more preferable examples of the second arm include those having the VH comprising the amino acid sequence set forth in SEQ ID No. 36.

The correspondence relations between the respective anti-CD3 antibody clones to construct the PD-1/CD3 bispecific antibody of the present invention, VH amino acid sequences thereof and SEQ ID numbers thereof are shown in Figure 7. The correspondence relations between the respective CDR amino acid sequences in the VH of the respective anti-CD3 antibody clones and SEQ ID numbers thereof are shown in Figure 8.

When the second arm specifically binding to CD3 of the present invention consists of a part of antibody, a VL thereof is preferably that of the common light chain, and such a common light chain is preferably the IGVK1-39/JK1 common light chain. A more preferable example thereof is the light chain having the VL having the CDR1 comprising the amino acid sequence set forth in SEQ ID No. 26, the CDR2 comprising the amino acid sequence set forth in SEQ ID No. 27 and the CDR3 comprising the amino acid sequence set forth in SEQ ID No. 28, and a further preferable example thereof is the light chain having the VL comprising the amino acid sequence set forth in SEQ ID No. 25. Furthermore, preferable examples of the constant region of the common light chain include the light chain constant region comprising the amino acid sequence set forth in SEQ ID No. 29.

Preferable examples of the "second arm specifically binding to CD3" of the present invention include those specifically binding to CD3ε.

Further, examples of other "second arms specifically binding to CD3" include a combination of the VH and VL of already-known anti-CD3 antibody, and examples of such anti-CD3 antibodies include Foralumab, Muromonab-CD3, Otelixizumab, Teplizumab, Visilizumab, and the like, and further include a combination of the VH and VL composing the CD3 binding site of any of bispecific antibody selected from Blinatumomab, Catumaxomab, Cibisatamab, Duvortuxizumab, Ertumaxomab, Flotetuzumab, Pasotuxizumab, Solitomab, Tebentafusp, Tepoditamab, AMG-596, MOR209, REGN1979, XmAb13676 and XmAb14045. Furthermore, examples of the second arms specifically binding to CD3 also include a combination of the VH and VL composing the CD3 binding site of the anti-CD3 antibody or bispecific antibody thereof disclosed in the patent publications identified by US20190016823, WO2018223004, WO2018223002, US20180291114, WO2018114754, CN107501412, CN106810611, US20170218079, US20170174779, CN106349391, WO2017070943, WO2017053856, WO2017021349, CN106084049, WO2017021354, WO2016179003, US20160176980, WO2016014974, WO2016016859, US20150266966, CN104098698, WO2014151438, WO2013186613, CN102219856, WO2010052013, WO2010037836, WO2007042261, WO2007033230, WO2003026692, WO2000005268, CN106084046, CN106146661 or WO2010037835. Further, examples of the second arms specifically binding to CD3 also include a combination of the VH of the above-mentioned anti-CD3 antibody and the common light chain.

An isotype of the PD-1/CD3 bispecific antibody of the present invention is preferably an IgG antibody, further preferably an IgG₁ antibody or IgG₄ antibody, and more preferably an IgG₁ antibody.

Examples of preferable embodiments of the PD-1/CD3 bispecific antibodies of the present invention include those of which the first arm specifically binding to PD-1 has
(A) the VH in which one to five arbitrary amino acid residues may be substituted with other amino acids (preferably, conservative amino acids thereof) in any one or more of the CDRs selected from the VH-CDR1, VH-CDR2 and VH-CDR3 in the VH presented by any selected from the above-mentioned (1a) to (4a) or (1b) to (5b), and
(B) the VL of common light chain having the CDR1 comprising the amino acid sequence set forth in SEQ ID No. 26, the CDR2 comprising the amino acid sequence set forth in SEQ ID No. 27 and the CDR3 comprising the amino acid sequence set forth in SEQ ID No. 28, and
   the second arm specifically binding to CD3 has
(C) the VH in which one to five arbitrary amino acid residues may be substituted with other amino acids (preferably, conservative amino acids thereof) in any one or more of the CDRs selected from the VH-CDR1, VH-CDR2 and VH-CDR3 in the VH presented by the above mentioned (1c), and
(D) the VL of common light chain having the CDR1 comprising the amino acid sequence set forth in SEQ ID No. 26, the CDR2 comprising the amino acid sequence set forth in SEQ ID No. 27 and the CDR3 comprising the amino acid sequence set forth in SEQ ID No. 28.

More preferable Examples thereof include the PD-1/CD3 bispecific antibody of which the first arm specifically binding to PD-1 has
(A) the VH presented by any one selected from the above-mentioned (1a) to (4a) or (1b) to (5b), and
(B) the VL of common light chain having the CDR1 comprising the amino acid sequence set forth in SEQ ID No. 26, the CDR2 comprising the amino acid sequence set forth in SEQ ID No. 27, and the CDR3 comprising the amino acid sequence set forth in SEQ ID No. 28, and
   the second arm specifically binding to CD3 has
(C) the VH presented by the above-mentioned (1c), and
(D) the VL of common light chain having the CDR1 comprising the amino acid sequence set forth in SEQ ID No. 26, the CDR2 comprising the amino acid sequence set forth in SEQ ID No. 27, and the CDR3 comprising the amino acid sequence set forth in SEQ ID No. 28.

Furthermore, examples of other preferable embodiments of the PD-1/CD3 bispecific antibodies of the present invention include those of which the first arm specifically binding to PD-1 has
(A) the VH comprising the amino acid sequence set forth in any one selected from SEQ ID Nos. 1 to 5, or a VH comprising an amino acid sequence having at least 80% identity to VH amino acid sequence thereof, and
(B) the VL of common light chain comprising the amino acid sequence set forth in SEQ ID No. 25, and
   the second arm specifically binding to CD3 has
(C) the VH comprising the amino acid sequence set forth in SEQ ID No. 36, or a VH comprising an amino acid sequence having an identity of at least 80% to VH amino acid sequence thereof, and
(D) the VL of common light chain comprising the amino acid sequence set forth in SEQ ID No. 25.

Examples of other more preferable embodiments thereof include the PD-1/CD3 bispecific antibody of which the first arm specifically binding to PD-1 has
(A) the VH comprising the amino acid sequence set forth in any one selected from SEQ ID Nos. 1 to 5, and
(B) the VL of common light chain comprising the amino acid sequence set forth in SEQ ID No. 25, and
   the second arm specifically binding to CD3 has
(C) the VH comprising the amino acid sequence set forth in SEQ ID No. 36, and
(D) the VL of common light chain comprising the amino acid sequence set forth in SEQ ID No. 25.

In the PD-1/CD3 bispecific antibody of the present invention, when it is an IgG₁ antibody, the IgG₁ antibody in which leucine at position 235 according to the EU numbering system was substituted with glycine, and/or glycine at position 236 was substituted with arginine on two heavy chain constant regions or hinge regions thereof, respectively, is preferable. Further, a bispecific antibody in which the C-terminal amino acids of heavy chains, for example, lysine at position 447 according to the EU numbering system have been deleted is preferable. Furthermore, when the PD-1/CD3 bispecific antibody is an IgG₄ antibody, an antibody in which serine at position 228 according to the EU numbering system, located in the hinge region, was substituted with proline is preferable.

Furthermore, when the PD-1/CD3 bispecific antibody is the IgG₁ antibody, preferable embodiments thereof include those in which in the constant region of the heavy chain having the VH of the first arm specifically binding to PD-1, leucine at position 351 according to the EU numbering system was substituted with lysine and threonine at position 366 was substituted with lysine, and in the constant region of the heavy chain having the VH of the second arm specifically binding to CD3, leucine at position 351 was substituted with aspartic acid and leucine at position 368 was substituted with glutamic acid. Furthermore, the IgG₁ antibody in which in the constant region of the heavy chain having the VH of the first arm specifically binding to PD-1, leucine at position 351 according to the EU numbering system was substituted with aspartic acid and leucine at position 368 was substituted with glutamic acid, and in the constant region of the heavy chain having the VH of the second arm specifically binding to CD3, leucine at position 351 was substituted with lysine and threonine at position 366 was substituted with lysine is also preferable, as well.

Furthermore, preferable embodiments, when the PD-1/CD3 bispecific antibody is an IgG₁ antibody, include the IgG1 antibody of which in each of two heavy chain constant regions, (1) each methionine at position 252 according to the EU numbering system was substituted with glutamic acid, proline, arginine or aspartic acid, (2) each asparagine at position 434 according to the EU numbering system was substituted with leucine, and/or (3) each glutamine at position 438 according to the EU numbering system was substituted with glutamic acid. It is expected that by these amino acid substitutions, half-life in blood of the PD-1/CD3 bispecific antibody can be shorted at least 50%, preferably at least 60%, more preferably at least 70%, furthermore preferably at least 80%, and most preferably at least 90%, compared to those without any of the same amino acid substitutions.

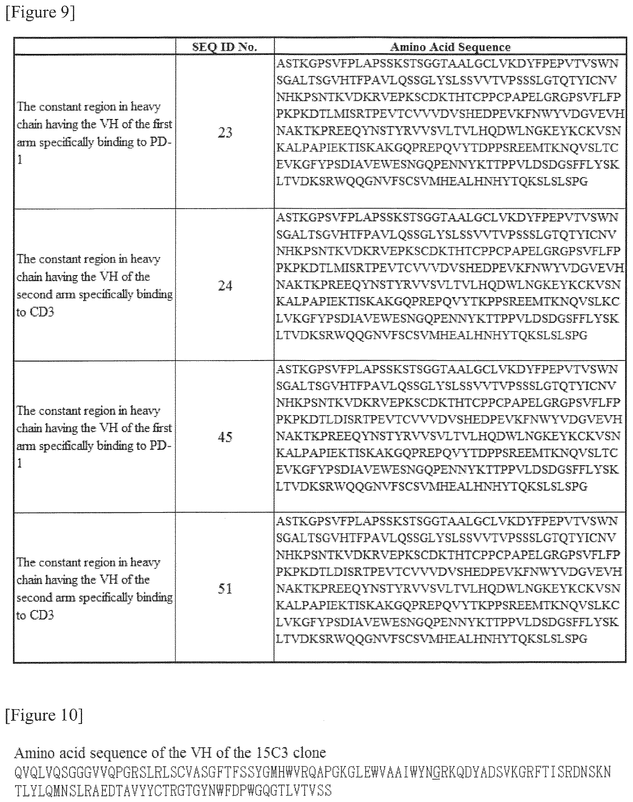

Examples of preferable embodiments of the PD-1/CD3 bispecific IgG₁ antibody in which all of the above-mentioned amino acid substitutions in the heavy chain constant region were taken include those in which the heavy chain having the VH of the first arm specifically binding to PD-1 has the heavy chain constant region comprising the amino acid sequence set forth in any one selected from SEQ ID No. 23, SEQ ID No. 42, SEQ ID No. 43, SEQ ID No. 44, SEQ ID No. 45, SEQ ID No. 46 and SEQ ID No. 47, and the heavy chain having the VH of the second arm specifically binding to CD3 has the heavy chain constant region comprising the amino acid sequence set forth in SEQ ID No. 24, SEQ ID No. 48, SEQ ID No. 49, SEQ ID No. 50, SEQ ID No. 51, SEQ ID No. 52 and SEQ ID No. 53. Some of those amino acid sequences are shown in Figure 9.

The most embodiments of the PD-1/CD3 bispecific antibody of the present invention are preferably the clones PD1-1(Bi), PD1-2(Bi), PD1-3(Bi), PD1-4(Bi) and PD1-5(Bi) generated in Example 8 of the present specification and the clones Mut1, Mut2, Mut3, Mut4, Mut5 and Mut6 generated in Example 17.

Examples of preferable features of the PD-1/CD3 bispecific antibody of the present invention include (1) allowing the interaction between PD-1 and PD-L1, interaction between PD-1 and PD-L2 or both of interactions thereof, (2) sufficiently reducing cytokine production and/or (3) specifically binding to PD-1 expressed on hematological cancer cell as a target cell and CD3 expressed on lymphocyte (e.g., cytotoxic lymphocyte) as an effector cell, respectively. Herein, the sentence "to allow the interaction between PD-1 and PD-L1, interaction between PD-1 and PD-L2 or both of interactions thereof' means the same as described in descriptions regarding the "first arm specifically binding to PD-1." On the other hand, the sentence "sufficiently reducing cytokine production" means that, for example, during intravenous administration or by 24 hours after that administration, by drip infusion of the PD-1/CD3 bispecific antibody of the present invention, for example, concentration in blood or tissue of cytokine including IL-2, IFN-γ and/or TNF-α does not increase or even if it increases, it is such a degree that it can be suppressed by steroid administration. And, the term "target cell" means cells subject to cytotoxic effects of lymphocytes as effector cells, and the term "effector cell" means cells giving its cytotoxic effects to target cells.

### Method for manufacturing and purifying the PD-1/CD3 bispecific antibody

The PD-1/CD3 bispecific protein of the present invention, specifically, a bispecific antibody and an antibody fragment thereof can also be manufactured by the method disclosed in WO2014/051433, WO2013/157953 or WO2013/157954.

Specifically, it can be manufactured by gene-transferring an expression vector in which (1) a polynucleotide encoding the heavy chain having the VH of the first arm specifically binding to PD-1, (2) a polynucleotide encoding the heavy chain having the VH of the second arm specifically binding to CD3, and (3) a polynucleotide encoding the common light chain have been inserted, respectively, into mammalian animal cells to transform them, and then by having them express and secret both of the heavy chain and common light chain.

Herein, any host cells for expressing the PD-1/CD3 bispecific antibody of the present invention can be used as long as they can be gene-introduced by expression vectors to express them. Preferable examples of host cells include insect cells such as SF-9 and SF-21, more preferably, mammalian cells such as mouse cells including CHO cells, BHK cells, SP2/0 cells and NS-0 myeloma cells, primate cells such as COS and Vero cells and MDCK cells, BRL 3A cells, hybridoma, tumor cells, immortalized primary cells, W138, HepG2, HeLa, HEK293, HT1080 and embryonic retina cells such as PER.C6, and the like. Note here that in selection of the expression system, expression vectors for mammalian cells and host cells therefor can often be used such that antibodies are appropriately glycosylated. Human cell lines, preferably, PER.C6 are advantageously used to obtain antibodies corresponding to glycosylated patterns for human.

Protein production in host cells transformed by gene-transferring the expression vectors can be carried out with reference to, for example, Current Protocols in Protein Science (1995), Coligan JE, Dunn BM, Ploegh HL, Speicher DW, Wingfield PT, ISBN 0-471-11184-8, Bendig, 1988. Further, general guidelines, procedures and practical methods to maximize the productivity of host cell culture can be carried out with reference to Mammalian Cell Biotechnology: a Practical Approach (M. Butler, ed., IRL Press, 1991). Expression of antibodies in host cells is described in, for example, publications such as EP0120694, EP0314161, EP0481790, EP0523949, US4816567, WO2000/63403 and the like.

Herein, culture conditions for host cells can be optimized by well-known methods, and the amount of protein production therein can be optimized. The culture can be carried out by batch culture, feeding culture, continuous culture or hollow-fiber culture in a petri dish, roller bottle or reaction chamber. In order to produce recombinant protein by cell culture in a large-scale and continuously, it is preferable to allow cells to proliferate in suspension. Furthermore, it is preferable to culture cells under a condition without any animal- or human-derived serum or animal- or human-derived serum components.

Antibodies expressed in host cells and recovered from them or culture thereof by well-known methods can be purified using well-known methods. Examples of purification method include immunoprecipitation method, centrifugation method, filtration, size-exclusion chromatography, affinity chromatography, cation and/or anion exchange chromatography, hydrophobic interaction chromatography and the like. Furthermore, protein A or protein G affinity chromatography may be preferably used (see, e.g., US4801687 and US5151504).

### [Pharmaceutical use]

The PD-1/CD3 bispecific protein of the present invention is useful for preventing, suppressing the progression of symptoms of or the recurrence of and/or treating hematological cancer.

Examples of hematological cancer which can be prevented, of which the progression of symptoms or the recurrence can be suppressed, and/or which can be treated by the PD-1/CD3 bispecific antibody of the present invention include multiple myeloma, malignant lymphoma (e.g., non-Hodgkin's Lymphoma (e.g., B-cell non-Hodgkin's lymphoma (e.g., precursor B-cell lymphoblastic lymphoma, precursor B-cell acute lymphoblastic leukemia, chronic B-lymphoid leukemia, B-cell prolymphocytic leukemia, lymphoplasmacytic lymphoma, nodal marginal zone B-cell lymphoma, extranodal marginal zone B-cell lymphoma, primary splenic marginal zone B-cell lymphoma, hairy cell leukemia, hairly cell leukemia-variant, follicular lymphoma, pediatric type follicular lymphoma, diffuse large B cell lymphoma, diffuse large B-cell lymphoma, not otherwise specified, splenic diffuse red pulp small B-cell lymphoma, lymphoplasmacytic lymphoma, primary mediastinal large B-cell lymphoma, primary effusion lymphoma, Burkitt's lymphoma, mantle cell lymphoma, monoclonal B-cell lymphocytosis, splenic B-cell lymphoma/leukemia, unclassifiable, IgM monoclonal gammopathy of undetermined significance, µ heavy chain disease, λ heavy chain disease, α heavy chain disease, plasma cell myeloma, solitary plasmacytoma of bone, extraosseous plasmacytoma, monoclonal immunoglobulin deposition disease, large B-cell lymphoma with IRF4 rearrangement, primary cutaneous follicle center lymphoma, T-cell/histiocyte-rich large B-cell lymphoma, primary diffuse large B-cell lymphoma of the central nervous system, primary cutaneous diffuse large B-cell lymphoma, leg type, EBV positive diffuse large B-cell lymphoma, not otherwise specified, EBV positive mucocutaneous ulcer, diffuse large B-cell lymphoma associated with chronic inflammation, lymphomatoid granulomatosis, intravascular large B-cell lymphoma, ALK positive large B-cell lymphoma, plasmablastic lymphoma, primary effusion lymphoma, HHV8 positive diffuse large B-cell lymphoma, not otherwise specified, Burkitt-like lymphoma with 11q aberration, high-grade B-cell lymphoma, with MYC and BCL2 and/or BCL6 rearrangement, high-grade B-cell lymphoma, not otherwise specified, or B-cell lymphoma, unclassifiable, with intermediate features between diffuse large B-cell lymphoma and classical Hodgkin lymphoma), T/NK-cell non-Hodgkin's lymphoma (e.g., precursor T-cell lymphoblastic lymphoma, chronic T-cell lymphocytic leukemia, T-cell acute lymphocytic leukemia (T-cell lymphoblastic leukemia), T-cell large granular lymphoblastic lymphoma, NK-cell large granular leukemia, aggressive NK-cell leukemia, peripheral T-cell lymphoma, peripheral T-cell lymphoma, not otherwise specified, unclassifiable peripheral T-cell lymphoma, angioimmunoblastic T-cell lymphoma, (CD30-positive) anaplastic large cell lymphoma, angiocentric lymphoma, intestinal T-cell lymphoma, enteropathy-type T-cell lymphoma, hepatosplenic gamma-delta T cell lymphoma, subcutaneous panniculitis-like T-cell lymphoma, mycosis fungoides, Sezary syndrome, Hodgkin-like/Hodgkin-related anaplastic large cell lymphoma, extranodal NK/T-cell lymphoma, adult T cell lymphoma, T-cell prolymphocytic leukemia, chronic lymphoproliferative disorder of NK-cells, systemic EBV positive T-cell lymphoma of childhood, hydroa vacciniforme-like lymphoproliferative disorder, extranodal NK/T-cell lymphoma, nasal type, enteropathy-associated T-cell lymphoma, monomorphic epitheliotropic intestinal T-cell lymphoma, indolent T-cell lymphoproliferative disorder of the GI tract, hepatosplenic T-cell lymphoma, primary cutaneous CD30 positive T-cell lymphoproliferative disorders, lymphomatoid papulosis, primary cutaneous anaplastic large cell lymphoma, primary cutaneous gamma-delta T-cell lymphoma, primary cutaneous CD8 positive aggressive epidermotropic cytotoxic T-cell lymphoma, primary cutaneous acral CD8 positive T-cell lymphoma, primary cutaneous CD4 positive small/medium T-cell lymphoproliferative disorder, follicular T-cell lymphoma, Nodal peripheral T-cell lymphoma with TFH phenotype, anaplastic large cell lymphoma, ALK positive, anaplastic large cell lymphoma, ALK negative, or breast implant-associated anaplastic large-cell lymphoma)), and Hodgkin's Lymphoma (e.g., classical Hodgkin's lymphoma (e.g., nodular sclerosis, mixed cellularity, lymphocyte-rich and lymphopenic) or nodular lymphocyte predominant Hodgkin's lymphoma)), leukemia (e.g., acute myelogenous leukemia, acute promyelocytic leukemia, acute lymphoblastic leukemia (lymphoblastic lymphoma), chronic lymphocytic leukemia (small lymphocytic lymphoma), myelodysplastic syndrome, and chronic myelogenous leukemia), primary central nervous system lymphoma, myeloproliferative syndrome and the like.

In the present invention, the term "treating" means cure or improvement of diseases or symptoms thereof. The term "preventing" means that the onset of diseases or symptoms thereof is prevented or delayed for a certain period of time. The term "suppressing of the progression of symptoms" means that the progress or aggravation of symptoms is suppressed to stop the progress of disease conditions. The meaning of "preventing" also includes suppressing the recurrence. The term "suppressing the recurrence" means that the recurrence of certain disease or syndrome thereof is prevented or a possibility of recurrence is reduced.

The PD-1/CD3 bispecific protein of the present invention is usually administered systemically or locally through parenteral administration. Specific examples of such administration methods include injection administration, intranasal administration, transpulmonary administration, percutaneous administration and the like. Examples of injection administration include intravenous injection, intramuscular injection, intraperitoneal injection and the like. For intravenous injection, drip intravenous infusion is preferable. The dose thereof varies depending on the age, body weight, symptoms, therapeutic effect, administration method, treating period and the like. The single dose thereof for an adult patient is usually within a range of 0.1 µg/kg to 300 mg/kg and particularly preferably within a range of 0.1 mg/kg to 10 mg/kg, once to several times per day by parenteral administration, or within a range of 30 minutes to 24 hours per day by intravenous sustaining administration. Needless to say, as mentioned above, since the dose varies depending on various conditions, it may be lower than the above-mentioned dose, or may be needed to be more than the above.

### Formulation

When the PD-1/CD3 bispecific protein of the present invention is formulated to be used as an injection or infusion solution for drip infusion, the injection or infusion solution may be in any form of an aqueous solution, suspension or emulsion, or may be formulated as a solid agent along with pharmaceutically acceptable carriers such that it can be dissolved, suspended or emulsified by adding a solvent at the time of use. Examples of solvents which can be used in the injection or the infusion solution for drip infusion include distilled water for injection, physiological saline, glucose solutions and isotonic solutions and the like (e.g., solutions in which sodium chloride, potassium chloride, glycerin, mannitol, sorbitol, boric acid, borax, propylene glycol or the like is dissolved.).

Herein, examples of the pharmaceutically acceptable carriers include a stabilizer, solubilizer, suspending agent, emulsifier, soothing agent, buffering agent, preservative, antiseptic agent, pH adjuster, antioxidant and the like. As a stabilizer, for example, various amino acids, albumin, globulin, gelatin, mannitol, glucose, dextran, ethylene glycol, propylene glycol, polyethylene glycol, ascorbic acid, sodium bisulfite, sodium thiosulfate, sodium edetate, sodium citrate, dibutylhydroxytoluene or the like can be used. As a solubilizer, for example, alcohol (e.g., ethanol etc.), polyalcohol (e.g., propylene glycol and polyethylene glycol, etc.), nonionic surfactant (e.g., Polysorbate 20 (registered trademark), Polysorbate 80 (registered trademark) and HCO-50, etc.) or the like can be used. As a suspending agent, for example, glyceryl monostearate, aluminum monostearate, methyl cellulose, carboxymethyl cellulose, hydroxymethyl cellulose, sodium lauryl sulfate or the like can be used. As an emulsifier, for example, gum arabic, sodium alginate, tragacanth or the like can be used. As a soothing agent, for example, benzyl alcohol, chlorobutanol, sorbitol or the like can be used. As a buffering agent, for example, phosphate buffer, acetate buffer, borate buffer, carbonate buffer, citrate buffer, Tris buffer, glutamic acid buffer, epsilon aminocaproic acid buffer or the like can be used. As a preservative, for example, methyl parahydroxybenzoate, ethyl parahydroxybenzoate, propyl parahydroxybenzoate, butyl parahydroxybenzoate, chlorobutanol, benzyl alcohol, benzalkonium chloride, sodium dehydroacetate, sodium edeate, boric acid, borax or the like can be used. As an antiseptic agent, for example, benzalkonium chloride, parahydroxybenzoic acid, chlorobutanol or the like can be used. As a pH adjuster, for example, hydrochloric acid, sodium hydroxide, phosphoric acid, acetic acid or the like can be used. As an antioxidant, for example, (1) aqueous antioxidants such as ascorbic acid, cysteine hydrochloride, sodium bisulfate, sodium metabisulfite and sodium sulfite, (2) oil-soluble antioxidants such as ascorbyl palmitate, butylated hydroxy anisole, butylated hydroxyl toluene, lecithin, propyl gallate and α-tocopherol and (3) metal chelating agents such as citric acid, ethylenediaminetetraacetic acid, sorbitol, tartaric acid and phosphoric acid can be used.

The injection or infusion solution for drip infusion can be produced by performing sterilization in the final process, or aseptic manipulation, for example, sterilization by filtration with a filter or the like and subsequently filling it to an aseptic container. The injection or infusion solution for drip infusion may be used by dissolving the vacuum dried and lyophilized aseptic powder (which may include pharmaceutically acceptable carrier powders) in an appropriate solvent at the time of use.

### Combination use or combination formulation

Furthermore, the PD-1/CD3 bispecific protein of the present invention may be used in combination with other agents which is used for preventing, suppressing the progression of symptoms of or the recurrence of and/or treating hematological cancer. In the present invention, examples of administration forms in combinational use with other agents (combinational use) may include a form of combination formulation containing both of ingredients in one formulation and a form being administered in separate formulations. Such combinational uses can complement the effect on preventing, suppressing the progression of symptoms of, suppressing the recurrence of and/or treating by other agents, or can maintain or reduce the dose or frequency of administration of other agents. When separately administering the PD-1/CD3 bispecific protein of the present invention and other agents, they may be simultaneously administered for a certain period of time, and then only the PD-1/CD3 bispecific protein or other agents may be administered. Alternatively, the PD-1/CD3 bispecific protein of the present invention may be initially administered, and after completion of administration thereof, other agents may be administered. Other agents may be initially administered, and after completion of administration thereof, the PD-1/CD3 bispecific protein of the present invention may be administered. The respective administration methods thereof may be the same as or different from each other. A kit containing a formulation containing the PD-1/CD3 bispecific protein of the present invention and a formulation containing other agents can also be provided. Herein, the doses of other agents can be appropriately selected based on the dose in clinical use. Further, other agents may be administered in combination of two or more kinds of arbitrary agents at an appropriate ratio. Furthermore, examples of other agents include not only those already known but also those newly discovered in the future.

For example, it may be used in combination with, for example, any one or more of agents selected from an alkylating agent (e.g., Dacarbazine, Nimustine, Temozolomide, Fotemustine, Bendamustine, Cyclophosphamide, Ifosfamide, Carmustine, Chlorambucil and Procarbazine, etc.), platinum agent (e.g., Cisplatin, Carboplatin, Nedaplatin and Oxaliplatin, etc.), antimetabolite (e.g., antifolate (e.g., Pemetrexed, Leucovorin and Methotrexate, etc.), pyridine metabolism inhibitor (e.g., TS-1 (registered Trademark), 5-fluorouracil, UFT, Carmofur, Doxifluridine, FdUrd, Cytarabine and Capecitabine, etc.), purine metabolism inhibitor (e.g., Fludarabine, Cladribine and Nelarabine, etc.), ribonucleotide reductase inhibitor, nucleotide analog (e.g., Gemcitabine etc.)), topoisomerase inhibitor (e.g., Irinotecan, Nogitecan and Etoposide, etc.), microtubule polymerization inhibitor (e.g., Vinblastine, Vincristine, Vindesine, Vinorelbine and Eribulin, etc.), microtubule depolymerization inhibitor (e.g., Docetaxel and Paclitaxel, etc.), antitumor antibiotic (e.g., Bleomycin, Mitomycin C, Doxorubicin, Daunorubicin, Idarubicin, Etoposide, Mitoxantrone, Vinblastine, Vincristine, Peplomycin, Amrubicin, Aclarubicin and Epirubicin, etc.), cytokine preparation (e.g., IFN-α2a, IFN-α2b, pegylated IFN-α2b, natural IFN-β and interleukin-2, etc.), molecular targeting drug, cancer immunotherapeutic drug, other antibody drugs and the like.

Herein, examples of the molecular targeting drugs include an ALK inhibitor (e.g., Crizotinib, Ceritinib, Ensartinib, Alectinib and Lorlanib, etc.), BCR-ABL inhibitor (e.g., Imatinib and Dasatinib, etc.), AXL inhibitor (e.g., ONO-7475 and Bemcentinib, etc.), CDK inhibitor (e.g., Dinaciclib, Abemaciclib, Palbociclib and trilaciclib, etc.), BTK inhibitor (e.g., Ibrutinib and Acarabultinib, etc.), PI3K-δ/γ inhibitor (e.g., Umbralisib, Parsaclisib and IPI-549, etc.), JAK-1/2 inhibitior (e.g., Itacitinib and Ruxolitinib, etc.), FAK inhibitor (e.g., Defactinib etc.), Syk/FLT3 dual inhibitor (e.g., Mivavotinib etc.), ATR inhibitor (e.g., Ceralasertib etc.), WEE1 kinase inhibitor (e.g., Adavosertib etc.), mTOR inhibitor (e.g., Temsirolimus, Everolimus, Vistusertib and Irinotecan, etc.), HDAC inhibitor (e.g., Vorinostat, Romidepsin, Entinostat, Chidamide, Mocetinostat, Citarinostat, Panobinostat and Valproate, etc.), EZH2 Inhibitor (e.g., Tazemetostat etc.), STAT3 Inhibitor (e.g., Napabucasin etc.), DNMT Inhibitor (e.g., Azacitidine etc.), BCL-2 inhibitor (e.g., Navitoclax and Venetoclax, etc.), SMO Inhibitor (e.g., Vismodegib etc.), anti-CD20 antibody (e.g., Rituximab, Blontuvetmab, Epitumomab, Ibritumomab tiuxetan, Ocaratuzumab, Ocrelizumab, Technetium (⁹⁹mTc) nofetumomab merpentan, Tositumomab, Veltuzumab, Ofatumumab, Ublituximab, Obinutuzumab and Nofetumomab, etc.), anti-CD30 antibody (e.g., Brentuximab vedotin and Iratumumab, etc.), anti-CD38 antibody (e.g., Daratumumab, Isatuximab, Mezagitamab, AT13/5 and MOR202, etc.), anti-TNFRSF10B antibody (e.g., Benufutamab, Conatumumab, Drozitumab, Lexatumumab, Tigatuzumab, Eftozanermin alfa and DS-8273a, etc.), anti-CD40 antibody (e.g., Bleselumab, Dacetuzumab, Iscalimab, Lucatumumab, Mitazalimab, Ravagalimab, Selicrelumab, Teneliximab, ABBV-428 and APX005M, etc.), anti-CD70 antibody (e.g., Cusatuzumab, Vorsetuzumab, Vorsetuzumab mafodotin and ARGX-110, etc.), anti-TNFRSF10A antibody (e.g., Mapatumumab etc.), anti-CD79b antibody (e.g., Iladatuzumab, Iladatuzumab vedotin and Polatuzumab vedotin, etc.), anti-TNFSF11 antibody (e.g., Denosumab etc.), anti-NCAM1 antibody (e.g., Lorvotuzumab mertansine etc.), anti-CD37 antibody (e.g., Lilotomab, Lutetium (¹⁷⁷lu) lilotomab satetraxetan, Naratuximab, Naratuximab emtansine and Otlertuzumab, etc.), anti-CD200 antibody (e.g., Samalizumab etc.), anti-CD30-CD16A bispecific antibody (e.g., AFM13 etc.), anti-IL3RA-CD3 bispecific antibody (Flotetuzumab and Vibecotamab), anti-GPRC5D-CD3 bispecific antibody (Talquetamab), anti-CD3-CD19 bispecific antibody (e.g., Duvortuxizumab and Blinatumomab), anti-TNFRSF 17-CD3 bispecific antibody (Teclistamab), anti-CLEC12A-CD3 bispecific antibody (Tepoditamab) and anti-CD20-CD3 bispecific antibody (e.g., Plamotamab, Odronextamab, Mosunetuzumab, Glofitamab, Epcoritamab and REGN1979, etc.) and the like.

Further, examples of the cancer immunotherapeutic drugs include an anti-PD-1 antibody (e.g., Nivolumab, Cemiplimab, Penbrolizumab, Spartalizumab, Tislelizumab, Dostarlimab, Toripalimab, Camrelizumab, Genolimzumab, Sintilimab, Lodapolimab, Retifanlimab, Balstilimab, Serplulimab, Budigalimab, Prolgolimab, Sasanlimab, Cetrelimab, Zimberelimab, AMP-514, STI-A1110, ENUM 388D4, ENUM 244C8, GLS1010, CS1003, BAT-1306, AK105, AK103, BI 754091, LZM009, CMAB819, Sym021, GB226, SSI-361, JY034, ISU106, HX008 and CX-188, etc.), anti-PD-L1 antibody (e.g., Atezolizumab, Avelumab, Durvalumab, Manelimab, Pacmilimab, Envafolimab, Cosibelimab, BMS-936559, STI-1010, STI-1011, STI-1014, SHR-1316, CS1001, MSB2311, BGB-A333, KL-A167, AK106, AK104, ZKAB001, FAZ053, CBT-502 and JS003, etc.), PD-1 antagonist (e.g., AUNP-12, the respective compounds of BMS-M1 to BMS-M10, BMS-1, BMS-2, BMS-3, BMS-8, BMS-37, BMS-200, BMS-202, BMS-230, BMS-242, BMS-1001, BMS-1166, the respective compounds of Incyte-1 to Incyte-6, the respective compounds of CAMC-1 to CAMC-4, RG_1 and DPPA-1, etc.), PD-L1/VISTA antagonist (e.g., CA-170), PD-L1/TIM3 antagonist (e.g., CA-327), anti-PD-L2 antibody, PD-L1 fusion protein, PD-L2 fusion protein (e.g., AMP-224 etc.), anti-CTLA-4 antibody (e.g., Ipilimumab, Zalifrelimab, Nurulimab and Tremelimumab, etc.), anti-LAG-3 antibody (e.g., Relatlimab, Ieramilimab, Fianlimab, Encelimab and Mavezelimab, etc.), anti-TIM3 antibody (e.g., MBG453 and Cobolimab, etc.), anti-KIR antibody (e.g., Lirilumab, IPH2101, LY3321367 and MK-4280, etc.), anti-BTLA antibody, anti-TIGIT antibody (e.g., Tiragolumab, Etigilimab, Vibostolimab and BMS-986207, etc.), anti-VISTA antibody (e.g., Onvatilimab etc.), anti-CD137 antibody (e.g., Urelumab and Utomilumab, etc.), anti-CSF-1R antibody or CSF-1R inhibitor (e.g., Cabiralizumab, Ecactuzumab, LY3022855, Axatilimab, MCS-110, IMC-CS4, AMG820, Pexidartinib, BLZ945 and ARRY-382, etc.), anti-OX40 antibody (e.g., MEDI 6469, Ivuxolimab, MEDI0562, MEDI6383, Efizonerimod, GSK3174998, BMS-986178 and MOXR0916, etc.), anti-HVEM antibody, anti-CD27 antibody (e.g., Varlilumab etc.), anti-GITR antibodies or GITR fused protein (e.g., Efaprinermin alfa, Efgivanermin alfa, MK-4166, INCAGN01876, GWN323 and TRX-518, etc.), anti-CD28 antibody, anti-CCR4 antibody (e.g., Mogamulizumab etc.), anti-B7-H3 antibody (e.g., Enoblituzumab, Mirzotamab, Mirzotamab clezutoclax and Omburtamab, etc.), anti-ICOS agonist antibody (e.g., Vopratelimab and GSK3359609, etc.), anti-CD4 antibody (e.g., Zanolimumab and IT1208, etc.), anti-DEC-205 antibody/NY-ESO-1 fusion protein (e.g., CDX-1401 etc.), anti-SLAMF7 antibody (e.g., Azintuxizumab, Azintuxizumab vedotin and Elotuzumab, etc.), anti-CD73 antibody (e.g., Oleclumab and BMS-986179, etc.), pegylated IL-2 (Bempegaldesleukin), anti-CD40 agonist antibody (e.g., ABBV-428, APX005M and RO7009789, etc.), IDO inhibitor (e.g., Epacadostat, Indoximod and Linrodostat, etc.), TLR agonist (e.g., Motolimod, CMP-001, G100, Tilsotolimod, SD-101 and MEDI9197, etc.), adenosine A2A receptor antagonist (e.g., Preladenant, AZD 635, Taminadenant and Ciforadenant, etc.), anti-NKG2A antibody (e.g., Monalizazumab etc.), anti-CSF-1 antibody (e.g., PD036032 etc.), immunopotentiating agent (e.g., PV-10 etc.), IL-15 superagonist (e.g., ALT-803), soluble LAG3 (e.g., IMP 321 etc.), anti-CD47 antibody or CD47 antagonist (e.g., ALX148 etc.), IL-12 antagonist (e.g., M9241 etc.) and the like.

Furthermore, examples of the antibody drugs include anti-IL-1β antibodies (e.g., Canakinumab etc.), anti-CCR2 antibodies (e.g., Plozalizumab etc.) and the like.

Furthermore, the PD-1/CD3 bispecific protein of the present invention may be used in combination with a steroid agent (e.g., cortisone, cortisone acetate, hydrocortisone, hydrocortisone sodium phosphate, hydrocortisone sodium succinate, fludrocortisone acetate, prednisolone, prednisolone acetate, prednisolone sodium succinate, prednisolone butylacetate, prednisolone sodium phosphate, halopredone acetate, methylprednisolone, methylprednisolone acetate, methylprednisolone sodium succinate, triamcinolone, triamcinolone acetate, triamcinolone acetonide, dexamethasone, dexamethasone acetate, dexamethasone valerate, dexamethasone cipecilate, dexamethasone propionate, dexamethasone sodium phosphate, dexamethasone palmitate, dexamethasone sodium metasulfobenzoate, parameterzone, parameterzone acetate, betamethasone, betamethasone dipropionate, betamethasone valerate, betamethasone acetate, betamethasone butyrate propionate, betamethasone sodium phosphate and the like).

The present invention will now be described in more detail by the following examples, but the scope of the present invention is not limited thereto. A person skilled in the art can make various changes and modifications, based on the description of the present invention, and such changes and modifications are also included in the present invention.

### [Examples]

### Example 1: Immunization of MeMo (registered trademark) mice using recombinant human PD-1-Fc fusion protein

When the first arm specifically binding to PD-1 of the present invention consists of a part of antibody, as a method to obtain it, a method for immunizing MeMo (registered trademark) mice (see WO2009/157771) with a recombinant human PD-1 protein was selected. The MeMo (registered trademark) mice are those genetically modified such that a gene fragment containing a non-recombinant human heavy chain V gene region, D gene region and J gene region, as well as the recombinant human κ light chain IgVκ1-39^{∗}01/IGJκ1^{∗}01 germ-line gene have been linked to a mouse constant region gene. By directly immunizing it with a target protein for antibody, antibodies composed of heavy chains and common light chains, with diversity, can be produced.

Twelve 12 to 16-week-old MeMo (registered trademark) MS5B/MS9 mice were immunized with recombinant human PD-1-Fc fusion protein (R&D Systems, serial number 1086-PD) emulsified using Gerbu adjuvant MM (Gerbu Biotechnik, serial number #3001) at an interval of 14 days. On days 0, 14 and 28 after immunization, the recombinant human PD-1-Fc fusion protein was subcutaneously administered, and thereafter, the recombinant human PD-1-Fc fusion protein dissolved in PBS was administered subcutaneously. On days 21, 35, 56, 77 and 98 after immunization, the antibody titer in serum was evaluated by flow cytometry using human PD-1-forced expressed HEK293 T cell lines. When the human PD-1-forced expressed HEK293T cell lines were stained with 1000-fold diluted serum, mouse lymph tissues of which the MFI value increased more than three times higher than that of the human PD-1 non-expressing HEK293T cell lines as a control were used for constructing a phage display library. Mice meeting the criteria for constructing the library were additionally immunized with the recombinant PD-1-Fc fusion protein for three days from the evaluation date of the antibody titer, and of which spleens and inguinal lymph nodes were collected. Spleens and inguinal lymph nodes were also collected from mice in which the antibody titer in serum to human PD-1 and cynomolgus monkey PD-1 was 1/100 or more, and the antibody titer was not increased by additional immunization. RNA was extracted from those lymphoid tissues, and then cDNA synthesis was carried out.

### Example 2: Construction of phage display library to obtain an anti-PD-1 antibody having the first arm specifically binding to PD-1 (protein immunization)

Using the DNA prepared in Example 1 and primers specific to immunoglobulin heavy chain variable region family, PCR reaction was carried out. The obtained PCR products were digested with restriction enzymes SfiI and XhoI, and inserted into MV1473 phagemid vector [having a gene (human κ light chain IgVκ1-39^{∗}01/IGJκ1^{∗}01 germ-line gene) encoding the common light chain] digested with the same restriction enzymes to construct the library.

### Example 3: Screening of an anti-PD-1 antibody having the first arm specifically binding to PD-1

Using plates coated with human PD-1-Fc fusion protein, human PD-1-His tag fusion protein, cynomolgus monkey PD-1-His tag fusion protein or mouse PD-1-His tag fusion protein, phage selection based on the binding property to PD-1 was carried out. When using human PD-1-Fc fusion protein, during incubation with phage, human IgG (SIGMA, serial number 14506) was added thereto to absorb Fc reactive clones. Binding phages capable of binding to human PD-1, cynomolgus monkey PD-1 and mouse PD-1 were enriched. Using selections on cynomolgus monkey PD-1 expressing HEK293 T cell lines, phages capable of binding to cynomolgus monkey PD-1 were enriched. Escherichia coli strain TG1 clones transformed with phages obtained by selection were obtained to produce a master plate.

Further, based on the binding property to PD-1 on a plate to which human PD-1-Fc fusion protein was adsorbed, the phage selection from periplasmic space extracts of the clones obtained by the above-mentioned selection was carried out. Note here that as the criteria for selection, clones with signals three times more than that (OD₄₅₀ value) in a negative control well (PBS) were defined as positive clones.

### Example 4: DNA sequencing of candidate clones for anti-PD-1 antibodies having the first arm specifically binding to PD-1

DNA sequencing for heavy chain variable region genes of positive clones obtained by screening in Example 3 was carried out. Analyzed DNA sequences were classified into super clusters (a group having the same-length CDR3, in which an amino acid sequence of CDR3 is 70% or more homologous each other) and clusters (a group in which amino acid sequences of the heavy chain CDR3 are same). 924 clones were obtained, which were classified into 146 types super clusters and 194 types clusters.

### Example 5: Screening based on the evaluation of the binding property to PD-1 expressing cells

From the respective classified super clusters, anti-PD-1 monoclonal antibody clones meeting the following conditions were screened and isolated
(1) having somatic mutations in CDRs with high frequency,
(2) having a germline gene of highly frequently used VH, and
(3) having a high signal in the screening based on the binding property to human PD-1-Fc fusion protein.

Using Fab fragments contained in those periplasmic space extracts, the binding properties to human PD-1 expressing CHO-S cell lines and cynomolgus monkey PD-1 expressing CHO-S cell lines were evaluated by detecting with anti-mouse IgG polyclonal antibodies. Among the evaluated 117 clones (105 types of clusters), in 22 clones including the anti-PD-1 monoclonal antibody clones PD1-1, PD1-2, PD1-3 and PD1-4, the bindings to the human PD-1 expressing CHO-S cell lines were detected.

### Example 6: Preparation of amino acid-substituted products of the anti-PD-1 monoclonal antibody having the first arm specifically binding to PD-1

The clones PD1-1 and PD1-4 contain deamidation motifs (Asn-Gly) in the framework 4 of heavy chain variable region thereof, respectively. In order to obtain the first arm with reduced risk of deamidation, a variant in which the deamidation motifs have been converted was prepared. Asparagine (Asn) at position 119 according to the EU numbering system for the clone PD1-4 was altered to glutamine by a well-known site-specific mutation method, to prepare and isolate the clone PD1-5. The binding property to human PD-1 expressing CHO-S cells of the same clone was equal to that of the PD1-4 clone.

### Example 7: Screening of anti-CD3 monoclonal antibodies having the second arm specifically binding to CD3

In order to obtain Fabs binding to CD3 having more stability and further reduced charge heterogeneity, using the anti-CD3 antibody clone having the VH of the anti-CD3 antibody 15C3 clone described in WO2005/118635 and IGVK1-39/JK1 common light chain, an anti-CD3 antibody having the "second arm specifically binding to CD3" of the present invention was obtained by the following method.

By converting the underlined 55th glycine in the VH amino acid sequence of 15C3 shown in Figure 10 into alanine, the anti-CD3 antibody clone CD3-1, having the binding property to human CD3 equal to the above-mentioned clone and having improved charge heterogeneity was obtained.

Further, in order to obtain a plurality of Fabs binding to CD3, capable of improving the VH/VL interaction with the common light chain (IgVκ1-39^{∗}01/IGJκ1^{∗}01), a phage display library expressing a plurality of Fabs consisted of VH variants of the CD3-1 clone in which amino acid residues thereof have been substituted was constructed, based on the VH of CD3-1 clone. This phage library was screened using HBP-ALL cells or recombinant human CD3ε-Fc protein. Phages binding to recombinant human CD3ε-Fc protein were chemically eluted, and used for reinfection to bacteria. A plurality of survived bacterial colonies were extracted, then phages therefrom were extracted, and then screened by flow cytometry, based on the binding to CD3 expressed on cell surface. All of the phages binding to CD3 were subjected to colony PCR to amplify cDNAs encoding VHs thereof, and DNA sequences thereof were determined, respectively.

As a result, the obtained anti-CD3 antibody clone CD3-2 having the VH comprising the amino acid sequence set forth in SEQ ID No. 36 showed the binding property to CD3 and charge homogeneity equivalent to those of the clone CD3-1.

### Example 8: Preparation of the PD-1/CD3 bispecific monoclonal antibody

When the first arm specifically binding to PD-1 of the present invention consists of a part of antibody, expression vectors expressing the respective heavy chains of the first arm specifically binding to PD-1 were prepared by linking DNAs encoding heavy chain variable regions of the anti-PD-1 monoclonal antibody clones PD1-1 to PD1-5 and PD1-6 selected in Example 5, respectively, to DNAs encoding IgG₁ heavy chain constant regions, respectively. On the other hand, an expression vector expressing the heavy chain of the second arm specifically binding to CD3 was prepared by linking a DNA encoding the heavy chain variable region of the anti-CD3 monoclonal antibody clone CD3-2 selected in Example 7 to a DNA encoding an IgG₁ heavy chain constant region. Herein, as genes expressing the heavy chain constant region, as to the first arm specifically binding to PD-1, a gene expressing Fc region having L351D/L368E variation (DE variation) was used. As to the second arm specifically binding to CD3, a gene expressing Fc region having L351K/T366K variation (KK variation) was used. These expression vectors were constructed so as to further contain a gene encoding the IGVK1-39/JK1 common light chain such that it is expressed together. Further, in order to eliminate the Fc effector activity, the genes expressing these heavy chain constant regions have been modified so as to be expressed as those in which leucine at position 235 was substituted with glycine and further glycine at position 236 was substituted with arginine in the heavy chain constant regions, and furthermore in order to avoid processing after translation, those modified so as to be expressed as those in which lysine at a position 447 of the C-terminus of the heavy chain constant region was deleted were used. Both of these expression vectors were gene-transferred into Free Style 293F cells to make them produce antibodies in culture supernatants. The culture supernatants were collected and then treated by protein A affinity chromatography, to purify the clones PD1-1(Bi), PD1-2(Bi), PD1-3(Bi), PD1-4(Bi) and PD1-5(Bi) as the PD-1/CD3 bispecific monoclonal antibody of the present invention, respectively. Furthermore, the clone PD1-6(Bi) was also produced by the same method. Note here that, in that having the first arms specifically binding to PD-1 derived from the anti-PD-1 monoclonal antibody clones PD1-1, PD1-2, PD1-3, PD1-4 and PD1-5, as well as the clone PD1-6, used in production thereof, these PD-1/CD3 bispecific monoclonal antibodies correspond to those anti-PD-1 monoclonal antibody clones, respectively.

### Example 9: Evaluation of the binding property of the PD-1/CD3 bispecific monoclonal antibody

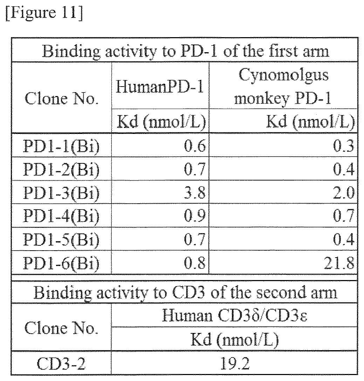

By Biacore (registered trademark) assay using human IgG1-Fc fused human PD-1 extracellular recombinant protein or 6×His tag fused cynomolgus monkey PD-1 extracellular recombinant protein, the binding affinities to the respective PD-1 recombinant proteins of the first arm of the PD-1/CD3 bispecific monoclonal antibodies obtained in Example 8 were evaluated. Note here that Series S Sensor Chip CM5 Sensor Chip (GE Health Care, serial number 29-1049-88) was used for immobilization of the recombinant proteins. Similarly, by Biacore (registered trademark) assay using human IgG1-Fc fused CD3δ/CD3ε extracellular recombinant protein, the binding affinity to CD3 of the second arm of the same antibody was evaluated. Figure 11 shows the binding affinities (Kd value) to PD-1 of the first arms and the binding affinity to CD3δ/CD3ε of the second arm with respect to the respective clones.

### Example 10: Verification of the binding property of the PD-1/CD3 bispecific monoclonal antibody

It was verified that the PD-1/CD3 bispecific monoclonal antibodies obtained in Example 8 specifically, simultaneously bind to PD-1 and CD3, respectively.

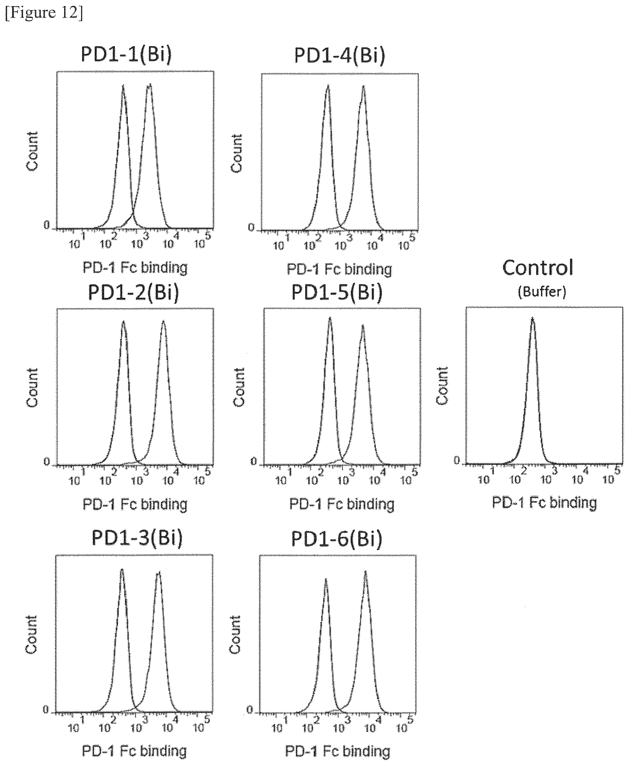

Initially, the clones PD1-1(Bi) to PD1-6(Bi) were added to human PD-1-deficient Jurkat cell lines (human T cell line) expressing human CD3, respectively, and which were incubated on ice for 15 minutes. After those cells were washed, soluble PD-1 recombinant proteins (R&D systems, serial number 1086-PD-050) labeled with 3-fold amount of biotin was added thereto, and which were incubated on ice for 15 minutes. After those cells were washed again, 100 µL of 1.25 µg/mL Alexa Fluor 488-labeled streptavidin (BioLegend, serial number 405235) was added thereto, and which were incubated on ice for 15 minutes. After those cells were further washed, the binding amounts of soluble PD-1 recombinant protein were evaluated by flow cytometry, respectively. Figure 12 shows results thereof in this assay.

All of the clones bound to PD-1 and CD3, simultaneously. Note here that no nonspecific binding in this binding system was detected.

### Example 11: Evaluation of the binding property of the first arm of the PD-1/CD3 bispecific monoclonal antibody

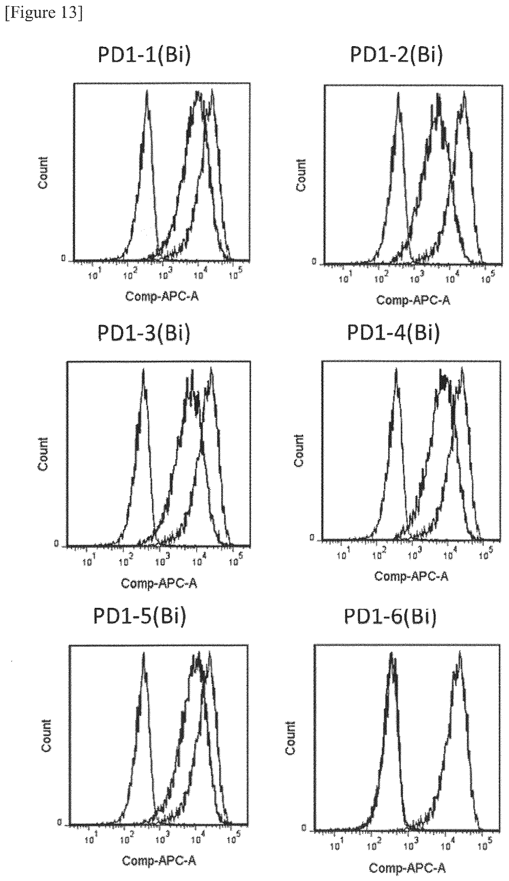

In order to evaluate effects on the PD-1/PD-L1 interaction of the first arms of the PD-1/CD3 bispecific monoclonal antibodies obtained in Example 8, the competitive binding assay with respect to the binding to PD-1 of the bispecific monoclonal antibody clones and soluble PD-L1 recombinant protein to PD-1 was carried out. Initially, the clones PD1-1(Bi) to PD1-6(Bi) were added to human PD-1 expressing CHO-S cell lines, respectively, and which were incubated on ice for 30 minutes. After those cells were washed, soluble PD-L1 recombinant proteins (R&D systems, serial number 156-B7-100) labeled with 1/20 amount of biotin was added thereto, and which were incubated on ice for 30 minutes. After those cells were washed again, PE-labeled streptavidin (BD Pharmingen, serial number 554061) was added thereto, and which were incubated on ice for 30 minutes. After those cells were further washed, the binding amounts of soluble PD-L1 recombinant proteins were evaluated by flow cytometry, respectively. Figure 13 shows results thereof.

Although the clones PD1-1(Bi) to PD1-5(Bi) were in the amount of 20 times more than that of soluble PD-L1 recombinant protein, they allowed the binding of soluble PD-L1 recombinant protein to PD-1. On the other hand, the clone PD1-6(Bi) completely inhibited the binding of soluble PD-L1 recombinant protein to PD-1 at the same conditions.

### Example 12: Evaluation of cross-competitive property of PD1-5(Bi) for binding to PD-1 of the respective clones of the PD-1/CD3 bispecific monoclonal antibodies

Cross-competition assay was conducted to evaluate the cross-competitive properties of PD1-5(Bi) for binding to PD-1 of the respective clones PD1-1(Bi) to PD1-5(Bi) of the PD-1/CD3 bispecific monoclonal antibody, obtained in Example 8.

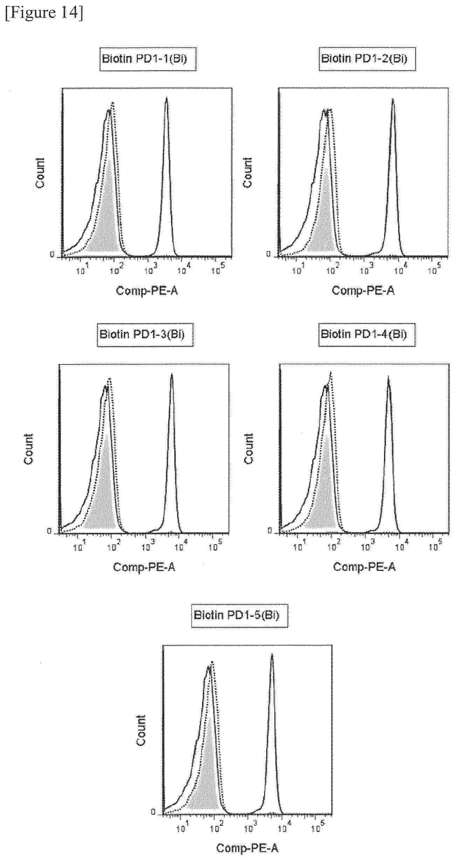

Initially, the clone PD1-5(Bi) was added to human PD-1-expressing CHO-S line cells, then which were incubated on ice for 20 minutes. Further, 1/100 amount of the biotin-labeled clones PD1-1(Bi) to PD1-5(Bi) compared to that of the already-added clone PD1-5(Bi) were added thereto, then which were further incubated on ice for 20 minutes. Those cells were washed, PE-labeled streptavidin (BD Pharmingen, model number 554061) was added thereto, and then which were incubated on ice for 20 minutes. After washing those cells again, the binding amounts of the biotin-labeled clones PD1-1(Bi) to PD1-5(Bi) were measured using flow cytometry. Figure 14 shows results thereof.

It was demonstrated that the clone PD1-5(Bi) can inhibit the respective bindings to PD-1 of the clones PD1-1(Bi) to PD1-5(Bi), and thus that the clone PD1-5(Bi) can cross-compete with their bindings to PD-1.

### Example 13: Suppressive effects of the PD-1/CD3 bispecific monoclonal antibody against cell proliferation of B cell lymphoma cell lines

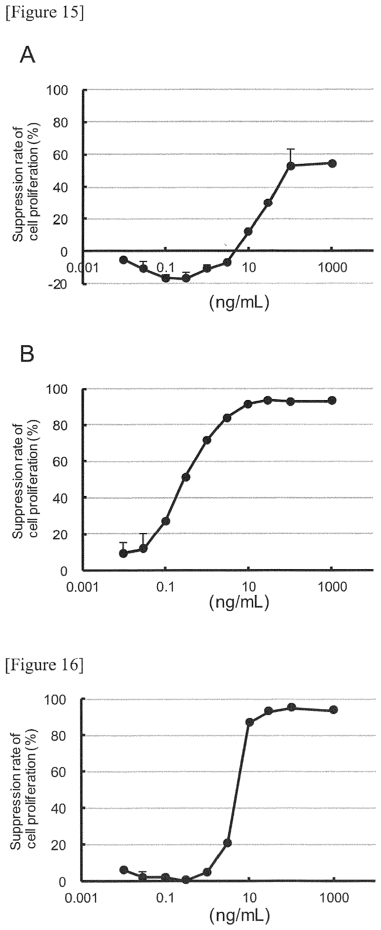

Using pan-T cells (STEMCELL, serial number ST-70024) (human T cells) derived from peripheral blood of healthy persons, the suppressive effect against cell proliferation of B cell lymphoma cell lines was evaluated. On U-bottom 96-well plates, human T cells and B-cell lymphoma cell line EB-1 or human PD-1-forced expressing SU-DHL-4 labeled with Vybrant DiD Cell-Labeling Solution (Thermo Fisher, serial number V22887) were seeded, and the clone PD1-5(Bi) as the PD-1/CD3 bispecific monoclonal antibody was added thereto, and then co-cultured for 48 hours. The cells were collected from each well, stained with Cell Viability Solution, and the number of DiD-positive viable cells was counted by flow cytometer. The number of DiD-positive living cells in the antibody-free sample was set to 100%, and the reduction rates of DiD-positive living cells in the samples to which the PD1-5(Bi) was added were calculated as the suppression rate against cell proliferation. Figures 15A and 15B show results thereof, respectively. The cell proliferations of B cell lymphoma cell lines were all suppressed by addition of PD1-5(Bi).

### Example 14: Suppressive effects of the PD-1/CD3 bispecific monoclonal antibody against cell proliferation of multiple myeloma cell line

Using pan-T cells (STEMCELL, serial number ST-70024) (human T cells) derived from peripheral blood of healthy persons, the suppressive effect against cell proliferation of multiple myeloma cell line was evaluated. On U-bottom 96-well plates, human T cells and multiple myeloma cell line RPMI8226 labeled with Vybrant DiD Cell-Labeling Solution (Thermo Fisher, serial number V22887) were seeded, and the clone PD1-5(Bi) as the PD-1/CD3 bispecific monoclonal antibody was added thereto, and then co-cultured for 48 hours. The cells were collected from each well, stained with Cell Viability Solution, and the number of DiD-positive viable cells was counted by flow cytometer. The number of DiD-positive living cells in the antibody-free sample was set to 100%, and the reduction rate of DiD-positive living cells in the sample to which the PD1-5(Bi) was added was calculated as the suppression rate against cell proliferation. Figure 16 shows results thereof. The cell proliferation of RPMI8226 was suppressed by addition of PD1-5(Bi).

### Example 15: Suppressive effects of the PD-1/CD3 bispecific monoclonal antibody against cell proliferation of adult T-cell lymphoma cell line

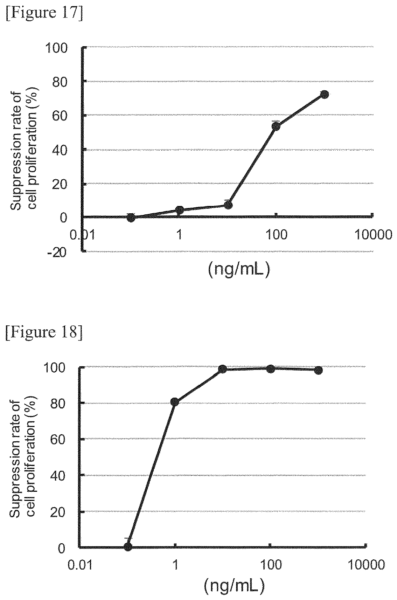

Using pan-T cells (STEMCELL, serial number ST-70024) (human T cells) derived from peripheral blood of healthy persons, the suppressive effect on cell proliferation of adult T-cell lymphoma cell line was evaluated. On U-bottom 96-well plates, human T cells and adult T-cell lymphoma cell line ILT-Mat labeled with Vybrant DiD Cell-Labeling Solution (Thermo Fisher, serial number V22887) were seeded, and the clone PD1-5(Bi) as the PD-1/CD3 bispecific monoclonal antibody was added thereto, and then co-cultured for 48 hours. The cells were collected from each well, stained with Cell Viability Solution, and the number of DiD-positive viable cells was counted by flow cytometer. The number of DiD-positive living cells in the antibody-free sample was set to 100%, and the reduction rate of DiD-positive living cells in the sample to which the PD1-5(Bi) was added was calculated as the suppression rate against cell proliferation. Figure 17 shows results thereof. The cell proliferation of ILT-Mat was suppressed by addition of PD1-5(Bi).

### Example 16: Suppressive effects of the PD-1/CD3 bispecific monoclonal antibody against cell proliferation of acute T cell leukemia cell line

Using pan-T cells (STEMCELL, serial number ST-70024) (human T cells) derived from peripheral blood of healthy persons, the suppressive effect on cell proliferation of adult T-cell lymphoma cell line was evaluated. On U-bottom 96-well plates, human T cells and human PD-1-forced expressing Jurkat (acute T cell leukemia cell line) labeled with Vybrant DiD Cell-Labeling Solution (Thermo Fisher, serial number V22887) were seeded, and the clone PD1-5(Bi) as the PD-1/CD3 bispecific monoclonal antibody was added thereto, and then co-cultured for 48 hours. The cells were collected from each well, stained with Cell Viability Solution, and the number of DiD-positive viable cells was counted by flow cytometer. The number of DiD-positive living cells in the antibody-free sample was set to 100%, and the reduction rate of DiD-positive living cells in the sample to which the PD1-5(Bi) was added was calculated as the suppression rate against cell proliferation. Figure 18 shows results thereof. The cell proliferation of human PD-1-forced expressing Jurkat was suppressed by addition of PD1-5(Bi).

### Example 17: Preparation of the PD-1/CD3 bispecific monoclonal antibody with amino acid mutations at the binding site to human FcRn and evaluation of the binding property thereof to FcRn

The antibody clones Mut1, Mut2, Mut3 and Mut4, of which methionine at position 252 in the EU numbering system in the heavy chain constant regions of the PD-1/CD3 bispecific monoclonal antibody clone PD1-5 (Bi) was substituted with glutamic acid, proline, arginine and aspartic acid, respectively, were prepared by methods according to well-known techniques regarding amino acid substitutions. Further, the antibody clone Mut5, of which asparagine at position 434 in the EU numbering system in the heavy chain constant regions of the clone PD1-5 (Bi) was substituted with leucine, and the antibody clone Mut6, of which glutamine at position 438 was substituted with glutamic acid, were prepared by the methods as the above, respectively.

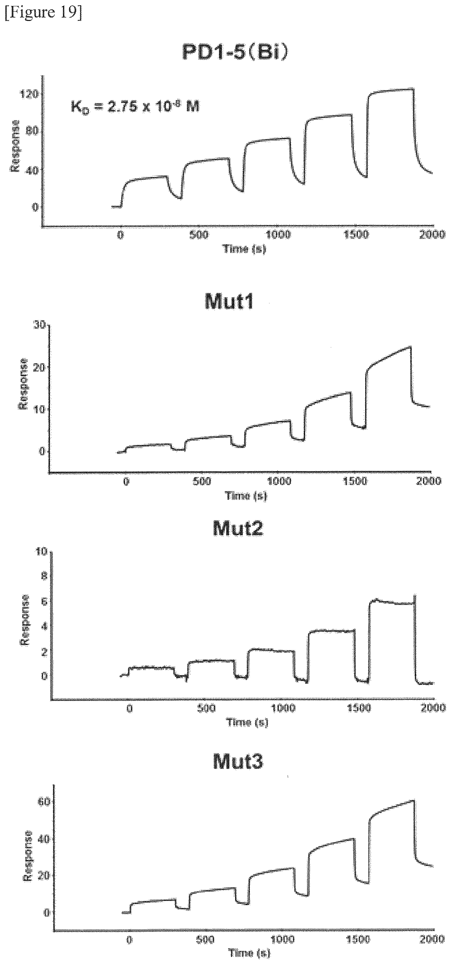

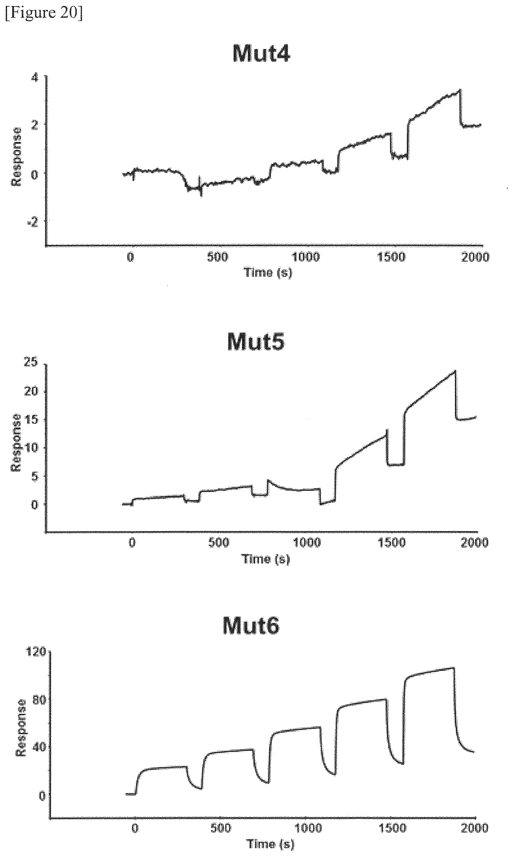

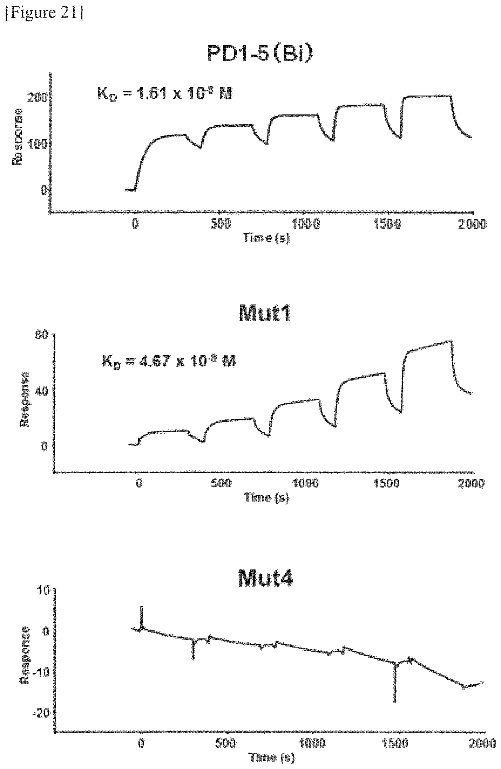

Then, the binding affinities to human FcRn or mouse FcRn of those antibody clones were evaluated using surface plasmon resonance, respectively. Series S Sensor Chip CAP of Biotin CAPture Kit (GE Healthcare, model no. 28-9202-34) was set in Biacore T200 (GE Healthcare), and human FcRn-human B2M (Immunitrack, model no. ITF01) and Murine FcRn-murine B2M (Immunitrack, model no. ITF07) were immobilized thereon, respectively. The binding properties of PD1-5 (Bi) and Mut1 to Mut6 were evaluated under conditions of pH 6.0, respectively. Figures 19 and 20 show results thereof about the binding properties with human FcRn, and Figure 21 shows those with mouse FcRn.

The respective affinities for human FcRn of the clones Mut1 to Mut6 were lower compared to that of the PD1-5 (Bi) (dissociation constant (KD value): 2.75 × 10⁻⁸ M), and in particular, those of the clones Mut4 and Mut2 significantly decreased. The respective affinities for mouse FcRn of the clones Mut1 and Mut4 were lower than that of PD1-5 (Bi) (KD value: 1.61 × 10⁻⁸ M) (KD value of Mut1: 4.67 × 10⁻⁸ M), in particular, that of the clone Mut4 significantly decreased.

### Example 18: Blood kinetics of the PD1/CD3 bispecific monoclonal antibody clone Mut4

*In vivo* blood kinetics of the clone Mut4 was evaluated using human CD3ε/human PD-1 knock-in C57BL/6 mice. The human CD3ε/human PD-1 knock-in C57BL/6 mice were produced by mating human CD3ε knock-in mice and human PD-1 knock-in mice produced according to the method described in Genesis. 2009 Jun; 47(6): 414-22, by a known method.

At 30 minutes and 1, 2, 4, 8, 24 and 288 hours after administration of the same clone into tail veins of the C57BL/6 mice, tail veins of mice were partially injured with scissors, and approximately 40µL of blood therefrom was collected into heparinized tubes. Blood was collected by centrifugation to prepare plasma, and the plasma concentration of the same clone was measured in the electrochemiluminescence (ECL) method. The half-life in blood was calculated from transition of plasma concentration.

As a result of measurement, the half-life in blood of the clone was 8.0 hours, which was shorter than the half-life in blood of the original PD1-5 (Bi), about 160 hours.

### Example 19: Suppressive effects of the PD-1/CD3 bispecific monoclonal antibody against cell proliferation of T cell acute lymphocytic leukemia cell line

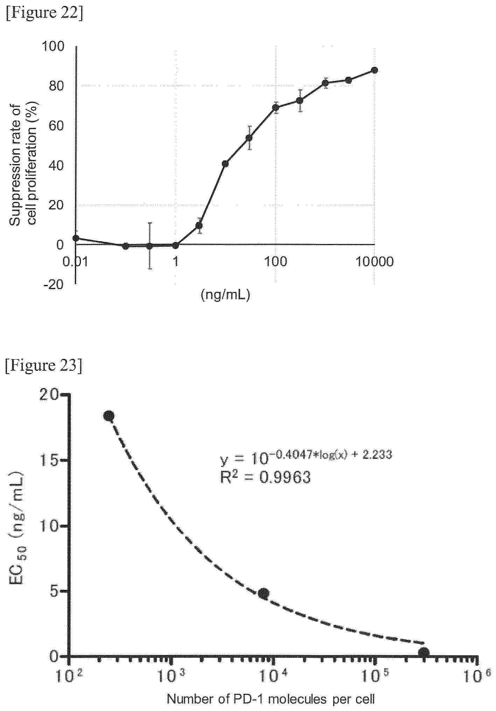

Using peripheral blood mononuclear cells (HemaCare, serial number PB009C-1) (human PBMC) derived from healthy person, the suppressive effect against cell proliferation of T cell acute lymphocytic leukemia cell lines was evaluated. On U-bottom 96-well plates, human PBMC and T cell acute lymphocytic leukemia cell line HPB-ALL labeled with Vybrant DiD Cell-Labeling Solution (Thermo Fisher, serial number V22887) were seeded, and the clone PD1-5(Bi) as the PD-1/CD3 bispecific monoclonal antibody was added thereto, and then co-cultured for 48 hours. The cells were collected from each well, stained with Cell Viability Solution, and the number of DiD-positive viable cells was counted by flow cytometer. The number of DiD-positive living cells in the antibody-free sample was set to 100%, and the reduction rate of DiD-positive living cells in the sample to which the PD1-5(Bi) was added was calculated as the suppressive rate against cell proliferation. Figure 22 shows results thereof. The cell proliferation of HPB-ALL was suppressed by addition of PD1-5(Bi).

### Example 20: Suppressive effects of the PD-1/CD3 bispecific monoclonal antibody against cell proliferation of peripheral T cell lymphoma cell line

Using peripheral blood mononuclear cells (Precision for Medicine, serial number 93000-10M) (human PBMC) derived from healthy persons, the suppressive effect against cell proliferation of T cell acute lymphocytic leukemia cell lines was evaluated. On U-bottom 96-well plates, human PBMC and cutaneous T-cell lymphoma (mycosis fungoides) cell line MJ labeled with Vybrant DiD Cell-Labeling Solution (Thermo Fisher, serial number V22887) were seeded, and the clone PD1-5(Bi) as the PD-1/CD3 bispecific monoclonal antibody was added thereto, and then co-cultured for 48 hours. The cells were collected from each well, stained with SYTOX Green Dead Cell Stain (Thermo Fisher Scientific, serial number S34860), and the number of DiD-positive viable cells was counted by flow cytometer. The number of DiD-positive living cells in the antibody-free sample was set to 100%, and the reduction rate of DiD-positive living cells in the sample to which the PD1-5(Bi) was added was calculated as the suppressive rate against cell proliferation. The cell proliferation of cutaneous T-cell lymphoma cell line MJ was suppressed by addition of PD1-5(Bi).

### Example 21: Evaluation of PD-1 expression level on a target cell and the cytotoxic activity of the PD-1/CD3 bispecific monoclonal antibody

The cytotoxic activities of the PD-1/CD3 bispecific monoclonal antibody clone PD1-5 (Bi) against human PD-1-forced expressing SU-DHL-4, EB-1 and RPMI8226 as target cells were measured according to the methods described in Example 13 and 14, respectively, and their EC50 (ng/mL) were calculated. On the other hand, the number of PD-1 molecules per cell expressed on the surface of these target cells was measured by flow cytometer. Figure 23 shows the relationship between the number of PD-1 molecules in the respective target cells and the EC50 of cytotoxic activity, represented in a scatter diagram. The broken line in the figure is calculated according to the non-linear regression analysis (logarithmic model: Y = 10^ (slope × logX + Yintercept), Y: EC50 (ng/mL), X: the number of PD-1 molecules, slope: slope of a straight line, Yintercept: Y intercept), and the mathematical formula in the figure corresponds to that of the log-log model. R² represents a coefficient of determination.

It was confirmed that the cytotoxic activity of human peripheral blood T cells in the presence of PD1-5 (Bi) on target cells was enhanced in response to the increase in PD-1 expression level on the target cells.

### [Industrial Applicability]

The PD-1/CD3 bispecific antibody or antibody fragment thereof of the present invention is useful for preventing, suppressing the progression of symptoms of or the recurrence of, and/or treating hematological cancer.

## Claims

1. An agent for preventing, suppressing the progression of symptoms of or the recurrence of and/or treating hematological cancer, comprising a bispecific protein having a first arm specifically binding to PD-1 and a second arm specifically binding to CD3 as an active ingredient.

2. The agent according to claim 1, wherein the bispecific protein is a bispecific antibody having the first arm specifically binding to PD-1 and the second arm specifically binding to CD3 or an antibody fragment thereof.

3. The agent according to claim 2, wherein the first arm specifically binding to PD-1, of the bispecific antibody or the antibody fragment thereof, has any one of VH selected from
(A) a VH having
(a) a VH-CDR1 comprising the amino acid sequence set forth in SEQ ID No. 6,
(b) a VH-CDR2 comprising the amino acid sequence set forth in SEQ ID No. 7, and
(c) a VH-CDR3 comprising the amino acid sequence set forth in SEQ ID No. 8,
(B) a VH having
(a) a VH-CDR1 comprising the amino acid sequence set forth in SEQ ID No. 9,
(b) a VH-CDR2 comprising the amino acid sequence set forth in SEQ ID No. 10, and
(c) a VH-CDR3 comprising the amino acid sequence set forth in SEQ ID No. 11,
(C) a VH having
(a) a VH-CDR1 comprising the amino acid sequence set forth in SEQ ID No. 12,
(b) a VH-CDR2 comprising the amino acid sequence set forth in SEQ ID No. 13, and
(c) a VH-CDR3 comprising the amino acid sequence set forth in SEQ ID No. 14,
(D) a VH having
(a) a VH-CDR1 comprising the amino acid sequence set forth in SEQ ID No. 15,
(b) a VH-CDR2 comprising the amino acid sequence set forth in SEQ ID No. 16, and
(c) a VH-CDR3 comprising the amino acid sequence set forth in SEQ ID No. 17, and
(E) a VH having
(a) a VH-CDR1 comprising the amino acid sequence set forth in SEQ ID No. 18,
(b) a VH-CDR2 comprising the amino acid sequence set forth in SEQ ID No. 19, and
(c) a VH-CDR3 comprising the amino acid sequence set forth in SEQ ID No. 20, and
(F) a VL having
(a) a VL-CDR1 comprising the amino acid sequence set forth in SEQ ID No. 26,
(b) a VL-CDR2 comprising the amino acid sequence set forth in SEQ ID No. 27, and
(c) a VL-CDR3 comprising the amino acid sequence set forth in SEQ ID No. 28, and the second arm specifically binding to CD3, of the bispecific antibody or the antibody fragment thereof, has
(A) a VH having
(a) a VH-CDR1 comprising the amino acid sequence set forth in SEQ ID No.37,
(b) a VH-CDR2 comprising the amino acid sequence set forth in SEQ ID No.38, and
(c) a VH-CDR3 comprising the amino acid sequence set forth in SEQ ID No.39, and
(B) a VL having
(a) a VL-CDR1 comprising the amino acid sequence set forth in SEQ ID No. 26,
(b) a VL-CDR2 comprising the amino acid sequence set forth in SEQ ID No. 27, and
(c) a VL-CDR3 comprising the amino acid sequence set forth in SEQ ID No. 28, and
wherein one to five arbitrary amino acid residues may be substituted with other amino acids in any one or more of VH-CDRs selected from the VH-CDR1, VH-CDR2 and VH-CDR3 in the first arm specifically binding to PD-1, respectively, and/or one to five arbitrary amino acid residues may be substituted with other amino acids in any one or more of VH-CDRs selected from the VH-CDR1, VH-CDR2 and VH-CDR3 in the second arm specifically binding to CD3, respectively.

4. The agent according to claim 2, wherein the first arm specifically binding to PD-1, of the bispecific antibody or the antibody fragment thereof, has any one of the VH selected from
(A) the VH having
(a) the VH-CDR1 comprising the amino acid sequence set forth in SEQ ID No. 6,
(b) the VH-CDR2 comprising the amino acid sequence set forth in SEQ ID No. 7, and
(c) the VH-CDR3 comprising the amino acid sequence set forth in SEQ ID No. 8,
(B) the VH having
(a) the VH-CDR1 comprising the amino acid sequence set forth in SEQ ID No. 9,
(b) the VH-CDR2 comprising the amino acid sequence set forth in SEQ ID No. 10, and
(c) the VH-CDR3 comprising the amino acid sequence set forth in SEQ ID No. 11,
(C) the VH having
(a) the VH-CDR1 comprising the amino acid sequence set forth in SEQ ID No. 12,
(b) the VH-CDR2 comprising the amino acid sequence set forth in SEQ ID No. 13, and
(c) the VH-CDR3 comprising the amino acid sequence set forth in SEQ ID No. 14,
(D) the VH having
(a) the VH-CDR1 comprising the amino acid sequence set forth in SEQ ID No. 15,
(b) the VH-CDR2 comprising the amino acid sequence set forth in SEQ ID No. 16, and
(c) the VH-CDR3 comprising the amino acid sequence set forth in SEQ ID No. 17, and
(E) the VH having
(a) the VH-CDR1 comprising the amino acid sequence set forth in SEQ ID No. 18,
(b) the VH-CDR2 comprising the amino acid sequence set forth in SEQ ID No. 19, and
(c) the VH-CDR3 comprising the amino acid sequence set forth in SEQ ID No. 20, and
(F) the VL having
(a) the VL-CDR1 comprising the amino acid sequence set forth in SEQ ID No. 26,
(b) the VL-CDR2 comprising the amino acid sequence set forth in SEQ ID No. 27, and
(c) the VL-CDR3 comprising the amino acid sequence set forth in SEQ ID No. 28, and
the second arm specifically binding to CD3, of the bispecific antibody or the antibody fragment thereof, has
(A) the VH having
(a) the VH-CDR1 comprising the amino acid sequence set forth in SEQ ID No.37,
(b) the VH-CDR2 comprising the amino acid sequence set forth in SEQ ID No.38, and
(c) the VH-CDR3 comprising the amino acid sequence set forth in SEQ ID No.39, and
(B) the VL having
(a) the VL-CDR1 comprising the amino acid sequence set forth in SEQ ID No. 26,
(b) the VL-CDR2 comprising the amino acid sequence set forth in SEQ ID No. 27, and
(c) the VL-CDR3 comprising the amino acid sequence set forth in SEQ ID No. 28.

5. The agent according to any of claims 2 to 4, wherein (i) the VH of the first arm specifically binding to PD-1 has
(a) the VH-CDR1 comprising the amino acid sequence set forth in SEQ ID No. 6,
(b) the VH-CDR2 comprising the amino acid sequence set forth in SEQ ID No. 7, and
(c) the VH-CDR3 comprising the amino acid sequence set forth in SEQ ID No. 8, and
(ii) the VH of the second arm specifically binding to CD3 has
(a) the VH-CDR1 comprising the amino acid sequence set forth in SEQ ID No. 37,
(b) the VH-CDR2 comprising the amino acid sequence set forth in SEQ ID No. 38, and
(c) the VH-CDR3 comprising the amino acid sequence set forth in SEQ ID No. 39.

6. The agent according to any of claims 2 to 4, wherein (i) the VH of the first arm specifically binding to PD-1 has
(a) the VH-CDR1 comprising the amino acid sequence set forth in SEQ ID No. 9,
(b) the VH-CDR2 comprising the amino acid sequence set forth in SEQ ID No. 10, and
(c) the VH-CDR3 comprising the amino acid sequence set forth in SEQ ID No. 11, and
(ii) the VH of the second arm specifically binding to CD3 has
(a) the VH-CDR1 comprising the amino acid sequence set forth in SEQ ID No. 37,
(b) the VH-CDR2 comprising the amino acid sequence set forth in SEQ ID No. 38, and
(c) the VH-CDR3 comprising the amino acid sequence set forth in SEQ ID No. 39.

7. The agent according to any of claims 2 to 4, wherein (i) the VH of the first arm specifically binding to PD-1 has
(a) the VH-CDR1 comprising the amino acid sequence set forth in SEQ ID No. 12,
(b) the VH-CDR2 comprising the amino acid sequence set forth in SEQ ID No. 13, and
(c) the VH-CDR3 comprising the amino acid sequence set forth in SEQ ID No. 14, and
(ii) the VH of the second arm specifically binding to CD3 has
(a) the VH-CDR1 comprising the amino acid sequence set forth in SEQ ID No. 37,
(b) the VH-CDR2 comprising the amino acid sequence set forth in SEQ ID No. 38, and
(c) the VH-CDR3 comprising the amino acid sequence set forth in SEQ ID No. 39.

8. The agent according to any of claims 2 to 4, wherein (i) the VH of the first arm specifically binding to PD-1 has
(a) the VH-CDR1 comprising the amino acid sequence set forth in SEQ ID No. 15,
(b) the VH-CDR2 comprising the amino acid sequence set forth in SEQ ID No. 16, and
(c) the VH-CDR3 comprising the amino acid sequence set forth in SEQ ID No. 17, and
(ii) the VH of the second arm specifically binding to CD3 has
(a) the VH-CDR1 comprising the amino acid sequence set forth in SEQ ID No. 37,
(b) the VH-CDR2 comprising the amino acid sequence set forth in SEQ ID No. 38, and
(c) the VH-CDR3 comprising the amino acid sequence set forth in SEQ ID No. 39.

9. The agent according to any of claims 2 to 4, wherein (i) the VH of the first arm specifically binding to PD-1 has
(a) the VH-CDR1 comprising the amino acid sequence set forth in SEQ ID No. 18,
(b) the VH-CDR2 comprising the amino acid sequence set forth in SEQ ID No. 19, and
(c) the VH-CDR3 comprising the amino acid sequence set forth in SEQ ID No. 20, and
(ii) the VH of the second arm specifically binding to CD3 has
(a) the VH-CDR1 comprising the amino acid sequence set forth in SEQ ID No. 37,
(b) the VH-CDR2 comprising the amino acid sequence set forth in SEQ ID No. 38, and
(c) the VH-CDR3 comprising the amino acid sequence set forth in SEQ ID No. 39.

10. The agent according to any one of claims 2 to 9, wherein the FR1, FR2 and FR3 regions in the VH of the first arm specifically binding to PD-1 correspond to amino acid sequences encoded by the germ-line V gene IGHV7-4-1 which may have somatic mutation(s), respectively, and the FR4 region comprises an amino acid sequence encoded by the germ-line J gene JH6c which may have somatic mutation(s) (excluding the amino acid sequence included in the VH-CDR3 region).

11. The agent according to any one of claims 2 to 10, wherein the VH of the first arm specifically binding to PD-1 comprises the amino acid sequence set forth in any one selected from SEQ ID No. 1, SEQ ID No. 2, SEQ ID No. 3, SEQ ID No. 4 and SEQ ID No. 5, or an amino acid sequence having an identity of at least 80% to the same VH amino acid sequence.

12. The agent according to any one of claims 2 to 11, wherein the VH of the second arm specifically binding to CD3 comprises the amino acid sequence set forth in SEQ ID No. 36, or an amino acid sequence having an identity of at least 80% to the same VH amino acid sequence.

13. The agent according to claim 2 or 4, wherein the VH of the first arm specifically binding to PD-1 comprises the amino acid sequence set forth in any one selected from SEQ ID No. 1, SEQ ID No. 2, SEQ ID No. 3, SEQ ID No. 4 and SEQ ID No. 5, and the VH of the second arm specifically binding to CD3 comprises the amino acid sequence set forth in SEQ ID No. 36.

14. The agent according to claim 2, 4 or 5, wherein the VH of the first arm specifically binding to PD-1 comprises the amino acid sequence set forth in SEQ ID No. 1, and the VH of the second arm specifically binding to CD3 comprises the amino acid sequence set forth in SEQ ID No. 36.

15. The agent according to claim 2, 4 or 6, wherein the VH of the first arm specifically binding to PD-1 comprises the amino acid sequence set forth in SEQ ID No. 2, and the VH of the second arm specifically binding to CD3 comprises the amino acid sequence set forth in SEQ ID No. 36.

16. The agent according to claim 2, 4 or 7, wherein the VH of the first arm specifically binding to PD-1 comprises the amino acid sequence set forth in SEQ ID No. 3, and the VH of the second arm specifically binding to CD3 comprises the amino acid sequence set forth in SEQ ID No. 36.

17. The agent according to claim 2, 4 or 8, wherein the VH of the first arm specifically binding to PD-1 comprises the amino acid sequence set forth in SEQ ID No. 4, and the VH of the second arm specifically binding to CD3 comprises the amino acid sequence set forth in SEQ ID No. 36.

18. The agent according to claim 2, 4 or 9, wherein the VH of the first arm specifically binding to PD-1 comprises the amino acid sequence set forth in SEQ ID No. 5, and the VH of the second arm specifically binding to CD3 comprises the amino acid sequence set forth in SEQ ID No. 36.

19. The agent according to any one of claims 2 to 18, wherein the first arm specifically binding to PD-1 and/or the second arm specifically binding to CD3 have/has the VL comprising the amino acid sequence set forth in SEQ ID No. 25, respectively.

20. An agent for preventing, suppressing the progression of symptoms of or the recurrence of and/or treating hematological cancer, comprising a bispecific antibody or an antibody fragment thereof, having a first arm specifically binding to PD-1 and a second arm specifically binding to CD3 as an active ingredient, wherein
(A) the first arm specifically binding to PD-1 has a VH comprising the amino acid sequence set forth in any one selected from SEQ ID No. 1, SEQ ID No. 2, SEQ ID No. 3, SEQ ID No. 4 and SEQ ID No. 5, and a VL comprising the amino acid sequence set forth in SEQ ID No. 25, and
(B) the second arm specifically binding to CD3 has a VH comprising the amino acid sequence set forth in SEQ ID No. 36, and a VL comprising the amino acid sequence set forth in SEQ ID No. 25.

21. An agent for preventing, suppressing the progression of symptoms of or the recurrence of and/or treating hematological cancer, comprising a bispecific antibody or an antibody fragment thereof, having a first arm specifically binding to PD-1 and a second arm specifically binding to CD3 as an active ingredient, wherein the first arm specifically binding to PD-1 cross-competes for (1) the binding to PD-1 with the first arm specifically binding to PD-1 having a VH comprising the amino acid sequence set forth in any one selected from SEQ ID No. 1, SEQ ID No. 2, SEQ ID No. 3, SEQ ID No. 4 and SEQ ID No. 5, and a VL comprising the amino acid sequence set forth in SEQ ID No. 25, or (2) the binding to PD-1 with a variable region of a monoclonal antibody specifically binding to PD-1 having the same VH and VL.

22. An agent for preventing, suppressing the progression of symptoms of or the recurrence of and/or treating hematological cancer, comprising a bispecific antibody or an antibody fragment thereof, having a first arm specifically binding to PD-1 and a second arm specifically binding to CD3 as an active ingredient, wherein the binding to PD-1 with the first arm specifically binding to PD-1 is cross-competed by (1) the first arm specifically binding to PD-1 having a VH comprising the amino acid sequence set forth in any one selected from SEQ ID No. 1, SEQ ID No. 2, SEQ ID No. 3, SEQ ID No. 4 and SEQ ID No. 5, and a VL comprising the amino acid sequence set forth in SEQ ID No. 25, or (2) a variable region of a monoclonal antibody specifically binding to PD-1 having the same VH and VL.

23. The agent according to claim 20 or 21, wherein the second arm specifically binding to CD3 cross-competes for (1) the binding to CD3 with the second arm specifically binding to CD3 having the VH comprising the amino acid sequence set forth in SEQ ID No. 36, and the VL comprising the amino acid sequence set forth in SEQ ID No. 25, or (2) the binding to CD3 with a variable region of a monoclonal antibody specifically binding to CD3 having the same VH and VL.

24. The agent according to any one of claims 2 to 23, wherein the bispecific antibody is an IgG antibody.

25. The agent according to claim 24, wherein the IgG antibody is an IgG₁ antibody or IgG₄ antibody.

26. The agent according to claim 24, wherein the IgG antibody is an IgG₁ antibody.

27. The agent according to claim 26, wherein the binding to Fc receptor of the IgG₁ antibody is eliminated or attenuated.

28. The agent according to claim 27, wherein in two heavy chain constant regions of the IgG₁ antibody, each leucine at position 235 according to the EU numbering system is substituted with glycine, and/or each glycine at position 236 is substituted with arginine.

29. The agent according to any one of claims 26 to 28, wherein in a constant region of a heavy chain having a VH of the first arm specifically binding to PD-1, leucine at position 351 according to the EU numbering system is substituted with lysine and threonine at position 366 is substituted with lysine, and in a constant region of a heavy chain having a VH of the second arm specifically binding to CD3, leucine at position 351 is substituted with aspartic acid and leucine at position 368 is substituted with glutamic acid.

30. The agent according to any one of claims 26 to 28, wherein in a constant region of a heavy chain having a VH of the first arm specifically binding to PD-1, leucine at position 351 according to the EU numbering system is substituted with aspartic acid, and leucine at position 368 is substituted with glutamic acid, and in a constant region of a heavy chain having a VH of the second arm specifically binding to CD3, leucine at position 351 is substituted with lysine, and threonine at position 366 is substituted with lysine.

31. The agent according to any one of claims 26 to 30, wherein in two heavy chain constant regions of the IgG₁ antibody, each lysine at position 447 according to the EU numbering system is deleted.

32. The agent according to any one of claims 24 to 31, wherein the binding of the IgG antibody to FcRn receptor is eliminated or attenuated.

33. The agent according to any one of claims 26 to 31, wherein in two heavy chain constant regions of the IgG₁ antibody, (1) each methionine at position 252 according to the EU numbering system is substituted with glutamic acid, proline, arginine or aspartic acid, (2) each asparagine at position 434 according to the EU numbering system is substituted with leucine and/or (3) each glutamine at position 438 according to the EU numbering system is substituted with glutamic acid.

34. The agent according to any one of claims 26 to 33, wherein each methionine at position 252 according to the EU numbering system in two heavy chain constant regions of the IgG₁ antibody is substituted with aspartic acid.

35. The agent according to claim 25, wherein the IgG antibody is an IgG₄ antibody, and in two heavy chain constant regions thereof, each serine at position 228 according to the EU numbering system is substituted with proline.

36. The agent according to any one of claims 2 to 34, wherein the heavy chain having the VH of the first arm specifically binding to PD-1 has a heavy chain constant region comprising the amino acid sequence set forth in any one selected from SEQ ID No. 23, SEQ ID No. 42, SEQ ID No. 43, SEQ ID No. 44, SEQ ID No. 45, SEQ ID No. 46 and SEQ ID No. 47.

37. The agent according to any one of claims 2 to 34 and 36, wherein the heavy chain having the VH of the second arm specifically binding to CD3 has a heavy chain constant region comprising the amino acid sequence set forth in any one selected from SEQ ID No. 24, SEQ ID No. 48, SEQ ID No. 49, SEQ ID No. 50, SEQ ID No. 51, SEQ ID No. 52 and SEQ ID No. 53.

38. The agent according to any one of claims 2 to 37, wherein the light chain having the VL of the first arm specifically binding to PD-1 and/or the light chain having the VL of the second arm specifically binding to CD3 have/has a light chain constant region comprising the amino acid sequence set forth in SEQ ID No. 29.

39. An agent for preventing, suppressing the progression of symptoms of or the recurrence of and/or treating hematological cancer, comprising a bispecific antibody or an antibody fragment thereof, having a first arm specifically binding to PD-1 and a second arm specifically binding to CD3 as an active ingredient, wherein
(A) a heavy chain having a VH of the first arm specifically binding to PD-1 has a VH comprising the amino acid sequence set forth in any one selected from SEQ ID No. 1, SEQ ID No. 2, SEQ ID No. 3, SEQ ID No. 4 and SEQ ID No. 5, and a heavy chain constant region comprising the amino acid sequence set forth in any one selected from SEQ ID No. 23, SEQ ID No. 42, SEQ ID No. 43, SEQ ID No. 44, SEQ ID No. 45, SEQ ID No. 46 and SEQ ID No. 47,
(B) a light chain having a VL of the first arm specifically binding to PD-1 has a VL comprising the amino acid sequence set forth in SEQ ID No. 25, and the light chain constant region comprising the amino acid sequence set forth in SEQ ID No. 29,
(C) a heavy chain having a VH of the second arm specifically binding to CD3 has a VH comprising the amino acid sequence set forth in SEQ ID No. 36, and a heavy chain constant region comprising the amino acid sequence set forth in any one selected from SEQ ID No. 24, SEQ ID No. 48, SEQ ID No. 49, SEQ ID No. 50, SEQ ID No. 51, SEQ ID No. 52 and SEQ ID No. 53, and
(D) a light chain having a VL of the second arm specifically binding to CD3 has a VL comprising the amino acid sequence set forth in SEQ ID No. 25, and a light chain constant region comprising the amino acid sequence set forth in SEQ ID No. 29.

40. The agent according to any one of claims 1 to 39, wherein hematological cancer(s) is/are one or more selected from multiple myeloma, malignant lymphoma, leukemia, primary central nervous system lymphoma and myeloproliferative syndrome.

41. The agent according to claim 40, wherein hematological cancer is malignant lymphoma, further which is Non-Hodgkin's Lymphoma or Hodgkin's Lymphoma.

42. The agent according to claim 41, wherein malignant lymphoma is Non-Hodgkin's lymphoma, further which is B-cell non-Hodgkin's lymphoma or T/NK-cell non-Hodgkin's lymphoma.

43. The agent according to claim 42, wherein Non-Hodgkin's lymphoma is B-cell non-Hodgkin's lymphoma, further which is precursor B-cell lymphoblastic lymphoma, precursor B-cell acute lymphoblastic leukemia, chronic B-lymphoid leukemia, B-cell prolymphocytic leukemia, lymphoplasmacytic lymphoma, nodal marginal zone B-cell lymphoma, extranodal marginal zone B-cell lymphoma, primary splenic marginal zone B-cell lymphoma, hairy cell leukemia, hairly cell leukemia-variant, follicular lymphoma, pediatric type follicular lymphoma, diffuse large B cell lymphoma, diffuse large B-cell lymphoma, not otherwise specified, splenic diffuse red pulp small B-cell lymphoma, lymphoplasmacytic lymphoma, primary mediastinal large B-cell lymphoma, primary effusion lymphoma, Burkitt's lymphoma, mantle cell lymphoma, monoclonal B-cell lymphocytosis, splenic B-cell lymphoma/leukemia, unclassifiable, IgM monoclonal gammopathy of undetermined significance, µ heavy chain disease, λ heavy chain disease, α heavy chain disease, plasma cell myeloma, solitary plasmacytoma of bone, extraosseous plasmacytoma, monoclonal immunoglobulin deposition disease, large B-cell lymphoma with IRF4 rearrangement, primary cutaneous follicle center lymphoma, T-cell/histiocyte-rich large B-cell lymphoma, primary diffuse large B-cell lymphoma of the central nervous system, primary cutaneous diffuse large B-cell lymphoma, leg type, EBV positive diffuse large B-cell lymphoma, not otherwise specified, EBV positive mucocutaneous ulcer, diffuse large B-cell lymphoma associated with chronic inflammation, lymphomatoid granulomatosis, intravascular large B-cell lymphoma, ALK positive large B-cell lymphoma, plasmablastic lymphoma, primary effusion lymphoma, HHV8 positive diffuse large B-cell lymphoma, not otherwise specified, Burkitt-like lymphoma with 11q aberration, high-grade B-cell lymphoma, with MYC and BCL2 and/or BCL6 rearrangement, high-grade B-cell lymphoma, not otherwise specified or B-cell lymphoma, unclassifiable, with intermediate features between diffuse large B-cell lymphoma and classical Hodgkin lymphoma.

44. The agent according to claim 42, wherein Non-Hodgkin's lymphoma is T/NK-cell non-Hodgkin's lymphoma, further which is precursor T-cell lymphoblastic lymphoma, chronic T-cell lymphocytic leukemia, T-cell acute lymphocytic leukemia (T-cell lymphoblastic leukemia), T-cell large granular lymphoblastic lymphoma, NK-cell large granular leukemia, aggressive NK-cell leukemia, peripheral T-cell lymphoma, peripheral T-cell lymphoma, not otherwise specified, unclassifiable peripheral T-cell lymphoma, angioimmunoblastic T-cell lymphoma, (CD30-positive) anaplastic large cell lymphoma, angiocentric lymphoma, intestinal T-cell lymphoma, enteropathy-type T-cell lymphoma, hepatosplenic gamma-delta T cell lymphoma, subcutaneous panniculitis-like T-cell lymphoma, mycosis fungoides, Sezary syndrome, Hodgkin-like/Hodgkin-related anaplastic large cell lymphoma, extranodal NK/T-cell lymphoma, adult T cell lymphoma, T-cell prolymphocytic leukemia, chronic lymphoproliferative disorder of NK-cells, systemic EBV positive T-cell lymphoma of childhood, hydroa vacciniforme-like lymphoproliferative disorder, extranodal NK/T-cell lymphoma, nasal type, enteropathy-associated T-cell lymphoma, monomorphic epitheliotropic intestinal T-cell lymphoma, indolent T-cell lymphoproliferative disorder of the GI tract, hepatosplenic T-cell lymphoma, primary cutaneous CD30 positive T-cell lymphoproliferative disorder, lymphomatoid papulosis, primary cutaneous anaplastic large cell lymphoma, primary cutaneous gamma-delta T-cell lymphoma, primary cutaneous CD8 positive aggressive epidermotropic cytotoxic T-cell lymphoma, primary cutaneous acral CD8 positive T-cell lymphoma, primary cutaneous CD4 positive small/medium T-cell lymphoproliferative disorder, follicular T-cell lymphoma, Nodal peripheral T-cell lymphoma with TFH phenotype, anaplastic large cell lymphoma, ALK positive, anaplastic large cell lymphoma, ALK negative or breast implant-associated anaplastic large-cell lymphoma.

45. The agent according to claim 41, wherein malignant lymphoma is Hodgkin's lymphoma, further which is classical Hodgkin's lymphoma or nodular lymphocyte predominant Hodgkin's lymphoma.

46. The agent according to claim 40, wherein hematological cancer is leukemia, further which is acute myelogenous leukemia, acute promyelocytic leukemia, acute lymphoblastic leukemia, chronic lymphocytic leukemia, myelodysplastic syndrome or chronic myelogenous leukemia.

47. The agent according to claim 42, wherein Non-Hodgkin's lymphoma is T/NK-cell non-Hodgkin's lymphoma, further which is peripheral T-cell lymphoma or adult T cell lymphoma.

48. The agent according to any one of claims 1 to 47, which is further used in combination with other anti-cancer drugs.

49. The agent according to claim 48, wherein other anti-cancer drug(s) is/are one or more drugs selected from an alkylating agent, platinum agent, antimetabolite, ribonucleotide reductase inhibitor, nucleotide analog, topoisomerase inhibitor, microtubule polymerization inhibitor, microtubule depolymerization inhibitor, antitumor antibiotic, cytokine preparation, molecular targeting drug and cancer immunotherapeutic drug.


SEQUENCE LISTING

<110>  ONO PHARMACEUTICAL CO., LTD.

<120>  Treatment of hematological cancer with PD-1/CD3 bispecific protein

<130>  P20-109WO

<150>  JP 2019-126503
<151>  2019-07-05

<150>  JP 2019-139752
<151>  2019-07-30

<160>  53

<170>  PatentIn version 3.5

<210>  1
<211>  127
<212>  PRT
<213>  Homo sapiens

<400>  1

Gln Val Gln Leu Val Gln Ser Gly Ser Glu Leu Lys Gln Pro Gly Ala
1               5                   10                  15

Ser Val Lys Val Ser Cys Lys Ala Ser Gly Tyr Thr Phe Thr His Tyr
            20                  25                  30

Gly Leu His Trp Val Arg Gln Ala Pro Gly Gln Gly Leu Glu Trp Met
        35                  40                  45

Gly Trp Leu Asn Thr Asn Thr Glu Asn Pro Thr Phe Ala Gln Gly Phe
    50                  55                  60

Thr Gly Arg Phe Val Phe Ser Leu Asp Thr Ser Val Thr Thr Ala Tyr
65                  70                  75                  80

Leu Gln Ile Ser Ser Leu Lys Ala Glu Asp Thr Ala Val Tyr Tyr Cys
                85                  90                  95

Ala Arg Gly Asp Met Val Val Pro Thr Thr Ile Trp Asn Tyr Tyr His
            100                 105                 110

Phe Met Asp Val Trp Gly Asn Gly Thr Leu Val Thr Val Ser Ser
        115                 120                 125

<210>  2
<211>  127
<212>  PRT
<213>  Homo sapiens

<400>  2

Gln Val Gln Leu Val Gln Ser Gly Ser Glu Leu Lys Gln Pro Gly Val
1               5                   10                  15

Ser Val Lys Val Ser Cys Lys Ala Ser Gly Tyr Thr Phe Thr His Tyr
            20                  25                  30

Gly Leu His Trp Val Arg Gln Ala Pro Gly Gln Gly Leu Glu Trp Met
        35                  40                  45

Gly Trp Ile Asn Thr Asn Thr Gly Asn Pro Thr Tyr Ala Gln Gly Phe
    50                  55                  60

Thr Gly Arg Phe Val Phe Ser Leu Asp Thr Ser Val Ser Thr Ala Tyr
65                  70                  75                  80

Leu Gln Ile Ser Ser Leu Lys Ala Glu Asp Thr Ala Val Tyr Tyr Cys
                85                  90                  95

Ala Arg Gly Asp Leu Val Val Pro Thr Thr Ile Trp Asn Tyr Tyr His
            100                 105                 110

Tyr Met Glu Val Trp Gly Lys Gly Thr Leu Val Thr Val Ser Ser
        115                 120                 125

<210>  3
<211>  127
<212>  PRT
<213>  Homo sapiens

<400>  3

Gln Val Gln Leu Val Gln Ser Gly Ser Glu Leu Lys Lys Pro Gly Ala
1               5                   10                  15

Ser Val Met Val Ser Cys Lys Ala Ser Gly Tyr Thr Phe Thr His Tyr
            20                  25                  30

Ala Leu His Trp Val Arg Gln Ala Pro Gly Gln Gly Leu Glu Trp Met
        35                  40                  45

Gly Trp Leu Asn Thr Asn Thr Glu Asn Pro Thr Tyr Ala Gln Gly Phe
    50                  55                  60

Thr Gly Arg Phe Val Phe Ser Leu Asp Thr Ser Val Thr Thr Ala Tyr
65                  70                  75                  80

Leu Gln Ile Asn Ser Leu Lys Ala Glu Asp Thr Ala Val Tyr Tyr Cys
                85                  90                  95

Ala Arg Gly Asp Met Val Val Pro Thr Thr Ile Trp Asn Tyr Tyr Tyr
            100                 105                 110

Tyr Met Asp Val Trp Gly Lys Gly Thr Thr Val Thr Val Ser Ser
        115                 120                 125

<210>  4
<211>  127
<212>  PRT
<213>  Homo sapiens

<400>  4

Gln Val Gln Leu Val Gln Ser Gly Ser Glu Leu Lys Gln Pro Gly Ala
1               5                   10                  15

Ser Val Lys Val Ser Cys Lys Ala Ser Gly Tyr Thr Phe Thr His Tyr
            20                  25                  30

Ala Leu His Trp Leu Arg Gln Ala Pro Gly Gln Gly Leu Glu Trp Met
        35                  40                  45

Gly Trp Leu Asn Thr Asn Thr Glu Asn Pro Thr Phe Ala Gln Gly Phe
    50                  55                  60

Thr Gly Arg Phe Val Phe Ser Leu Asp Thr Ser Val Thr Thr Ala Tyr
65                  70                  75                  80

Leu Gln Ile Ser Ser Leu Lys Ala Glu Asp Thr Ala Val Tyr Tyr Cys
                85                  90                  95

Ala Arg Gly Asp Met Val Val Pro Thr Thr Ile Trp Asn Tyr Tyr His
            100                 105                 110

Phe Met Asp Val Trp Gly Asn Gly Thr Thr Val Thr Val Ser Ser
        115                 120                 125

<210>  5
<211>  127
<212>  PRT
<213>  Homo sapiens

<400>  5

Gln Val Gln Leu Val Gln Ser Gly Ser Glu Leu Lys Gln Pro Gly Ala
1               5                   10                  15

Ser Val Lys Val Ser Cys Lys Ala Ser Gly Tyr Thr Phe Thr His Tyr
            20                  25                  30

Ala Leu His Trp Leu Arg Gln Ala Pro Gly Gln Gly Leu Glu Trp Met
        35                  40                  45

Gly Trp Leu Asn Thr Asn Thr Glu Asn Pro Thr Phe Ala Gln Gly Phe
    50                  55                  60

Thr Gly Arg Phe Val Phe Ser Leu Asp Thr Ser Val Thr Thr Ala Tyr
65                  70                  75                  80

Leu Gln Ile Ser Ser Leu Lys Ala Glu Asp Thr Ala Val Tyr Tyr Cys
                85                  90                  95

Ala Arg Gly Asp Met Val Val Pro Thr Thr Ile Trp Asn Tyr Tyr His
            100                 105                 110

Phe Met Asp Val Trp Gly Gln Gly Thr Thr Val Thr Val Ser Ser
        115                 120                 125

<210>  6
<211>  5
<212>  PRT
<213>  Homo sapiens

<400>  6

His Tyr Gly Leu His
1               5

<210>  7
<211>  17
<212>  PRT
<213>  Homo sapiens

<400>  7

Trp Leu Asn Thr Asn Thr Glu Asn Pro Thr Phe Ala Gln Gly Phe Thr
1               5                   10                  15

Gly

<210>  8
<211>  18
<212>  PRT
<213>  Homo sapiens

<400>  8

Gly Asp Met Val Val Pro Thr Thr Ile Trp Asn Tyr Tyr His Phe Met
1               5                   10                  15

Asp Val

<210>  9
<211>  5
<212>  PRT
<213>  Homo sapiens

<400>  9

His Tyr Gly Leu His
1               5

<210>  10
<211>  17
<212>  PRT
<213>  Homo sapiens

<400>  10

Trp Ile Asn Thr Asn Thr Gly Asn Pro Thr Tyr Ala Gln Gly Phe Thr
1               5                   10                  15

Gly

<210>  11
<211>  18
<212>  PRT
<213>  Homo sapiens

<400>  11

Gly Asp Leu Val Val Pro Thr Thr Ile Trp Asn Tyr Tyr His Tyr Met
1               5                   10                  15

Glu Val

<210>  12
<211>  5
<212>  PRT
<213>  Homo sapiens

<400>  12

His Tyr Ala Leu His
1               5

<210>  13
<211>  17
<212>  PRT
<213>  Homo sapiens

<400>  13

Trp Leu Asn Thr Asn Thr Glu Asn Pro Thr Tyr Ala Gln Gly Phe Thr
1               5                   10                  15

Gly

<210>  14
<211>  18
<212>  PRT
<213>  Homo sapiens

<400>  14

Gly Asp Met Val Val Pro Thr Thr Ile Trp Asn Tyr Tyr Tyr Tyr Met
1               5                   10                  15

Asp Val

<210>  15
<211>  5
<212>  PRT
<213>  Homo sapiens

<400>  15

His Tyr Ala Leu His
1               5

<210>  16
<211>  17
<212>  PRT
<213>  Homo sapiens

<400>  16

Trp Leu Asn Thr Asn Thr Glu Asn Pro Thr Phe Ala Gln Gly Phe Thr
1               5                   10                  15

Gly

<210>  17
<211>  18
<212>  PRT
<213>  Homo sapiens

<400>  17

Gly Asp Met Val Val Pro Thr Thr Ile Trp Asn Tyr Tyr His Phe Met
1               5                   10                  15

Asp Val

<210>  18
<211>  5
<212>  PRT
<213>  Homo sapiens

<400>  18

His Tyr Ala Leu His
1               5

<210>  19
<211>  17
<212>  PRT
<213>  Homo sapiens

<400>  19

Trp Leu Asn Thr Asn Thr Glu Asn Pro Thr Phe Ala Gln Gly Phe Thr
1               5                   10                  15

Gly

<210>  20
<211>  18
<212>  PRT
<213>  Homo sapiens

<400>  20

Gly Asp Met Val Val Pro Thr Thr Ile Trp Asn Tyr Tyr His Phe Met
1               5                   10                  15

Asp Val

<210>  21
<211>  98
<212>  PRT
<213>  Homo sapiens

<400>  21

Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr Ser Tyr
            20                  25                  30

Ala Met Asn Trp Val Arg Gln Ala Pro Gly Gln Gly Leu Glu Trp Met
        35                  40                  45

Gly Trp Ile Asn Thr Asn Thr Gly Asn Pro Thr Tyr Ala Gln Gly Phe
    50                  55                  60

Thr Gly Arg Phe Val Phe Ser Leu Asp Thr Ser Val Ser Thr Ala Tyr
65                  70                  75                  80

Leu Gln Ile Cys Ser Leu Lys Ala Glu Asp Thr Ala Val Tyr Tyr Cys
                85                  90                  95

Ala Arg

<210>  22
<211>  98
<212>  PRT
<213>  Homo sapiens

<400>  22

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Trp Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg

<210>  23
<211>  329
<212>  PRT
<213>  Homo sapiens

<400>  23

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Gly Arg Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Asp Pro Pro Ser Arg Glu Glu
225                 230                 235                 240

Met Thr Lys Asn Gln Val Ser Leu Thr Cys Glu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly
                325

<210>  24
<211>  329
<212>  PRT
<213>  Homo sapiens

<400>  24

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Gly Arg Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Lys Pro Pro Ser Arg Glu Glu
225                 230                 235                 240

Met Thr Lys Asn Gln Val Ser Leu Lys Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly
                325

<210>  25
<211>  107
<212>  PRT
<213>  Homo sapiens

<400>  25

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Pro
                85                  90                  95

Thr Phe Gly Gln Gly Thr Lys Val Glu Ile Lys
            100                 105

<210>  26
<211>  11
<212>  PRT
<213>  Homo sapiens

<400>  26

Arg Ala Ser Gln Ser Ile Ser Ser Tyr Leu Asn
1               5                   10


<210>  27
<211>  7
<212>  PRT
<213>  Homo sapiens

<400>  27

Ala Ala Ser Ser Leu Gln Ser
1               5


<210>  28
<211>  9
<212>  PRT
<213>  Homo sapiens

<400>  28

Gln Gln Ser Tyr Ser Thr Pro Pro Thr
1               5


<210>  29
<211>  107
<212>  PRT
<213>  Homo sapiens

<400>  29

<210>  30
<211>  381
<212>  DNA
<213>  Homo sapiens

<220>
<221>  CDS
<222>  (1)..(381)

<400>  30
cag gtg cag ctg gtg caa tct ggg tct gag ttg aag cag cct ggg gcc      48
Gln Val Gln Leu Val Gln Ser Gly Ser Glu Leu Lys Gln Pro Gly Ala
1               5                   10                  15

tca gtg aag gtt tcc tgt aag gct tct gga tac acc ttc act cac tat      96
Ser Val Lys Val Ser Cys Lys Ala Ser Gly Tyr Thr Phe Thr His Tyr
            20                  25                  30

ggt ttg cat tgg gtg cga cag gcc cct gga caa ggg ctt gag tgg atg      144
Gly Leu His Trp Val Arg Gln Ala Pro Gly Gln Gly Leu Glu Trp Met
        35                  40                  45

gga tgg ctc aac acc aac act gag aac cca acg ttt gcc cag ggc ttc      192
Gly Trp Leu Asn Thr Asn Thr Glu Asn Pro Thr Phe Ala Gln Gly Phe
    50                  55                  60

aca gga cgg ttt gtc ttc tcc ttg gac acc tct gtc acc acg gca tat      240
Thr Gly Arg Phe Val Phe Ser Leu Asp Thr Ser Val Thr Thr Ala Tyr
65                  70                  75                  80

ctg cag atc agc agc ctg aag gct gag gac act gcc gta tat tac tgt      288
Leu Gln Ile Ser Ser Leu Lys Ala Glu Asp Thr Ala Val Tyr Tyr Cys
                85                  90                  95

gcg aga ggg gat atg gta gta ccg act act ata tgg aac tac tac cac      336
Ala Arg Gly Asp Met Val Val Pro Thr Thr Ile Trp Asn Tyr Tyr His
            100                 105                 110

ttc atg gac gtc tgg ggc aac ggc acc ctg gtc acc gtc tcg agt          381
Phe Met Asp Val Trp Gly Asn Gly Thr Leu Val Thr Val Ser Ser
        115                 120                 125

<210>  31
<211>  381
<212>  DNA
<213>  Homo sapiens

<220>
<221>  CDS
<222>  (1)..(381)

<400>  31
cag gtg cag ctg gtg caa tct ggg tct gag ttg aag cag cct ggg gtc      48

Gln Val Gln Leu Val Gln Ser Gly Ser Glu Leu Lys Gln Pro Gly Val
1               5                   10                  15

tca gtg aag gtt tcc tgc aag gct tct gga tac acc ttc act cac tat      96
Ser Val Lys Val Ser Cys Lys Ala Ser Gly Tyr Thr Phe Thr His Tyr
            20                  25                  30

ggt tta cat tgg gtg cga cag gcc cct gga caa ggg ctt gag tgg atg      144
Gly Leu His Trp Val Arg Gln Ala Pro Gly Gln Gly Leu Glu Trp Met
        35                  40                  45

gga tgg atc aac acc aac act ggg aac cca acg tat gcc cag ggc ttc      192
Gly Trp Ile Asn Thr Asn Thr Gly Asn Pro Thr Tyr Ala Gln Gly Phe
    50                  55                  60

aca gga cgg ttt gtc ttc tcc ttg gac acc tct gtc agc acg gca tat      240
Thr Gly Arg Phe Val Phe Ser Leu Asp Thr Ser Val Ser Thr Ala Tyr
65                  70                  75                  80

ctg cag atc agc agc cta aag gct gaa gac act gcc gtg tat tac tgt      288
Leu Gln Ile Ser Ser Leu Lys Ala Glu Asp Thr Ala Val Tyr Tyr Cys
                85                  90                  95

gcg aga ggg gat tta gta gta cca act act ata tgg aac tac tac cac      336
Ala Arg Gly Asp Leu Val Val Pro Thr Thr Ile Trp Asn Tyr Tyr His
            100                 105                 110

tac atg gag gtc tgg ggc aaa ggc acc ctg gtc acc gtc tcg agt          381
Tyr Met Glu Val Trp Gly Lys Gly Thr Leu Val Thr Val Ser Ser
        115                 120                 125


<210>  32
<211>  381
<212>  DNA
<213>  Homo sapiens


<220>
<221>  CDS
<222>  (1)..(381)

<400>  32
cag gtg cag ctg gtg caa tct ggg tct gag ttg aag aag cct ggg gcc      48
Gln Val Gln Leu Val Gln Ser Gly Ser Glu Leu Lys Lys Pro Gly Ala
1               5                   10                  15

tca gtg atg gtt tcc tgc aag gct tct gga tac acc ttc act cac tat      96
Ser Val Met Val Ser Cys Lys Ala Ser Gly Tyr Thr Phe Thr His Tyr
            20                  25                  30

gct ttg cat tgg gtg cgc cag gcc cct gga caa ggg ctt gag tgg atg      144
Ala Leu His Trp Val Arg Gln Ala Pro Gly Gln Gly Leu Glu Trp Met
        35                  40                  45

gga tgg ctc aat acc aac act gag aat cca acg tat gcc cag ggc ttc      192
Gly Trp Leu Asn Thr Asn Thr Glu Asn Pro Thr Tyr Ala Gln Gly Phe
    50                  55                  60

aca gga cgg ttt gtc ttc tcc ttg gac acc tct gtc acc acg gca tat      240
Thr Gly Arg Phe Val Phe Ser Leu Asp Thr Ser Val Thr Thr Ala Tyr
65                  70                  75                  80

ctg cag atc aac agc cta aag gct gag gac act gcc gtg tat tac tgt 288
Leu Gln Ile Asn Ser Leu Lys Ala Glu Asp Thr Ala Val Tyr Tyr Cys
                85                  90                  95

gcg aga ggg gat atg gta gta cca act act ata tgg aac tac tac tac 336
Ala Arg Gly Asp Met Val Val Pro Thr Thr Ile Trp Asn Tyr Tyr Tyr
            100                 105                 110

tac atg gac gtc tgg ggc aaa ggg acc acg gtc acc gtc tcg agt 381
Tyr Met Asp Val Trp Gly Lys Gly Thr Thr Val Thr Val Ser Ser

        115                 120                 125

<210> 33
<211> 381
<212> DNA
<213> Homo sapiens

<220>
<221> CDS
<222> (1)..(381)

<400> 33
cag gtg cag ctg gtg caa tct ggg tct gag ttg aag cag cct ggg gcc 48
Gln Val Gln Leu Val Gln Ser Gly Ser Glu Leu Lys Gln Pro Gly Ala
1               5                   10                  15

tca gtg aag gtt tcc tgt aag gct tct gga tac acc ttc act cac tat 96
Ser Val Lys Val Ser Cys Lys Ala Ser Gly Tyr Thr Phe Thr His Tyr
            20                  25                  30

gct ttg cat tgg ttg cga cag gcc cct gga caa ggg ctt gag tgg atg 144
Ala Leu His Trp Leu Arg Gln Ala Pro Gly Gln Gly Leu Glu Trp Met
        35                  40                  45

gga tgg ctc aac acc aac act gag aac cca acg ttt gcc cag ggc ttc 192
Gly Trp Leu Asn Thr Asn Thr Glu Asn Pro Thr Phe Ala Gln Gly Phe
    50                  55                  60

aca gga cgt ttt gtc ttc tct ttg gac acc tct gtc acc acg gca tat 240
Thr Gly Arg Phe Val Phe Ser Leu Asp Thr Ser Val Thr Thr Ala Tyr
65                  70                  75                  80

ctg cag atc agc agc ctg aag gct gag gac act gcc gta tat tac tgt 288
Leu Gln Ile Ser Ser Leu Lys Ala Glu Asp Thr Ala Val Tyr Tyr Cys
                85                  90                  95

gcg aga ggg gat atg gta gta ccg act act ata tgg aac tac tac cac 336
Ala Arg Gly Asp Met Val Val Pro Thr Thr Ile Trp Asn Tyr Tyr His
            100                 105                 110

ttc atg gac gtc tgg ggc aac ggg acc acg gtc acc gtc tcg agt 381
Phe Met Asp Val Trp Gly Asn Gly Thr Thr Val Thr Val Ser Ser
        115                 120                 125

<210> 34
<211> 381
<212> DNA
<213> Homo sapiens

<220>
<221> CDS
<222> (1)..(381)

<400> 34
cag gtg cag ctg gtg caa tct ggg tct gag ttg aag cag cct ggg gcc 48
Gln Val Gln Leu Val Gln Ser Gly Ser Glu Leu Lys Gln Pro Gly Ala
1               5                   10                  15

tca gtg aag gtt tcc tgt aag gct tct gga tac acc ttc act cac tat 96
Ser Val Lys Val Ser Cys Lys Ala Ser Gly Tyr Thr Phe Thr His Tyr
            20                  25                  30

gct ttg cat tgg ttg cga cag gcc cct gga caa ggg ctt gag tgg atg 144
Ala Leu His Trp Leu Arg Gln Ala Pro Gly Gln Gly Leu Glu Trp Met
        35                  40                  45

gga tgg ctc aac acc aac act gag aac cca acg ttt gcc cag ggc ttc 192
Gly Trp Leu Asn Thr Asn Thr Glu Asn Pro Thr Phe Ala Gln Gly Phe
    50                  55                  60

aca gga cgt ttt gtc ttc tct ttg gac acc tct gtc acc acg gca tat 240
Thr Gly Arg Phe Val Phe Ser Leu Asp Thr Ser Val Thr Thr Ala Tyr
65                  70                  75                  80

ctg cag atc agc agc ctg aag gct gag gac act gcc gta tat tac tgt 288
Leu Gln Ile Ser Ser Leu Lys Ala Glu Asp Thr Ala Val Tyr Tyr Cys
                85                  90                  95

gcg aga ggg gat atg gta gta ccg act act ata tgg aac tac tac cac 336
Ala Arg Gly Asp Met Val Val Pro Thr Thr Ile Trp Asn Tyr Tyr His
            100                 105                 110

ttc atg gac gtc tgg ggc cag ggg acc acg gtc acc gtc tcg agt 381
Phe Met Asp Val Trp Gly Gln Gly Thr Thr Val Thr Val Ser Ser
        115                 120                 125


<210> 35
<211> 321
<212> DNA
<213> Homo sapiens


<220>
<221> CDS
<222> (1)..(321)

<400> 35
gac atc cag atg acc cag tct cca tcc tcc ctg tct gca tct gta gga 48
Asp Ile Gln Met Thr Gln Ser Pro Ser Ser Leu Ser Ala Ser Val Gly
1               5                   10                  15

gac aga gtc acc atc act tgc cgg gca agt cag agc att agc agc tac 96
Asp Arg Val Thr Ile Thr Cys Arg Ala Ser Gln Ser Ile Ser Ser Tyr
            20                  25                  30

tta aat tgg tat cag cag aaa cca ggg aaa gcc cct aag ctc ctg atc 144
Leu Asn Trp Tyr Gln Gln Lys Pro Gly Lys Ala Pro Lys Leu Leu Ile
        35                  40                  45

tat gct gca tcc agt ttg caa agt ggg gtc cca tca agg ttc agt ggc 192

Tyr Ala Ala Ser Ser Leu Gln Ser Gly Val Pro Ser Arg Phe Ser Gly
    50                  55                  60

agt gga tct ggg aca gat ttc act ctc acc atc agc agt ctg caa cct      240
Ser Gly Ser Gly Thr Asp Phe Thr Leu Thr Ile Ser Ser Leu Gln Pro
65                  70                  75                  80

gaa gat ttt gca act tac tac tgt caa cag agt tac agt acc cct cca      288
Glu Asp Phe Ala Thr Tyr Tyr Cys Gln Gln Ser Tyr Ser Thr Pro Pro
                85                  90                  95

acg ttc ggc caa ggg acc aag gtg gag atc aaa                          321
Thr Phe Gly Gln Gly Thr Lys Val Glu Ile Lys
            100                 105

<210>  36
<211>  118
<212>  PRT
<213>  Homo sapiens

<400>  36

Gln Val Gln Leu Val Gln Ser Gly Gly Gly Val Val Gln Pro Gly Arg
1               5                   10                  15

Ser Leu Arg Leu Ser Cys Val Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Gln Ile Trp Tyr Asn Ala Arg Lys Gln Glu Tyr Ser Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Arg Gly Thr Gly Tyr Asn Trp Phe Asp Pro Trp Gly Gln Gly Thr
            100                 105                 110

Leu Val Thr Val Ser Ser
        115

<210>  37
<211>  5
<212>  PRT
<213>  Homo sapiens

<400>  37

Ser Tyr Gly Met His

1               5


<210>  38
<211>  17
<212>  PRT
<213>  Homo sapiens

<400>  38

Gln Ile Trp Tyr Asn Ala Arg Lys Gln Glu Tyr Ser Asp Ser Val Lys
1               5                   10                  15

Gly


<210>  39
<211>  9
<212>  PRT
<213>  Homo sapiens

<400>  39

Gly Thr Gly Tyr Asn Trp Phe Asp Pro
1               5


<210>  40
<211>  354
<212>  DNA
<213>  Homo sapiens


<220>
<221>  CDS
<222>  (1)..(354)

<400>  40
cag gtg cag ctg gtg cag tct ggc ggc gga gtg gtg cag ccc ggc aga      48
Gln Val Gln Leu Val Gln Ser Gly Gly Gly Val Val Gln Pro Gly Arg
1               5                   10                  15

agc ctg aga ctg agc tgc gtg gcc agc ggc ttc acc ttc agc agc tac      96
Ser Leu Arg Leu Ser Cys Val Ala Ser Gly Phe Thr Phe Ser Ser Tyr
            20                  25                  30

ggc atg cac tgg gtc cgc cag gcc cct ggc aag gga ctg gaa tgg gtg      144
Gly Met His Trp Val Arg Gln Ala Pro Gly Lys Gly Leu Glu Trp Val
        35                  40                  45

gcc cag atc tgg tac aac gcc cgg aag cag gaa tac tct gac agc gtg      192
Ala Gln Ile Trp Tyr Asn Ala Arg Lys Gln Glu Tyr Ser Asp Ser Val
    50                  55                  60

aag ggc cgg ttc acc atc agc cgg gac aac agc aag aac acc ctg tac      240
Lys Gly Arg Phe Thr Ile Ser Arg Asp Asn Ser Lys Asn Thr Leu Tyr
65                  70                  75                  80

ctg cag atg aac agc ctc cgg gcc gag gac acc gcc gtg tac tac tgt      288
Leu Gln Met Asn Ser Leu Arg Ala Glu Asp Thr Ala Val Tyr Tyr Cys
                85                  90                  95

acc cgg ggc acc ggc tac aat tgg ttc gac cct tgg ggc cag ggc acc      336

Thr Arg Gly Thr Gly Tyr Asn Trp Phe Asp Pro Trp Gly Gln Gly Thr
            100                 105                 110

ctg gtc acc gtc tcc agt                                         354
Leu Val Thr Val Ser Ser
        115


<210>  41
<211>  11
<212>  PRT
<213>  Homo sapiens

<400>  41

Trp Gly Lys Gly Thr Thr Val Thr Val Ser Ser
1               5                   10


<210>  42
<211>  329
<212>  PRT
<213>  Homo sapiens

<400>  42

Ala Ser Thr Lys Gly Pro Ser Val Phe Pro Leu Ala Pro Ser Ser Lys
1               5                   10                  15


Ser Thr Ser Gly Gly Thr Ala Ala Leu Gly Cys Leu Val Lys Asp Tyr
            20                  25                  30


Phe Pro Glu Pro Val Thr Val Ser Trp Asn Ser Gly Ala Leu Thr Ser
        35                  40                  45


Gly Val His Thr Phe Pro Ala Val Leu Gln Ser Ser Gly Leu Tyr Ser
    50                  55                  60


Leu Ser Ser Val Val Thr Val Pro Ser Ser Ser Leu Gly Thr Gln Thr
65                  70                  75                  80


Tyr Ile Cys Asn Val Asn His Lys Pro Ser Asn Thr Lys Val Asp Lys
                85                  90                  95


Arg Val Glu Pro Lys Ser Cys Asp Lys Thr His Thr Cys Pro Pro Cys
            100                 105                 110


Pro Ala Pro Glu Leu Gly Arg Gly Pro Ser Val Phe Leu Phe Pro Pro
        115                 120                 125


Lys Pro Lys Asp Thr Leu Glu Ile Ser Arg Thr Pro Glu Val Thr Cys
    130                 135                 140


Val Val Val Asp Val Ser His Glu Asp Pro Glu Val Lys Phe Asn Trp

145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Asp Pro Pro Ser Arg Glu Glu
225                 230                 235                 240

Met Thr Lys Asn Gln Val Ser Leu Thr Cys Glu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly
                325

<210>  43
<211>  329
<212>  PRT
<213>  Homo sapiens

<400>  43

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser

        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Gly Arg Gly Pro Ser Val Phe Leu Phe Pro Pro
        115                 120                 125

Lys Pro Lys Asp Thr Leu Pro Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Asp Pro Pro Ser Arg Glu Glu
225                 230                 235                 240

Met Thr Lys Asn Gln Val Ser Leu Thr Cys Glu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly
                325

<210>  44
<211>  329
<212>  PRT
<213>  Homo sapiens

<400>  44

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Gly Arg Gly Pro Ser Val Phe Leu Phe Pro Pro
        115                 120                 125

Lys Pro Lys Asp Thr Leu Arg Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Asp Pro Pro Ser Arg Glu Glu
225                 230                 235                 240

Met Thr Lys Asn Gln Val Ser Leu Thr Cys Glu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly
                325


<210>  45
<211>  329
<212>  PRT
<213>  Homo sapiens

<400>  45

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Gly Arg Gly Pro Ser Val Phe Leu Phe Pro Pro
        115                 120                 125

Lys Pro Lys Asp Thr Leu Asp Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Asp Pro Pro Ser Arg Glu Glu
225                 230                 235                 240

Met Thr Lys Asn Gln Val Ser Leu Thr Cys Glu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly
                325

<210>  46
<211>  329
<212>  PRT
<213>  Homo sapiens

<400>  46

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Gly Arg Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Asp Pro Pro Ser Arg Glu Glu
225                 230                 235                 240

Met Thr Lys Asn Gln Val Ser Leu Thr Cys Glu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Leu His Tyr Thr
305                 310                 315                 320

Gln Lys Ser Leu Ser Leu Ser Pro Gly
                325

<210>  47
<211>  329
<212>  PRT
<213>  Homo sapiens

<400>  47

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
        100                 105                 110

Pro Ala Pro Glu Leu Gly Arg Gly Pro Ser Val Phe Leu Phe Pro Pro
    115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
        180                 185                 190

His Gln Asp Trp Leu Asn Gly Lys Glu Tyr Lys Cys Lys Val Ser Asn
    195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Asp Pro Pro Ser Arg Glu Glu
225                 230                 235                 240

Met Thr Lys Asn Gln Val Ser Leu Thr Cys Glu Val Lys Gly Phe Tyr
                245                 250                 255

Pro Ser Asp Ile Ala Val Glu Trp Glu Ser Asn Gly Gln Pro Glu Asn
        260                 265                 270

Asn Tyr Lys Thr Thr Pro Pro Val Leu Asp Ser Asp Gly Ser Phe Phe
    275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Glu Lys Ser Leu Ser Leu Ser Pro Gly
                325

<210>  48
<211>  329

<212>  PRT
<213>  Homo sapiens

<400>  48

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Gly Arg Gly Pro Ser Val Phe Leu Phe Pro Pro
        115                 120                 125

Lys Pro Lys Asp Thr Leu Glu Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Lys Pro Pro Ser Arg Glu Glu
225                 230                 235                 240

Met Thr Lys Asn Gln Val Ser Leu Lys Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly
                325

<210>  49
<211>  329
<212>  PRT
<213>  Homo sapiens

<400>  49

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Gly Arg Gly Pro Ser Val Phe Leu Phe Pro Pro
        115                 120                 125

Lys Pro Lys Asp Thr Leu Pro Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Lys Pro Pro Ser Arg Glu Glu
225                 230                 235                 240

Met Thr Lys Asn Gln Val Ser Leu Lys Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly
                325

<210>  50
<211>  329
<212>  PRT
<213>  Homo sapiens

<400>  50

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Gly Arg Gly Pro Ser Val Phe Leu Phe Pro Pro
        115                 120                 125

Lys Pro Lys Asp Thr Leu Arg Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Lys Pro Pro Ser Arg Glu Glu
225                 230                 235                 240

Met Thr Lys Asn Gln Val Ser Leu Lys Cys Leu Val Lys Gly Phe Tyr
                245                 250                 255

Pro Ser Asp Ile Ala Val Glu Trp Glu Ser Asn Gly Gln Pro Glu Asn

260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly
                325

<210>  51
<211>  329
<212>  PRT
<213>  Homo sapiens

<400>  51

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Gly Arg Gly Pro Ser Val Phe Leu Phe Pro Pro
        115                 120                 125

Lys Pro Lys Asp Thr Leu Asp Ile Ser Arg Thr Pro Glu Val Thr Cys
    130                 135                 140

Val Val Val Asp Val Ser His Glu Asp Pro Glu Val Lys Phe Asn Trp

145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Lys Pro Pro Ser Arg Glu Glu
225                 230                 235                 240

Met Thr Lys Asn Gln Val Ser Leu Lys Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly
                325

<210>  52
<211>  329
<212>  PRT
<213>  Homo sapiens

<400>  52

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser

        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Gly Arg Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Lys Pro Pro Ser Arg Glu Glu
225                 230                 235                 240

Met Thr Lys Asn Gln Val Ser Leu Lys Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Leu His Tyr Thr
305                 310                 315                 320

Gln Lys Ser Leu Ser Leu Ser Pro Gly
                325

<210>  53
<211>  329
<212>  PRT
<213>  Homo sapiens

<400>  53

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Gly Arg Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Lys Pro Pro Ser Arg Glu Glu
225                 230                 235                 240

Met Thr Lys Asn Gln Val Ser Leu Lys Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Glu Lys Ser Leu Ser Leu Ser Pro Gly
                325